# EUROPEAN PATENT APPLICATION

(11) **EP 4 491 182 A1**
(43) Date of publication of application: **15.01.2025**
(21) Application number: 23766144.2
(22) Date of filing: 10.03.2023
(51) Int. Cl.: A61K 31/4745, C07D 455/03, C12Q 1/6886, A61P 35/00

(54) **APPLICATION OF TETRACYCLIC COMPOUND IN TREATMENT OF TUMORS**

(30) Priority: 11.03.2022 CN 202210241115
(71) Applicant: Shanghai Shijiang Biotechnology Co., Ltd, Shanghai 200333 (CN)
(72) Inventor: SHI, Yufeng, Nanjing, Jiangsu 210032 (CN); MA, Wenjiang, Nanjing, Jiangsu 210032 (CN)
(74) Representative: reuteler & cie SA
(86) International application number: PCT/CN2023/080841
(87) International publication number: WO 2023/169567

(57) **Abstract**

The present invention relates to an application of a tetracyclic compound in the treatment of tumors. Specifically provided is a use of a compound of formula I, an optical isomer or racemate thereof, a solvate thereof, a pharmaceutically acceptable salt thereof or a deuterated compound thereof, which is used for preparing a composition or a formulation, said composition or formulation being used for preventing and/or treating tumors. A compound of the invention has a uniquely advantageous precision treatment effect on tumors having low expression or no expression of the NNMT gene, high expression of a DNA methylase, high expression of UHRFI, a high NNMT gene nucleotide site methylation level, and/or a high NNMT gene region DNA CpG site methylation level.

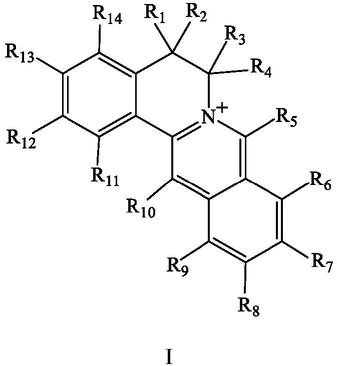

## Description

### FIELD OF THE INVENTION

The present invention relates to the field of medicine. Specifically, the present invention relates to application of tetracyclic compound in treatment of tumors.

### BACKGROUND TECHNOLOGY

Tumor is a common disease that seriously endangers human health, and the mortality rate of malignant tumors is rising. Due to the heterogeneity of tumor, simply using the same treatment method or medication based on source or pathological characteristic of tumors can easily lead to improper treatment, which can delay valuable treatment time and opportunities for patient. Therefore, it is very necessary to take precision treatment according to the different features of tumor. With the development of biological technology, tumors are typed at the molecular level such as gene and protein level, etc, and more and more changes in tumor-related gene and the expression and activity of protein have been discovered, changes in tumor-related gene and the expression and activity of protein play an important role in the development of malignant tumors, the discovery and application of biomarkers can provide precise guidance for the application of related drug and make precision treatment of tumor possible, thereby achieving targeted administration of drug, significantly improving treatment effect, reducing the dose of drug and reducing toxic side effects.

Therefore, there is an urgent need in the art to develop a drug that can achieve precision treatment on tumor.

### SUMMARY OF THE INVENTION

It is an object of the present invention to provide a compound that can have excellent precision treatment effect on tumor with low or no expression of NNMT gene, high expression of DNA methylase, high expression of UHRFl, high methylation level of nucleotide site of NNMT gene, and/or high methylation level of DNA CpG site of NNMT gene.

In the first aspect of the present invention, it provides a use of a compound of formula I, or an optical isomer thereof, or a racemate thereof, or a solvate thereof, or a pharmaceutically acceptable salt thereof, or a deuterated compound thereof in the preparation of a composition or a preparation for preventing and/or treating tumor; 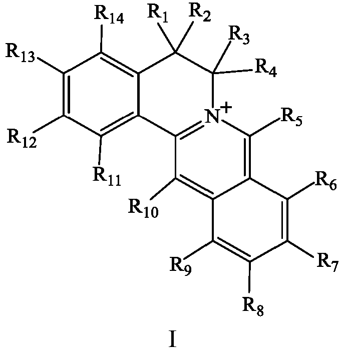 wherein,
R₁, R₂, R₃ and R₄ are each independently hydrogen, substituted or unsubstituted C1-C6 alkyl;
R₅ is hydrogen, substituted or unsubstituted C1-C10 alkyl, substituted or unsubstituted C3-C10 cycloalkyl;
R₆ is -O-R₁₅ or -N (R₁₆ R₁₇);
R₇ is hydrogen, substituted or unsubstituted C1-C6 alkoxyl, substituted or unsubstituted C1-C8 haloalkoxyl;
R₈ is hydrogen, substituted or unsubstituted C1-C6 alkyl, halogen;
R₉ is hydrogen, substituted or unsubstituted C1-C6 alkyl, halogen;
R₁₀ is hydrogen, substituted or unsubstituted C1-C16 alkyl, substituted or unsubstituted C6-C12 aryl-substituted or unsubstituted C1-C6 alkyl-, substituted or unsubstituted C1-C6 alkyl-substituted or unsubstituted C6-C12 aryl-C1-C6 alkyl-, substituted or unsubstituted C1-C6 haloalkoxyl-substituted or unsubstituted C6-C12 aryl-substituted or unsubstituted C1-C6 alkyl-, substituted or unsubstituted C1-C6 haloalkyl-substituted or unsubstituted C6-C12 aryl-substituted or unsubstituted C1-C6 alkyl-, substituted or unsubstituted C1-C6 alkoxyl-substituted or unsubstituted C6-C12 aryl-substituted or unsubstituted C1-C6 alkyl-;
R₁₁ and R₁₄ are each independently hydrogen, substituted or unsubstituted C1-C6 alkyl;
R₁₂ and R₁₃ are connected to form a substituted or unsubstituted 3-10 membered heterocycloalkane ring;
R₁₅ is substituted or unsubstituted C1-C18 alkyl, deuterated C1-C18 alkyl, substituted or unsubstituted C6-C12 aryl, substituted or unsubstituted C6-C12 aryl-substituted or unsubstituted C6-C12 aryl-substituted or unsubstituted C1-C8 alkyl-, substituted or unsubstituted C6-C12 aryl-O-substituted or unsubstituted C1-C6 alkyl-, substituted or unsubstituted C1-C10 haloalkyl, substituted or unsubstituted C2-C16 acyl, substituted or unsubstituted C2-C8 alkenyl-substituted or unsubstituted C1-C4 alkyl-substituted or unsubstituted C2-C8 alkenyl-substituted or unsubstituted C1-C4 alkyl-, substituted or unsubstituted C3-C14 cycloalkyl-substituted or unsubstituted C2-C6 acyl-, substituted or unsubstituted C6-C12 aryl-substituted or unsubstituted C2-C8 alkenyl-C(O)-substituted or unsubstituted C1-C4 alkyl-, or 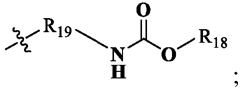
R₁₆ and R₁₇ are each independently hydrogen, substituted or unsubstituted C1-C12 alkyl, substituted or unsubstituted C6-C12 aryl-substituted or unsubstituted C1-C4 alkyl-;
R₁₈ is substituted or unsubstituted C1-C8 alkyl;
R₁₉ is substituted or unsubstituted C1-C10 alkylidene.
In another preferred embodiment, the "substituted" means that one or more (preferably 1, 2, 3, or 4) hydrogen atoms on the ring or group are each independently substituted by the substituent selected from the group consisting of C1-C6 alkyl, C2-C6 alkenyl, halogen, C3-C6 cycloalkyl, C1-C6 haloalkyl, C1-C6 alkoxyl, C1-C6 haloalkoxyl, C3-C6 cycloalkoxyl, C2-C6 acyl, C6-C12 aryl, 5-12 membered heteroaryl, C6-C12 aryl-O-, 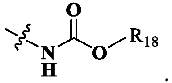
In another preferred embodiment, the "substituted" means that one or more (preferably 1, 2, 3, or 4) hydrogen atoms on the ring or group are each independently substituted by the substituent selected from the group consisting of C1-C4 alkyl, C2-C4 alkenyl, halogen, C3-C6 cycloalkyl, C1-C4 haloalkyl, C1-C4 alkoxyl, C1-C4 haloalkoxyl, C3-C6 cycloalkoxyl, C2-C4 acyl, C6-C8 aryl, 5-8 membered heteroaryl, C6-C8 aryl-O-,

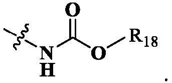

In another preferred embodiment, the heterocyclic ring of the heterocycloalkane ring and heteroaryl has 1-4 (preferably 1, 2 or 3) heteroatoms independently selected from the group consisting of N, O and S.

In another preferred embodiment, the heterocyclic ring of the heterocycloalkane ring has 1-4 (preferably 1, 2 or 3) heteroatoms independently selected from the group consisting of N, O and S.

In another preferred embodiment, the heterocyclic ring of the heteroaryl each independently has 1-4 (preferably 1, 2 or 3) heteroatoms independently selected from the group consisting of N, O and S.

In another preferred embodiment, each "substituted" means that one or more (preferably 1, 2, 3, 4, 5, 6, 7 or 8) hydrogen atoms on the ring or group are each independently substituted by a substituent.

In another preferred embodiment, the heterocycloalkyl has 1 or 2 C=C ring double bonds.

In another preferred embodiment, the haloalkoxyl is trifluoromethoxyl.

In another preferred embodiment, the haloalkyl is trifluoromethyl.

In another preferred embodiment, R₁, R₂, R₃ and R₄ are each independently hydrogen, substituted or unsubstituted C1-C4 alkyl.

In another preferred embodiment, R₁, R₂, R₃ and R₄ are each independently hydrogen.

In another preferred embodiment, R₁ is hydrogen.

In another preferred embodiment, R₂ is hydrogen.

In another preferred embodiment, R₃ is hydrogen.

In another preferred embodiment, R₄ is hydrogen.

In another preferred embodiment, R₅ is hydrogen, substituted or unsubstituted C1-C10 alkyl, substituted or unsubstituted C3-C8 cycloalkyl.

In another preferred embodiment, R₅ is hydrogen, substituted or unsubstituted C1-C8 alkyl, substituted or unsubstituted C3-C8cycloalkyl.

In another preferred embodiment, R₅ is hydrogen, substituted or unsubstituted C1-C6 alkyl, substituted or unsubstituted C4-C7 cycloalkyl.

In another preferred embodiment, R₅ is hydrogen, substituted or unsubstituted C3-C10 alkyl, substituted or unsubstituted C3-C8 cycloalkyl.

In another preferred embodiment, R₅ is hydrogen, substituted or unsubstituted C3-C10 alkyl, substituted or unsubstituted C4-C6 cycloalkyl.

In another preferred embodiment, R₅ is hydrogen, substituted or unsubstituted C4-C8 alkyl, substituted or unsubstituted C4-C7 cycloalkyl.

In another preferred embodiment, R₅ is hydrogen, substituted or unsubstituted C4-C8 alkyl, substituted or unsubstituted C4-C6 cycloalkyl.

In another preferred embodiment, R₅ is hydrogen, ethyl, butyl, hexyl or cyclopentyl.

In another preferred embodiment, R₇ is hydrogen, substituted or unsubstituted C1-C4 alkoxyl, substituted or unsubstituted C1-C6 haloalkoxyl.

In another preferred embodiment, R₇ is hydrogen, substituted or unsubstituted C1-C2 alkoxyl, substituted or unsubstituted C1-C6 haloalkoxyl.

In another preferred embodiment, R₇ is hydrogen, substituted or unsubstituted C1-C2 alkoxyl, substituted or unsubstituted C1-C4 haloalkoxyl.

In another preferred embodiment, R₇ is hydrogen, methoxyl, or halopropoxyl.

In another preferred embodiment, R₇ is hydrogen, methoxyl, or chloropropoxyl.

In another preferred embodiment, R₈ is hydrogen, substituted or unsubstituted C1-C4 alkyl or halogen.

In another preferred embodiment, R₈ is hydrogen or halogen.

In another preferred embodiment, R₈ is hydrogen or bromine.

In another preferred embodiment, R₉ is hydrogen, substituted or unsubstituted C1-C4 alkyl or halogen.

In another preferred embodiment, R₉ is hydrogen or halogen.

In another preferred embodiment, R₉ is hydrogen or bromine.

In another preferred embodiment, R₁₀ is hydrogen, substituted or unsubstituted C1-C16 alkyl, substituted or unsubstituted C6-C12 aryl-substituted or unsubstituted C1-C6 alkyl-, substituted or unsubstituted C1-C6 alkyl- substituted or unsubstituted C6-C10 aryl-substituted or unsubstituted C1-C6 alkyl-, substituted or unsubstituted C1-C6 haloalkoxyl-substituted or unsubstituted C6-C10 aryl-substituted or unsubstituted C1-C6 alkyl-, substituted or unsubstituted C1-C6 haloalkyl-substituted or unsubstituted C6-C10 aryl-substituted or unsubstituted C1-C6 alkyl-, substituted or unsubstituted C1-C6 alkoxyl-substituted or unsubstituted C6-C10 aryl-substituted or unsubstituted C1-C6 alkyl-;

In another preferred embodiment, R₁₀ is hydrogen, substituted or unsubstituted C1-C16 alkyl (e,g, substituted or unsubstituted C4-C14 alkyl), substituted or unsubstituted C6-C8 aryl-substituted or unsubstituted C1-C4 alkyl-, substituted or unsubstituted C1-C6 alkyl-substituted or unsubstituted C6-C8 aryl-substituted or unsubstituted C1-C4 alkyl-, substituted or unsubstituted C1-C4 haloalkoxyl-substituted or unsubstituted C6-C8 aryl-substituted or unsubstituted C1-C4 alkyl-, substituted or unsubstituted C1-C4 haloalkyl-substituted or unsubstituted C6-C8 aryl-substituted or unsubstituted C1-C4 alkyl-, substituted or unsubstituted C1-C4 alkoxyl-substituted or unsubstituted C6-C8 aryl-substituted or unsubstituted C1-C4 alkyl-.

In another preferred embodiment, R₁₀ is hydrogen, substituted or unsubstituted C1-C16 alkyl (e,g, substituted or unsubstituted C4-C14 alkyl), substituted or unsubstituted C6-C8 aryl-substituted or unsubstituted C1-C4 alkyl-, substituted or unsubstituted C1-C6 alkyl- substituted or unsubstituted C6-C8 aryl-substituted or unsubstituted C1-C2 alkyl-, substituted or unsubstituted C1-C4 haloalkoxyl-substituted or unsubstituted C6-C8 aryl-substituted or unsubstituted C1-C2 alkyl-, substituted or unsubstituted C1-C4 haloalkyl-substituted or unsubstituted C6-C8 aryl-substituted or unsubstituted C1-C2 alkyl-, substituted or unsubstituted C1-C4 alkoxyl-substituted or unsubstituted C6-C8 aryl-substituted or unsubstituted C1-C4 alkyl-.

In another preferred embodiment, R₁₀ is hydrogen, substituted or unsubstituted C1-C16 alkyl (e,g, substituted or unsubstituted C4-C14 alkyl, or substituted or unsubstituted C1-C14 alkyl), substituted or unsubstituted C6-C8 aryl-substituted or unsubstituted C1-C4 alkyl-, substituted or unsubstituted C1-C4 alkyl- substituted or unsubstituted C6-C8 aryl-substituted or unsubstituted C1-C2 alkyl-, substituted or unsubstituted C1-C2 haloalkoxyl-substituted or unsubstituted C6-C8 aryl-substituted or unsubstituted C1-C2 alkyl-, substituted or unsubstituted C1-C2 haloalkyl-substituted or unsubstituted C6-C8 aryl-substituted or unsubstituted C1-C2 alkyl-, substituted or unsubstituted C1-C2 alkoxyl-substituted or unsubstituted C6-C8 aryl-substituted or unsubstituted C1-C4 alkyl-.

In another preferred embodiment, R₁₀ is hydrogen, substituted or unsubstituted butyl-substituted or unsubstituted phenyl-substituted or unsubstituted methyl-, substituted or unsubstituted propyl-substituted or unsubstituted phenyl-substituted or unsubstituted methyl-, substituted or unsubstituted ethyl, substituted or unsubstituted propyl, substituted or unsubstituted butyl, substituted or unsubstituted pentyl, substituted or unsubstituted hexyl, substituted or unsubstituted heptyl, substituted or unsubstituted octyl, substituted or unsubstituted decyl, substituted or unsubstituted dodecyl, substituted or unsubstituted tetradecyl, substituted or unsubstituted phenyl-substituted or unsubstituted ethyl-, substituted or unsubstituted phenyl-substituted or unsubstituted or unsubstituted propyl-, trifluoromethoxyl-substituted or unsubstituted phenyl-substituted or unsubstituted methyl-, trifluoromethyl-substituted or unsubstituted phenyl-substituted or unsubstituted methyl-, substituted or unsubstituted methoxyl-substituted or unsubstituted phenyl-substituted or unsubstituted propyl-.

In another preferred embodiment, R₁₀ is hydrogen, substituted or unsubstituted C5-C14 alkyl.

In another preferred embodiment, the substituted or unsubstituted C5-C14 alkyl is substituted or unsubstituted pentyl, substituted or unsubstituted dodecyl, or substituted or unsubstituted tetradecyl.

In another preferred embodiment, R₁₀ is hydrogen, butyl-phenyl-methyl-, propyl-phenyl-methyl-, ethyl, propyl, butyl, pentyl, hexyl, heptyl, octyl, decyl, dodecyl, tetradecyl, phenyl-ethyl-, phenyl-propyl-, trifluoromethoxyl-phenyl-methyl-, trifluoromethyl-phenyl-methyl-, methoxyl-phenyl-propyl-.

In another preferred embodiment, R₁₁ and R₁₄ are each independently hydrogen, substituted or unsubstituted C1-C4 alkyl.

In another preferred embodiment, R₁₁ and R₁₄ are each independently hydrogen.

In another preferred embodiment, R₁₂ and R₁₃ are connected to form a substituted or unsubstituted 3-8 membered heterocycloalkane ring.

In another preferred embodiment, R₁₂ and R₁₃ are connected to form a substituted or unsubstituted 3 membered heterocycloalkane ring, substituted or unsubstituted 4 membered heterocycloalkane ring, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring.

In another preferred embodiment, R₁₂ and R₁₃ are connected to form a substituted or unsubstituted 5 membered heterocycloalkane ring.

In another preferred embodiment, R₁₂ and R₁₃ are connected to form a substituted or unsubstituted 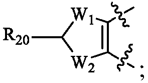
W₁ and W₂ are each independently O or S;
R₂₀ is hydrogen, substituted or unsubstituted C1-C6 alkyl, substituted or unsubstituted C3-C8 cycloalkyl.

In another preferred embodiment, R₁₂ and R₁₃ are connected to form

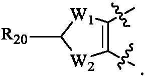

In another preferred embodiment, W₁ is O or S.

In another preferred embodiment, W₂ is O or S.

In another preferred embodiment, R₁₂ and R₁₃ are connected to form a substituted or unsubstituted 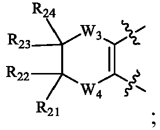 W₃ and W₄ are each independently O or S;
R₂₁, R₂₂, R₂₃ and R₂₄ are each independently hydrogen, substituted or unsubstituted C1-C6 alkyl, substituted or unsubstituted C3-C8 cycloalkyl.

In another preferred embodiment, R₁₂ and R₁₃ are connected to form

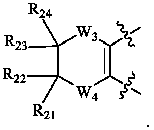

In another preferred embodiment, W₃ is O or S.

In another preferred embodiment, W₄ is O or S.

In another preferred embodiment, R₁₅ is substituted or unsubstituted C1-C18 alkyl, deuterated C1-C18 alkyl, substituted or unsubstituted C6-C12 aryl, substituted or unsubstituted C6-C12 aryl-substituted or unsubstituted C6-C12 aryl-substituted or unsubstituted C1-C6 alkyl-, substituted or unsubstituted C6-C10 aryl-O-substituted or unsubstituted C1-C6 alkyl-, substituted or unsubstituted C1-C8 haloalkyl, substituted or unsubstituted C2-C14 acyl, substituted or unsubstituted C2-C6 alkenyl-substituted or unsubstituted C1-C4 alkyl-substituted or unsubstituted C2-C6 alkenyl-substituted or unsubstituted C1-C4 alkyl-, substituted or unsubstituted C3-C14 cycloalkyl-substituted or unsubstituted C2-C6 acyl-, substituted or unsubstituted C6-C10 aryl-substituted or unsubstituted C2-C6 alkenyl-C(O)-substituted or unsubstituted C1-C4 alkyl-, or

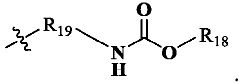

In another preferred embodiment, R₁₅ is substituted or unsubstituted C1-C16 alkyl, deuterated C1-C16 alkyl, substituted or unsubstituted C6-C8 aryl, substituted or unsubstituted C6-C8 aryl-substituted or unsubstituted C6-C8 aryl-substituted or unsubstituted C1-C4 alkyl-, substituted or unsubstituted C6-C8 aryl-O-substituted or unsubstituted C1-C4 alkyl-, substituted or unsubstituted C1-C6 haloalkyl (e.g., substituted or unsubstituted C3-C6 haloalkyl, or substituted or unsubstituted C2-C6 haloalkyl), substituted or unsubstituted C2-C14 acyl (e.g., substituted or unsubstituted C5-C12 acyl), substituted or unsubstituted C2-C4 alkenyl-substituted or unsubstituted C1-C3 alkyl-substituted or unsubstituted C2-C4 alkenyl-substituted or unsubstituted C1-C2 alkyl-, substituted or unsubstituted C3-C12 cycloalkyl (e.g., substituted or unsubstituted C6-C12 cycloalky)-substituted or unsubstituted C2-C4 acyl-, substituted or unsubstituted C6-C8 aryl-substituted or unsubstituted C2-C4 alkenyl-C(O)-substituted or unsubstituted C1-C2 alkyl-, or

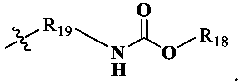

In another preferred embodiment, R₁₅ is methyl, deuterated methyl, substituted or unsubstituted C6-C10 alkyl, haloethyl, halopropyl, halopentyl, halohexyl, haloheptyl, or

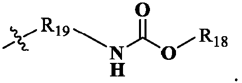

In another preferred embodiment, the substituted or unsubstituted C6-C10 alkyl is substituted or unsubstituted hexyl, substituted or unsubstituted octyl, or substituted or unsubstituted decyl.

In another preferred embodiment, the halohexyl is chlorohexyl or bromohexyl.

In another preferred embodiment, the haloethyl is bromoethyl, chloroethyl, or fluoroethyl.

In another preferred embodiment, the halopropyl is bromopropyl, chloropropyl.

In another preferred embodiment, R₁₅ is substituted or unsubstituted methyl, deuterated methyl, substituted or unsubstituted ethyl, substituted or unsubstituted propyl, substituted or unsubstituted hexyl, substituted or unsubstituted octyl, substituted or unsubstituted decyl, substituted or unsubstituted dodecyl, substituted or unsubstituted pentadecyl, substituted or unsubstituted hexadecyl, substituted or unsubstituted phenyl-O-substituted or unsubstituted butyl-, haloethyl, halopropyl, halopentyl, halohexyl, haloheptyl, substituted or unsubstituted hexanoyl, substituted or unsubstituted pentanoyl, substituted or unsubstituted dodecanoyl, substituted or unsubstituted butenyl-substituted or unsubstituted ethyl-substituted or unsubstituted propenyl-substituted or unsubstituted methyl-, substituted or unsubstituted cyclodecyl-substituted or unsubstituted acetyl-, substituted or unsubstituted methyl-substituted or unsubstituted phenyl-substituted or unsubstituted ethenyl-C(O)-substituted or unsubstituted methyl-, 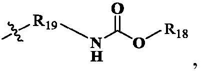 substituted or unsubstituted phenyl- substituted or unsubstituted phenyl-substituted or unsubstituted methyl-.

In another preferred embodiment, R₁₅ is methyl, deuterated methyl, ethyl, propyl, hexyl, octyl, decyl, dodecyl, pentadecyl, hexadecyl, phenyl-O-butyl-, haloethyl, halopropyl, halopentyl, halohexyl, haloheptyl, hexanoyl, pentanoyl, dodecanoyl, butenyl-ethyl-propenyl-methyl-, cyclodecyl-acetyl-, methyl-phenyl-ethenyl-C(O)-methyl-, 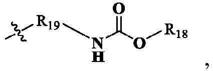 phenyl-phenyl-methyl-.

In another preferred embodiment, R₁₅ is methyl, deuterated methyl, ethyl, propyl, hexyl, octyl, decyl, dodecyl, pentadecyl, hexadecyl, phenyl-O-butyl-, bromoethyl, chloroethyl, fluoroethyl, bromopropyl, chloropropyl, halopentyl, chlorohexyl, haloheptyl, bromohexyl, hexanoyl, pentanoyl, dodecanoyl, butenyl-ethyl-propenyl-methyl-, cyclodecyl-acetyl-, 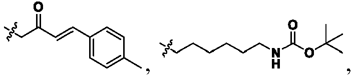 phenyl-phenyl-methyl-.

In another preferred embodiment, the deuterated methyl is

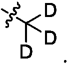

In another preferred embodiment, R₁₆ and R₁₇ are each independently hydrogen, substituted or unsubstituted C1-C10 alkyl, substituted or unsubstituted C6-C10 aryl-substituted or unsubstituted C1-C4 alkyl-.

In another preferred embodiment, R₁₆ and R₁₇ are each independently hydrogen, substituted or unsubstituted C4-C10 alkyl, substituted or unsubstituted C6-C8 aryl-substituted or unsubstituted C1-C4 alkyl-.

In another preferred embodiment, R₁₆ and R₁₇ are each independently hydrogen, substituted or unsubstituted C6-C10 alkyl, substituted or unsubstituted C6-C8 aryl-substituted or unsubstituted C1-C2 alkyl-.

In another preferred embodiment, R₁₆ and R₁₇ are each independently hydrogen, octyl, phenyl-ethyl-.

In another preferred embodiment, R₁₈ is substituted or unsubstituted C1-C6 alkyl.

In another preferred embodiment, R₁₈ is substituted or unsubstituted C2-C6 alkyl.

In another preferred embodiment, R₁₈ is butyl.

In another preferred embodiment, R₁₈ is tertbutyl. In another preferred embodiment, R₁₈ is

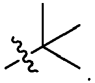

In another preferred embodiment, R₁₉ is substituted or unsubstituted C1-C8 alkylidene.

In another preferred embodiment, R₁₉ is substituted or unsubstituted C4-C8 alkylidene.

In another preferred embodiment, R₁₉ is hexylidene.

In another preferred embodiment, R₁₉ is n-hexylidene. In another preferred embodiment, R₁₉ is

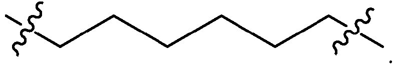

In another preferred embodiment, R₂₀ is hydrogen, substituted or unsubstituted C1-C4 alkyl, substituted or unsubstituted C3-C8 cycloalkyl.

In another preferred embodiment, R₂₀ is hydrogen, substituted or unsubstituted C1-C4 alkyl, substituted or unsubstituted C3-C6 cycloalkyl.

In another preferred embodiment, R₂₀ is hydrogen.

In another preferred embodiment, R₂₁, R₂₂, R₂₃ and R₂₄ are each independently hydrogen, substituted or unsubstituted C1-C4 alkyl, substituted or unsubstituted C3-C8 cycloalkyl.

In another preferred embodiment, R₂₁, R₂₂, R₂₃ and R₂₄ are each independently hydrogen, substituted or unsubstituted C1-C4 alkyl, substituted or unsubstituted C3-C6 cycloalkyl.

In another preferred embodiment, R₂₁, R₂₂, R₂₃ and R₂₄ are each independently hydrogen.

In another preferre d embodiment, the halogen is fluorine, chlorine, bromine or iodine.

In another preferred embodiment, the aryl is phenyl.

In another preferred embodiment, the halo is mono-halo, di-halo, tri-halo or full-halo.

In another preferred embodiment, the chloro is mono-chloro, di- chloro, tri-chloro or full- chloro.

In another preferred embodiment, the bromo is mono-bromo, di-bromo, tri-bromo or full-bromo.

In another preferred embodiment, the fluoro is mono-fluoro, di-fluoro, tri-fluoro or full-fluoro.

In another preferred embodiment, halo is fluoro, chloro, bromo or iodo.

In another preferred embodiment, in the substituted or unsubstituted phenyl, the substituted or unsubstituted phenyl is mono-substituted or unsubstituted phenyl.

In another preferred embodiment, in the substituted or unsubstituted phenyl, the substituted or unsubstituted phenyl is mono-substituted or unsubstituted phenyl, and the substitution is ortho, para or meta substitution on the phenyl.

In another preferred embodiment, the substituted or unsubstituted phenyl is mono-substituted, di-substituted, tri-substituted or unsubstituted phenyl.

In another preferred embodiment, the substituted or unsubstituted phenyl is mono-substituted, di-substituted, tri-substituted or unsubstituted phenyl, and the substitution is ortho, para and/or meta substitution on the phenyl. In another preferred embodiment, the phenyl-O-butyl- is

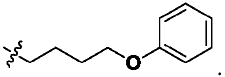

In another preferred embodiment, the bromoethyl is monobromoethyl. In another preferred embodiment, the bromoethyl is

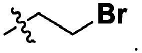

In another preferred embodiment, the chloroethyl is monochloroethyl.

In another preferred embodiment, the chloroethyl is

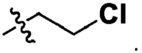

In another preferred embodiment, the fluoroethyl is monofluoroethyl.

In another preferred embodiment, the fluoroethyl is

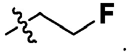

In another preferred embodiment, the bromopropyl is monobromopropyl.

In another preferred embodiment, the bromopropyl is

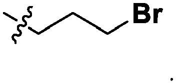

In another preferred embodiment, the chloropropyl is monochloropropyl.

In another preferred embodiment, the chloropropyl is

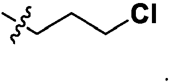

In another preferred embodiment, the chlorohexyl is monochlorohexyl.

In another preferred embodiment, the chlorohexyl is

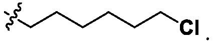

In another preferred embodiment, the bromohexyl is monobromohexyl.

In another preferred embodiment, the bromohexyl is

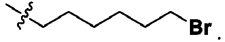

In another preferred embodiment, the chloropropoxyl is

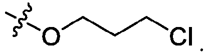

In another preferred embodiment, the hexanoyl is

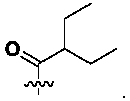

In another preferred embodiment, the pentanoyl is

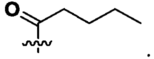

In another preferred embodiment, the dodecanoyl is

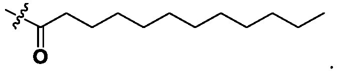

In another preferred embodiment, the cyclodecyl-acetyl- is

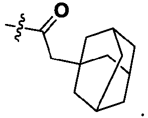

In another preferred embodiment, the butenyl-ethyl-propenyl-methyl- is

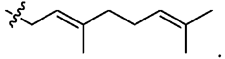

In another preferred embodiment, the propyl is n-propyl or isopropyl. In another preferred embodiment, the propyl is

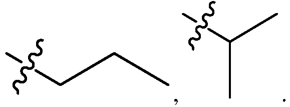

In another preferred embodiment, the butyl is n-butyl or tert-butyl.

In another preferred embodiment, the butyl is

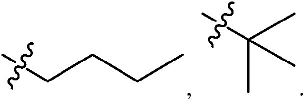

In another preferred embodiment, the pentyl is

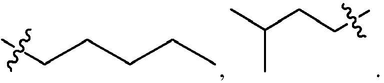

In another preferred embodiment, the hexyl is n-hexyl. In another preferred embodiment, the hexyl is

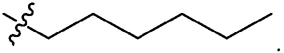

In another preferred embodiment, the heptyl is n-heptyl.

In another preferred embodiment, the heptyl is

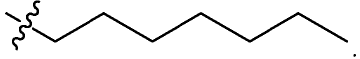

In another preferred embodiment, the octyl is n-octyl.

In another preferred embodiment, the octyl is

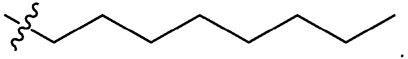

In another preferred embodiment, the decyl is n-decyl.

In another preferred embodiment, the decyl is

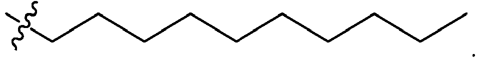

In another preferred embodiment, the dodecyl is n-dodecyl.

In another preferred embodiment, the dodecyl is

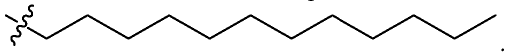

In another preferred embodiment, the tetradecyl is n-tetradecyl.

In another preferred embodiment, the tetradecyl is

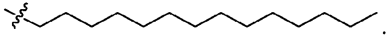

In another preferred embodiment, the pentadecyl is n-pentadecyl.

In another preferred embodiment, the pentadecyl is

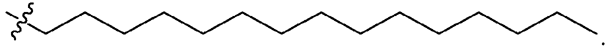

In another preferred embodiment, the hexadecyl is n-hexadecyl.

In another preferred embodiment, the hexadecyl is

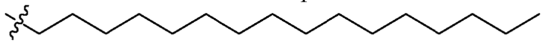

In another preferred embodiment, the cyclopentyl is

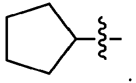

In another preferred embodiment, the propyl-phenyl-methyl- is

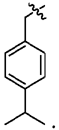

In another preferred embodiment, the butyl-phenyl-methyl- is

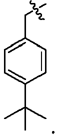

In another preferred embodiment, the phenyl-ethyl- is

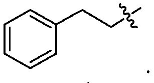

In another preferred embodiment, the phenyl-propyl- is

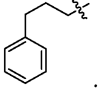

In another preferred embodiment, the trifluoromethoxyl-phenyl-methyl- is

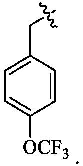

In another preferred embodiment, the trifluoromethyl-phenyl-methyl- is

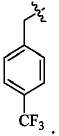

In another preferred embodiment, the methoxyl-phenyl-propyl- is

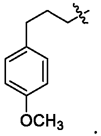

In another preferred embodiment, the compound of formula I has the following structure of formula I-1: 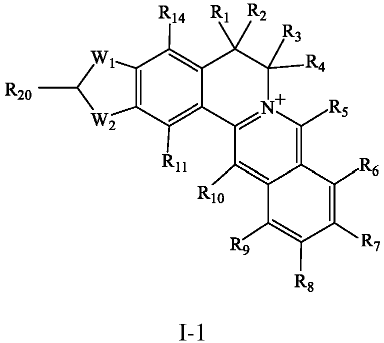 wherein,
R₁, R₂, R₃, R₄, R₅, R₆, R₇, R₈, R₉, R₁₀, R₁₁, R₁₄, W₁, W₁ and R₂₀ are each independently defined as described above.

In another preferred embodiment, the compound of formula I has the following structure of formula 1-2: 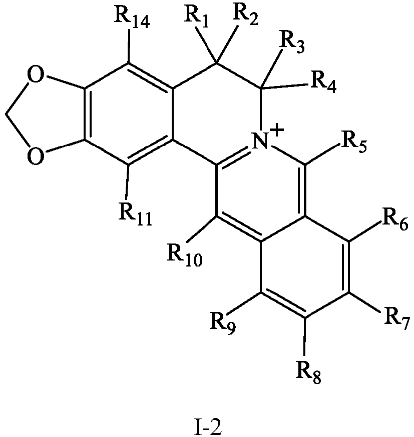 wherein,
R₁, R₂, R₃, R₄, R₅, R₆, R₇, R₈, R₉, R₁₀, R₁₁ and R₁₄ are each independently defined as described above.

In another preferred embodiment, the compound of formula I has the following structure of formula I-3: 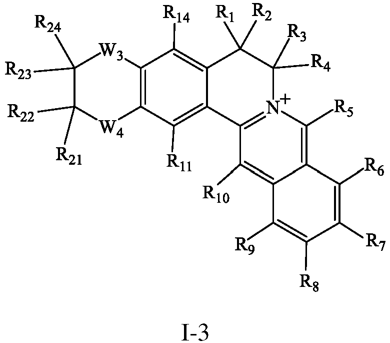 wherein,
R₁, R₂, R₃, R₄, R₅, R₆, R₇, R₈, R₉, R₁₀, R₁₁, R₁₄, W₃, W₄, R₂₁, R₂₂, R₂₃ and R₂₄ are each independently defined as described above.

In another preferred embodiment, the compound of formula I has the following structure of formula I-4: 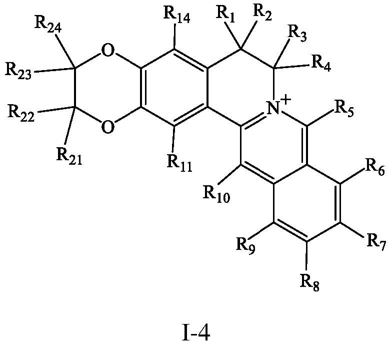 wherein,
R₁, R₂, R₃, R₄, R₅, R₆, R₇, R₈, R₉, R₁₀, R₁₁, R₁₄, R₂₁, R₂₂, R₂₃ and R₂₄ are each independently defined as described above.

In another preferred embodiment, the compound of formula I, or an optical isomer thereof, or a racemate thereof, or a solvate thereof, or a pharmaceutically acceptable salt thereof, or a deuterated compound thereof is selected from the following group:

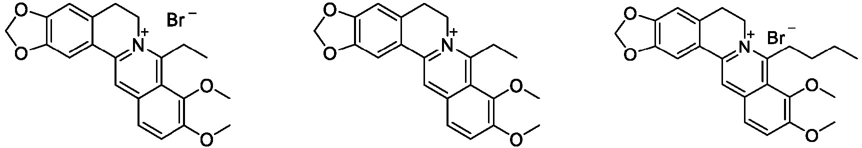

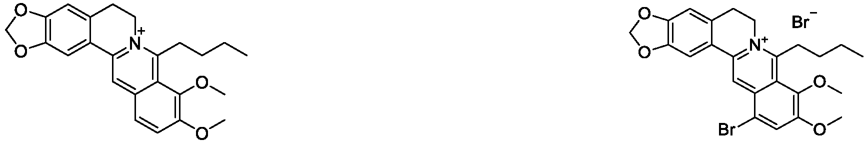

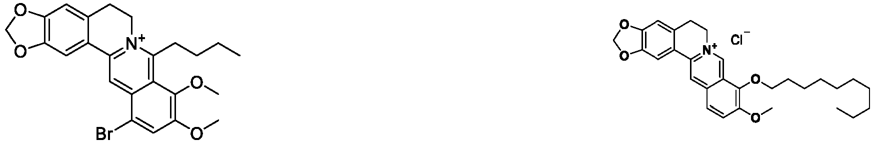

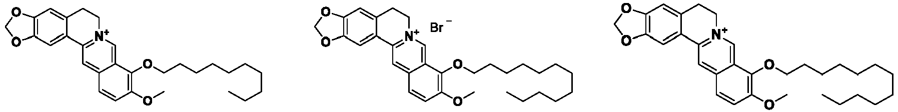

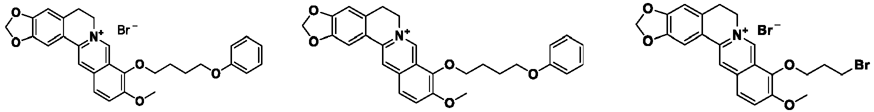

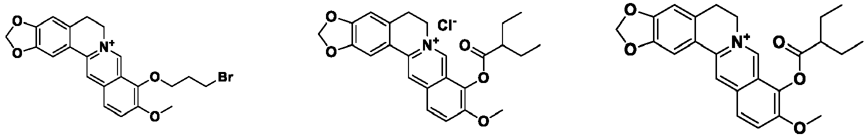

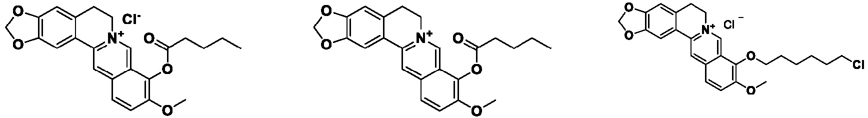

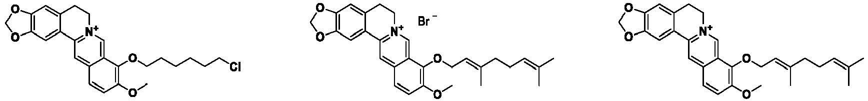

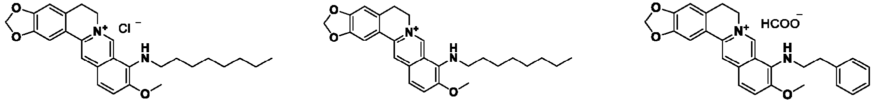

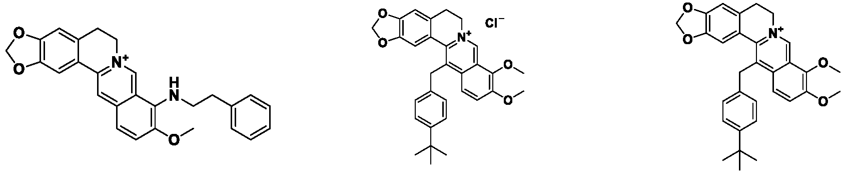

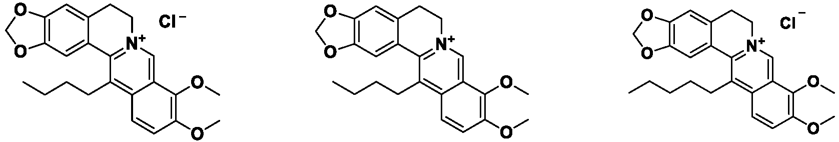

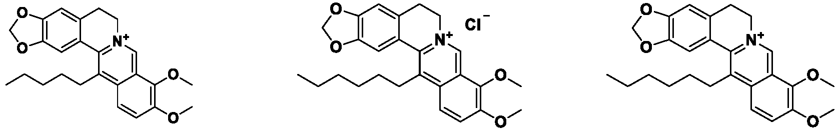

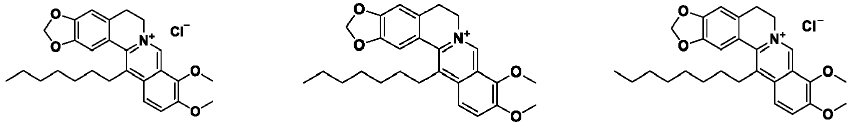

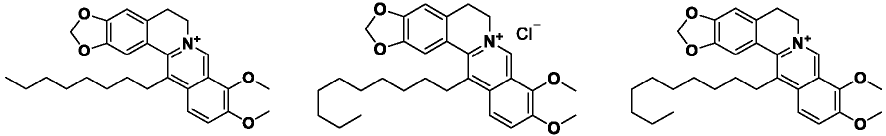

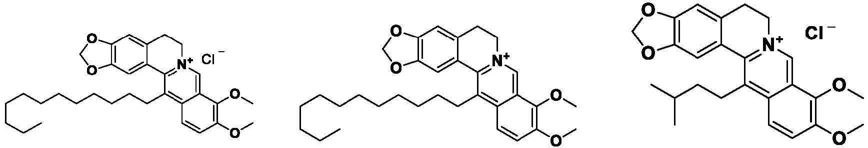

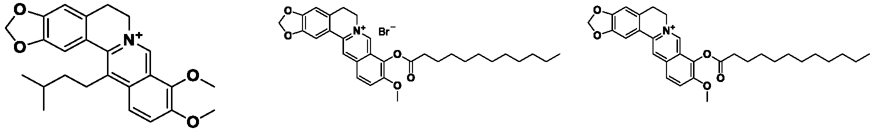

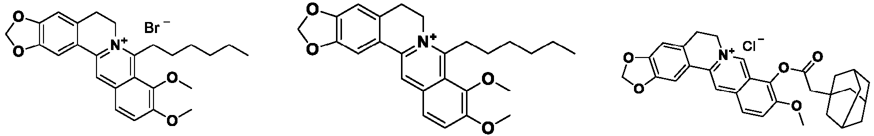

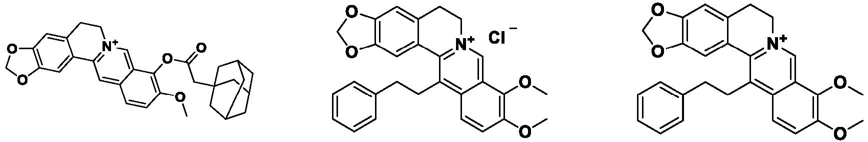

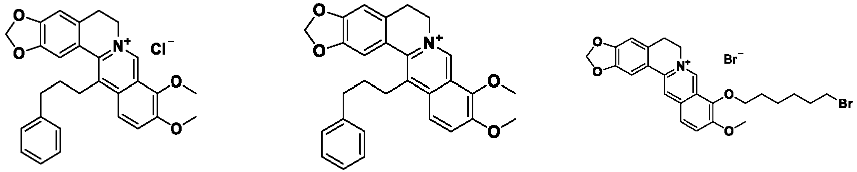

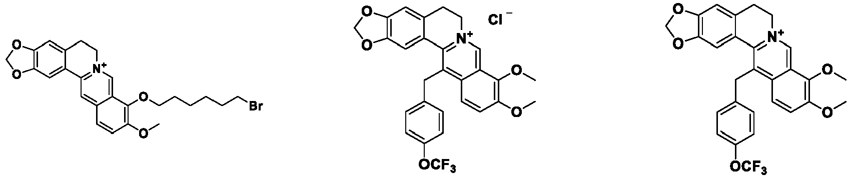

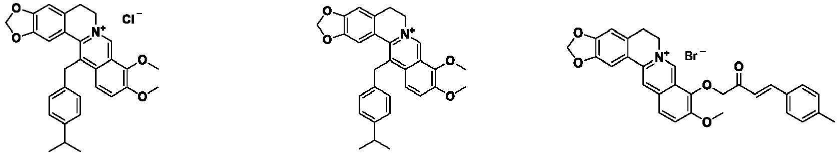

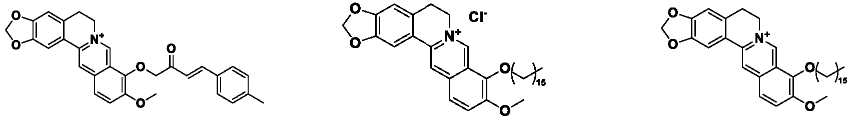

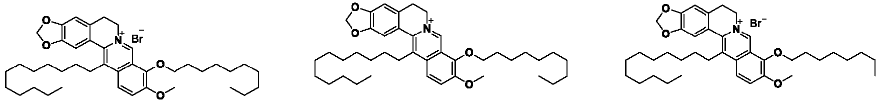

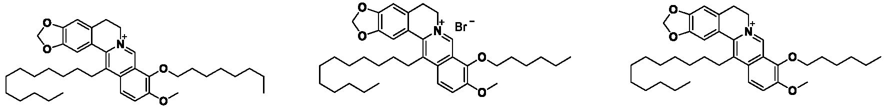

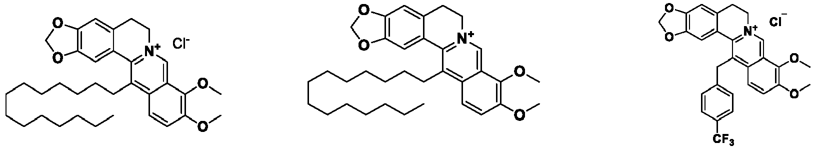

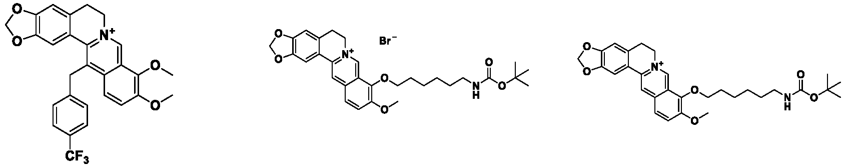

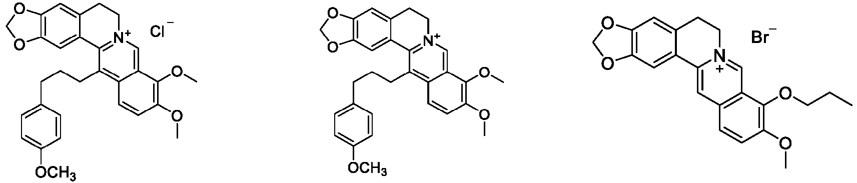

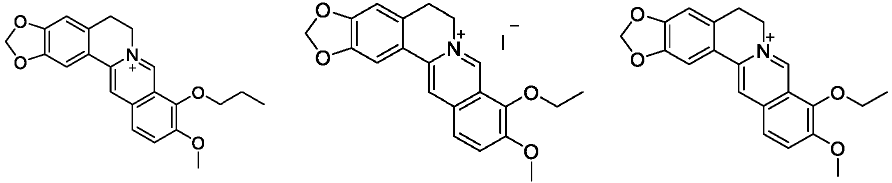

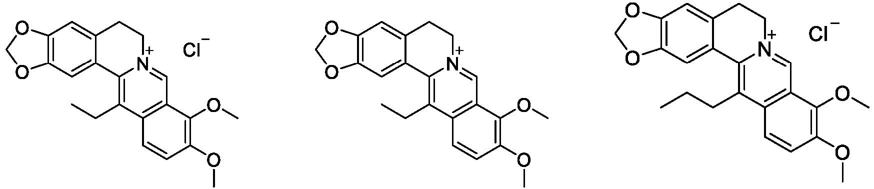

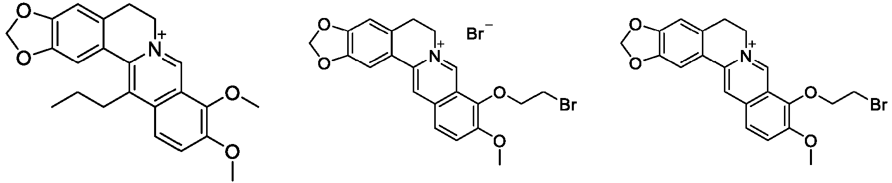

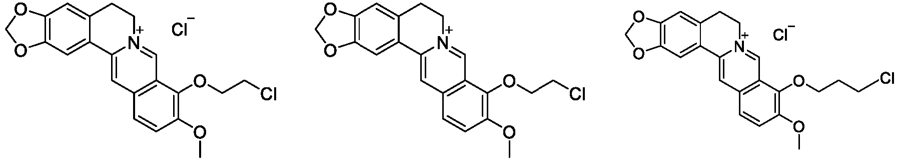

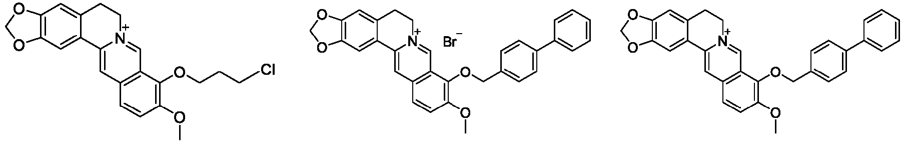

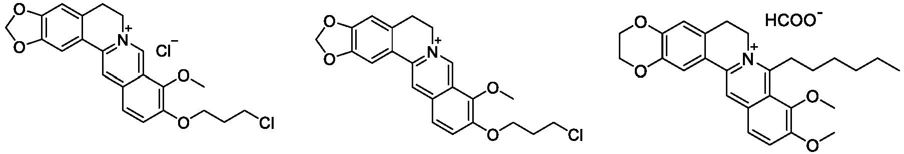

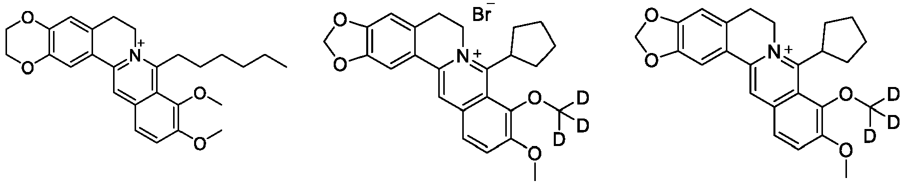

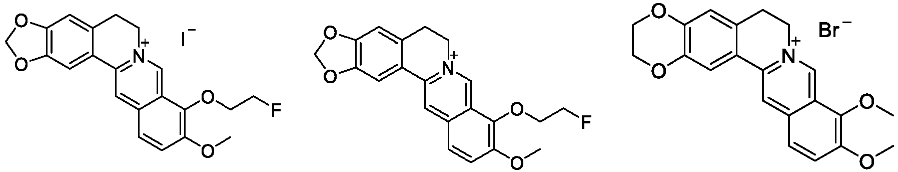

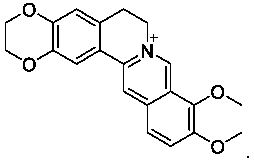

In another preferred embodiment, the pharmaceutically acceptable salt of the compound of formula I comprises the salt formed by the compound of formula I and an acid.

In another preferred embodiment, the acid comprises one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methane sulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises the salt root formed by the loss of a H⁺ in the acid.

In another preferred embodiment, the salt root of the pharmaceutically acceptable salt of the compound of formula I comprises the salt root formed by the loss of a H⁺ in hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises F⁻, Cl⁻, Br, I⁻, HCOO⁻, CH₃COO⁻, SO₄²⁻or NO₃⁻.

In another preferred embodiment, the pharmaceutically acceptable salt of the compound of formula I has the following structure: 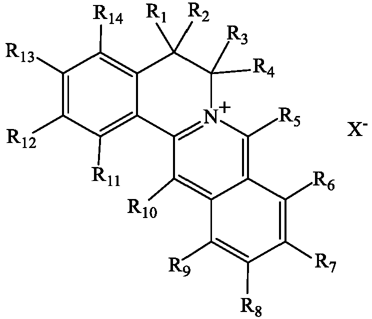 wherein,
X⁻ is salt root.

In another preferred embodiment, X⁻ is anionic salt root.

In another preferred embodiment, X⁻ is salt root formed by the loss of a H⁺ in the acid.

In another preferred embodiment, X⁻ is salt root formed by the loss of a H⁺ in hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, X⁻ comprises F⁻, Cl⁻, Br⁻, I⁻, HCOO⁻, CH₃COO⁻, SO₄²⁻or NO₃⁻. In another preferred embodiment, the tumor is human-derived tumor.

In another preferred embodiment, the tumor is human tumor.

In another preferred embodiment, the tumor comprises tumor with low or no expression of NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1.

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRFl.

In another preferred embodiment, the tumor comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the methylation of nucleotide site of NNMT gene comprises the methylation of cytosine nucleotide site of NNMT gene.

In another preferred embodiment, the methylation of nucleotide site of NNMT gene comprises the methylation of cytosine of NNMT gene.

In another preferred embodiment, the methylation of nucleotide site of NNMT gene comprises the methylation of the 5'carbon atom on the cytosine of the NNMT gene.

In another preferred embodiment, the tumor comprises tumor with high methylation level of DNA CpG site of NNMT gene.

In another preferred embodiment, the methylation of DNA CpG site of NNMT gene comprises the methylation of cytosine nucleotide site of DNA CpG site of NNMT gene.

In another preferred embodiment, the methylation of DNA CpG site of NNMT gene comprises the methylation of cytosine of DNA CpG site of NNMT gene.

In another preferred embodiment, the methylation of DNA CpG site of NNMT gene comprises the methylation of the 5'carbon atom on the cytosine of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same cell or a normal cell (e.g., para-tumor tissue cell).

In another preferred embodiment, the tumor with low or no expression of NNMT gene means the ratio (E1/E0) of the expression level E1 of NNMT gene in the tumor cell to the expression level E0 of NNMT gene in the same cell or a normal cell (e.g., para-tumor tissue cell) is < 1.0.

In another preferred embodiment, the low or no expression of NNMT gene means the ratio (E1/E0) of the expression E1 of NNMT gene in a cell ( e.g., tumor cell ) to the expression E0 of NNMT gene in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or high expression of NNMT gene.

In another preferred embodiment, the same cell refers to the same type of cell with normal or high expression of NNMT gene.

In another preferred embodiment, the normal cell refers to normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal or high expression of NNMT gene.

In another preferred embodiment, the cell with normal or high expression of NNMT gene comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same cell or a normal cell (e.g., para-tumor tissue cell).

In another preferred embodiment, the tumor with high expression of DNA methylase means the ratio (A1/A0) of the expression level A1 of DNA methylase in the tumor cell to the expression level A0 of DNA methylase in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low expression of DNA methylase.

In another preferred embodiment, the same cell refers to the same type of cell with normal or low expression of DNA methylase.

In another preferred embodiment, the normal cell refers to normal tissue cell (e.g., tumor origin cell, tumor-adjacent cell or para-tumor tissue cell) with normal expression of DNA methylase.

In another preferred embodiment, A0 refers to the expression level of DNA methylase in the cell with normal or low expression of DNA methylase.

In another preferred embodiment, the cell with normal or low expression of DNA methylase comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor cell is higher than that in the same cell or a normal cell (e.g., para-tumor tissue cell).

In another preferred embodiment, the tumor with high expression of DNMT1 means the ratio (B1/B0) of the expression level B1 of DNMT1 in the tumor cell to the expression level B0 of DNMT1 in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low expression of DNMT1.

In another preferred embodiment, the same cell refers to the same type of cell with normal or low expression of DNMT1.

In another preferred embodiment, the normal cell refers to normal tissue cell (e.g., tumor origin cell, tumor-adjacent cell or para-tumor tissue cell) with normal expression of DNMT1.

In another preferred embodiment, B0 refers to the expression level of DNMT1 in the cell with normal or low expression of DNMT1.

In another preferred embodiment, the cell with normal or low expression of DNMT1 comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNMT3a means that DNMT3a protein can be detected in 20 µg of protein extracted from tumor using DNMT3a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same cell or a normal cell (e.g., para-tumor tissue cell).

In another preferred embodiment, the tumor with high expression of DNMT3a means the ratio (C1/C0) of the expression level C1 of DNMT3a in the tumor cell to the expression level C0 of DNMT3a in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low expression of DNMT3a.

In another preferred embodiment, the same cell refers to the same type of cell with normal or low expression of DNMT3a.

In another preferred embodiment, the normal cell refers to normal tissue cell (e.g., tumor origin cell, tumor-adjacent cell or para-tumor tissue cell) with normal expression of DNMT3a.

In another preferred embodiment, C0 refers to the expression level of DNMT3a in the cell with normal or low expression of DNMT3a.

In another preferred embodiment, the cell with normal or low expression of DNMT3a comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor cell is higher than that in the same cell or a normal cell (e.g., para-tumor tissue cell).

In another preferred embodiment, the tumor with high expression of DNMT3b means the ratio (D1/D0) of the expression level D1 of DNMT3b in the tumor cell to the expression level D0 of DNMT3b in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low expression of DNMT3b.

In another preferred embodiment, the same cell refers to the same type of cell with normal or low expression of DNMT3b.

In another preferred embodiment, the normal cell refers to normal tissue cell (e.g., tumor origin cell, tumor-adjacent cell or para-tumor tissue cell) with normal expression of DNMT3b

In another preferred embodiment, D0 refers to the expression level of DNMT3b in the cell with normal or low expression of DNMT3b.

In another preferred embodiment, the cell with normal or low expression of DNMT3b comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF1 means the expression level of UHRF1 in tumor cell is higher than that in the same cell or a normal cell (e.g., para-tumor tissue cell).

In another preferred embodiment, the tumor with high expression of UHRF1 means the ratio (F1/F0) of the expression level F1 of UHRF1 in the tumor cell to the expression level F0 of UHRF1 in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low expression of UHRF1.

In another preferred embodiment, the same cell refers to the same type of cell with normal or low expression of UHRF1.

In another preferred embodiment, the normal cell refers to normal tissue cell (e.g., tumor origin cell, tumor-adjacent cell or para-tumor tissue cell) with normal expression of UHRF1

In another preferred embodiment, F0 refers to the expression level of UHRF1 in the cell with normal or low expression of UHRF1.

In another preferred embodiment, the cell with normal or low expression of UHRF1 comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the high methylation level of nucleotide site of NNMT gene means the methylation level of nucleotide site of NNMT gene in a cell ( e.g., tumor cell) is higher than that in the same cell or a normal cell (e.g., para-tumor tissue cell).

In another preferred embodiment, the high methylation level of nucleotide site of NNMT gene means the ratio (L1/L0) of the methylation level L1 of nucleotide site of NNMT gene in a cell ( e.g., tumor cell) to the methylation level L0 of nucleotide site of NNMT gene in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of nucleotide site of NNMT gene means the methylation level of nucleotide site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell ) with normal or low methylation level of nucleotide site of NNMT gene.

In another preferred embodiment, the same cell refers to the same type of cell with normal or low methylation level of nucleotide site of NNMT gene.

In another preferred embodiment, the normal cell refers to normal tissue cell (e.g., tumor origin cell, tumor-adjacent cell or para-tumor tissue cell) with normal methylation level of nucleotide site of NNMT gene.

In another preferred embodiment, the cell with normal or low methylation level of nucleotide site of NNMT gene comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the high methylation level of nucleotide site of NNMT gene means the methylation level (M%) of nucleotide site of NNMT gene in a cell ( e.g., tumor cell) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide site of one or more (e.g., 2, 3, 4, 5, 6, or 7 ) of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide site of one or more (e.g., 2, 3, 4, 5, 6, or 7 ) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell ( e.g., tumor cell ) is higher than that in the same cell or a normal cell (e.g., para-tumor tissue cell).

In another preferred embodiment, the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of DNA CpG site of NNMT gene means the methylation level of DNA CpG site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same cell comprises the same type of cell.

In another preferred embodiment, the same cell comprises the same type of tumor cell.

In another preferred embodiment, the same cell refers to the cell ( e.g., the same type of tumor cell) with normal or low methylation level of DNA CpG site of NNMT gene.

In another preferred embodiment, the same cell refers to the same type of cell with normal or low methylation level of DNA CpG site of NNMT gene.

In another preferred embodiment, the normal cell refers to normal tissue cell (e.g., tumor origin cell, tumor-adjacent cell or para-tumor tissue cell) with normal methylation level of DNA CpG site of NNMT gene.

In another preferred embodiment, the cell with normal or low methylation level of DNA CpG site of NNMT gene comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the high methylation level of DNA CpG site of NNMT gene means the methylation level (M%) of DNA CpG site of NNMT gene in a cell ( e.g., tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated DNA CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated DNA CpG sites to the number of all DNA CpG sites in the NNMT gene.

In another preferred embodiment, the methylation level of DNA CpG site of NNMT gene refers to the ratio of the number of methylated DNA CpG nucleotides to the number of all DNA CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of DNA CpG site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of the site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the NNMT gene inhibitor is administered to achieve low or no expression of the NNMT gene in tumor.

In another preferred embodiment, the DNA methylase promoter is administered to achieve high expression of DNA methylase in tumor.

In another preferred embodiment, the DNMT1 promoter is administered to achieve high expression of DNMT1 in tumor.

In another preferred embodiment, the DNMT3a promoter is administered to achieve high expression of DNMT3a in tumor.

In another preferred embodiment, the DNMT3b promoter is administered to achieve high expression of DNMT3b in tumor.

In another preferred embodiment, the UHRF1 promoter is administered to achieve high expression of UHRF1 in tumor.

In another preferred embodiment, the methylation promoter of nucleotide site of NNMT gene is administered to achieve high methylation level of nucleotide site of NNMT gene in tumor.

In another preferred embodiment, the methylation promoter of DNA CpG site of NNMT gene is administered to achieve high methylation level of DNA CpG site of NNMT gene in tumor.

In another preferred embodiment, the inhibitor comprises specific inhibitor.

In another preferred embodiment, the promoter comprises specific promoter.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNMT1 promoter comprises promoter that can achieve high expression of DNMT1 in tumor.

In another preferred embodiment, the DNMT3a promoter comprises promoter that can achieve high expression of DNMT3a in tumor.

In another preferred embodiment, the DNMT3b promoter comprises promoter that can achieve high expression of DNMT3b in tumor.

In another preferred embodiment, the UHRF1 promoter comprises promoter that can achieve high expression of UHRF1 in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation promoter of DNA CpG site of NNMT gene in tumor.

In another preferred embodiment, the tumor is selected from the group consisting of lung cancer, renal carcinoma, breast cancer, colon cancer, lymphoma, leukemia, pancreatic cancer, brain tumor, liver cancer, prostate cancer, and combinations thereof.

In another preferred embodiment, the lung cancer is selected from the group consisting of non-small cell lung cancer, small cell lung cancer, and combinations thereof.

In another preferred embodiment, the lung cancer cell comprises NCI-H82 cell.

In another preferred embodiment, the colon cancer comprises colon adenocarcinoma.

In another preferred embodiment, the colon cancer cell comprises SW48 cell.

In another preferred embodiment, the breast cancer cell comprises MDA-MB453 cell.

In another preferred embodiment, the breast cancer comprises triple negative breast cancer.

In another preferred embodiment, the lymphoma is selected from the group consisting of B-cell lymphoma, skin T-cell lymphoma, and combinations thereof.

In another preferred embodiment, the lymphoma comprises diffuse large B-cell lymphoma.

In another preferred embodiment, the brain tumor is selected from the group consisting of brain glioblastoma, neuroglioma, brain medulloblastoma, brain neuroblastoma, and combination thereof.

In another preferred embodiment, the brain medulloblastoma comprises cerebellar medulloblastoma.

In another preferred embodiment, the brain glioblastoma comprises glioblastoma multiforme.

In another preferred embodiment, the brain tumor comprises glioblastoma.

In another preferred embodiment, the brain tumor cell comprises one or more of GB-1 cell, SF126 cell, D341 Med cell, Kelly cell and NB-1 cell.

In another preferred embodiment, the renal carcinoma is selected from the group consisting of clear cell renal cell adenocarcinoma, renal carcinoma Wilms, and combination thereof.

In another preferred embodiment, the renal carcinoma comprises clear cell renal cell adenocarcinoma.

In another preferred embodiment, the renal carcinoma comprises renal carcinoma Wilms.

In another preferred embodiment, the renal carcinoma cell comprises renal carcinoma Wilms cell.

In another preferred embodiment, the renal carcinoma cell comprises one or more of G-401 cell and 786-O cell.

In another preferred embodiment, the pancreatic cancer comprises pancreatic ductal carcinoma.

In another preferred embodiment, the pancreatic cancer cell comprises CGPAC cell.

In another preferred embodiment, the leukemia is selected from the group consisting of T-lymphocyte leukemia, myeloid leukemia, and combinations thereof.

In another preferred embodiment, the T-lymphocytic leukemia comprises acute T-lymphocytic leukemia.

In another preferred embodiment, the myeloid leukemia comprises type M4 of acute myeloid leukemia.

In another preferred embodiment, the myeloid leukemia comprises FAB type M4 of acute myeloid leukemia.

In another preferred embodiment, the expression comprises protein expression and/or mRNA expression.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the expression is mRNA expression or protein expression.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the second aspect of the present invention, it provides a marker for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor, the marker comprises the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the methylation level of DNA CpG site of NNMT gene comprises the methylation level of DNA CpG site in promoter region of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" comprises "the patient tumor is sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" comprises "the patient tumor is not sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, the tumor with low or no expression of NNMT gene is as described in the first aspect of the invention.

In another preferred embodiment, the DNA methylase is selected from the group consisting of DNMT1, DNMT3a, DNMT3b, and combinations thereof.

In another preferred embodiment, the tumor with high expression of DNA methylase (e.g., DNMT1) is as described in the first aspect of the invention.

In another preferred embodiment, the tumor with high expression of UHRF1 is as described in the first aspect of the invention.

In another preferred embodiment, the tumor with high methylation level of nucleotide site of NNMT gene is as described in the first aspect of the invention.

In another preferred embodiment, the tumor with high methylation level of DNA CpG site of NNMT gene is as described in the first aspect of the invention.

In another preferred embodiment, the high expression of NNMT gene means the ratio (E1/E0) of the expression E1 of NNMT gene in a cell ( e.g., tumor cell) to the expression E0 of NNMT gene in the same cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the tumor with low expression of DNA methylase means the ratio (A1/A0) of the expression level A1 of DNA methylase in the tumor cell to the expression level A0 of DNA methylase in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT1 means the ratio (B1/B0) of the expression level B1 of DNMT1 in the tumor cell to the expression level B0 of DNMT1 in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3a means the ratio (C1/C0) of the expression level C1 of DNMT3a in the tumor cell to the expression level C0 of DNA methylase in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3b means the ratio (D1/D0) of the expression level D1 of DNMT3b in the tumor cell to the expression level D0 of DNA methylase in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell ( e.g., tumor cell) to the methylation level L0 of nucleotide site of NNMT gene in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In the third aspect of the present invention, it provides a detection kit, which comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the level comprises protein level and/or mRNA level.

In another preferred embodiment, the expression comprises mRNA expression and/or protein expression.

In the fourth aspect of the present invention, it provides a use of the detection kit according to the third aspect of the present invention in the preparation of concomitant diagnose kit for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" is as described in the second aspect of the invention.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" is as described in the second aspect of the invention.

In the fifth aspect of the present invention, it provides a medicine kit, which comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the expression level of NNMT gene, the expression level of DNA methylase, the expression level of UHRF1, the methylation level of nucleotide site of NNMT gene, and/or the methylation level of DNA CpG site of NNMT gene comprises the expression level of NNMT gene, the expression level of DNA methylase, the expression level of UHRF1, the methylation level of nucleotide site of NNMT gene, and/or the methylation level of DNA CpG site of NNMT gene in the tumor.

In another preferred embodiment, the detection sample comprises tumor.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

In the sixth aspect of the present invention, it provides a method for preventing and/or treating tumor, which comprises administering the compound of formula I, or an optical isomer thereof, or a racemate thereof, or a solvate thereof, or a pharmaceutically acceptable salt thereof, or a deuterated compound thereof according to the first aspect of the present invention to a subject in need.

In another preferred embodiment, the tumor is as described in the first aspect of the invention.

In another preferred embodiment, the subject is human and non-human mammals (rodent, rabbit, monkey, livestock, dog, cat, etc.).

In another preferred embodiment, the method comprises:
firstly achieving low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the method comprises:
firstly administering NNMT gene inhibitor, DNA methylase promoter, UHRF1 promoter, methylation promoter of nucleotide site of NNMT gene, and/or methylation promoter of DNA CpG site of NNMT gene to achieve low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In the seventh aspect of the present invention, it provides a device or system, the device or system comprises:
(i) a detection module, the detection module is used to detect the expression level of NNMT gene, the expression level of DNA methylase, the expression level of UHRF1, the methylation level of nucleotide site of NNMT gene, and/or the methylation level of DNA CpG site of NNMT gene;
(ii) an output module, the output module comprises the information as follows:
   the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

In another preferred embodiment, the detection sample comprises tumor.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In another preferred embodiment, the expression level of NNMT gene and the methylation level of DNA CpG site in promoter region of NNMT gene can be obtained by the procession of the data processing module.

In the eighth aspect of the present invention, it provides a use of NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNA methylase is selected from the group consisting of DNMT1, DNMT3a, DNMT3b, and combinations thereof.

In another preferred embodiment, the DNA methylase promoter comprises DNMT1 promoter.

In another preferred embodiment, the DNMT1 promoter comprises promoter that can achieve high expression of DNMT1 in tumor.

In another preferred embodiment, the DNA methylase promoter comprises DNMT3a promoter.

In another preferred embodiment, the DNMT3a promoter comprises promoter that can achieve high expression of DNMT3a in tumor.

In another preferred embodiment, the DNA methylase promoter comprises DNMT3b promoter.

In another preferred embodiment, the DNMT3b promoter comprises promoter that can achieve high expression of DNMT3b in tumor.

In another preferred embodiment, the UHRF1 promoter comprises promoter that can achieve high expression of UHRF1 in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation level of nucleotide site of NNMT gene in tumor.

In another preferred embodiment, the inhibitor comprises specific inhibitor.

In another preferred embodiment, the promoter comprises specific promoter.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the tumor is as described in the first aspect of the invention.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the ninth aspect of the present invention, it provides an active ingredient combination, the active ingredient combination comprises:
(1) a first active ingredient, the first active ingredient comprises anti-tumor drug; and
(2) a second active ingredient, the second active ingredient comprises NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the molar ratio of the first active ingredient to the second active ingredient is 0.01-600:1, preferably 0.05-500:1, more preferably 0.1-400:1, more preferably 0.2-200:1, more preferably 0.5-100:1, more preferably 0.5-80:1, most preferably 1-50:1.

In another preferred embodiment, at least one active ingredient is independent in the active ingredient combination.

In another preferred embodiment, the first active ingredient and the second active ingredient are independent of each other in the active ingredient combination.

In the tenth aspect of the present invention, it provides a composition, the composition comprises:
(1) a first active ingredient, the first active ingredient comprises anti-tumor drug; and
(2) a second active ingredient, the second active ingredient comprises NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the composition is a pharmaceutical composition.

In another preferred embodiment, the pharmaceutical composition further comprises a pharmaceutically acceptable carrier.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In the eleventh aspect of the present invention, it provides a medicine kit, the medicine kit comprises:
(A) a first preparation comprising a first active ingredient, the first active ingredient comprises anti-tumor drug; and
(B) a second preparation comprising a second active ingredient, the second active ingredient comprises NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug is the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anti-tumor drug.

In another preferred embodiment, the method for using together means the second preparation comprising NNMT gene inhibitor, DNA methylase promoter, UHRF1 promoter, methylation promoter of nucleotide site of NNMT gene, and/or methylation promoter of DNA CpG site of NNMT gene is administered firstly, then the anti-tumor drug is administered.

In the twelfth aspect of the present invention, it provides a method for inhibiting tumor cell, which comprises contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method is vitro method.

In another preferred embodiment, the method is non-therapeutic and non-diagnostic method. In another preferred embodiment, the contact is performed in vitro culture.

In another preferred embodiment, the tumor is as described in the first aspect of the invention. In another preferred embodiment, the method comprises:
firstly achieving low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention to inhibit tumor cell, thereby inhibiting tumor cell.

In another preferred embodiment, the method comprises:
firstly administering NNMT gene inhibitor, DNA methylase promoter, UHRF1 promoter, methylation promoter of nucleotide site of NNMT gene, and/or methylation promoter of DNA CpG site of NNMT gene to achieve low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In the thirteenth aspect of the present invention, it provides a use of medicine kit according to the fifth aspect of the present invention in the preparation of medicine box for preventing and/or treating tumor.

In another preferred embodiment, the medicine box further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

In the fourteenth aspect of the present invention, it provides a compound of formula I, or an optical isomer thereof, or a racemate thereof, or a solvate thereof, or a pharmaceutically acceptable salt thereof, or a deuterated compound thereof; 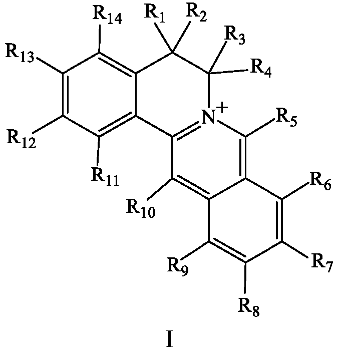 wherein,
R₁, R₂, R₃ and R₄ are each independently hydrogen, substituted or unsubstituted C1-C6 alkyl;
R₅ is hydrogen, substituted or unsubstituted C1-C10 alkyl, substituted or unsubstituted C3-C10 cycloalkyl;
R₆ is -O-R₁₅ or -N (R₁₆R₁₇);
R₇ is hydrogen, substituted or unsubstituted C1-C6 alkoxyl, substituted or unsubstituted C1-C8 haloalkoxyl;
R₈ is hydrogen, substituted or unsubstituted C1-C6 alkyl, halogen;
R₉ is hydrogen, substituted or unsubstituted C1-C6 alkyl, halogen;
R₁₀ is hydrogen, substituted or unsubstituted C1-C16 alkyl, substituted or unsubstituted C6-C12 aryl-substituted or unsubstituted C1-C6 alkyl-, substituted or unsubstituted C1-C6 alkyl-substituted or unsubstituted C6-C12 aryl-C1-C6 alkyl-, substituted or unsubstituted C1-C6 haloalkoxyl-substituted or unsubstituted C6-C12 aryl-substituted or unsubstituted C1-C6 alkyl-, substituted or unsubstituted C1-C6 haloalkyl-substituted or unsubstituted C6-C12 aryl-substituted or unsubstituted C1-C6 alkyl-, substituted or unsubstituted C1-C6 alkoxyl-substituted or unsubstituted C6-C12 aryl-substituted or unsubstituted C1-C6 alkyl-;
R₁₁ and R₁₄ are each independently hydrogen, substituted or unsubstituted C1-C6 alkyl;
R₁₂ and R₁₃ are connected to form a substituted or unsubstituted 3-10 membered heterocycloalkane ring;
R₁₅ is substituted or unsubstituted C1-C18 alkyl, deuterated C1-C18 alkyl, substituted or unsubstituted C6-C12 aryl, substituted or unsubstituted C6-C12 aryl-substituted or unsubstituted C6-C12 aryl-substituted or unsubstituted C1-C8 alkyl-, substituted or unsubstituted C6-C12 aryl-O-substituted or unsubstituted C1-C6 alkyl-, substituted or unsubstituted C1-C10 haloalkyl, substituted or unsubstituted C2-C16 acyl, substituted or unsubstituted C2-C8 alkenyl-substituted or unsubstituted C1-C4 alkyl-substituted or unsubstituted C2-C8 alkenyl-substituted or unsubstituted C1-C4 alkyl-, substituted or unsubstituted C3-C14 cycloalkyl-substituted or unsubstituted C2-C6 acyl-, substituted or unsubstituted C6-C12 aryl-substituted or unsubstituted C2-C8 alkenyl-C(O)-substituted or unsubstituted C1-C4 alkyl-, or 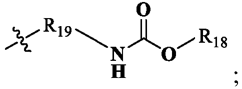
R₁₆ and R₁₇ are each independently hydrogen, substituted or unsubstituted C1-C12 alkyl, substituted or unsubstituted C6-C12 aryl-substituted or unsubstituted C1-C4 alkyl-;
R₁₈ is substituted or unsubstituted C1-C8 alkyl;
R₁₉ is substituted or unsubstituted C1-C10 alkylidene.

In another preferred embodiment, the compound of formula I, or an optical isomer thereof, or a racemate thereof, or a solvate thereof, or a pharmaceutically acceptable salt thereof, or a deuterated compound thereof is as described in the first aspect of the invention.

In another preferred embodiment, the compound of formula I, or an optical isomer thereof, or a racemate thereof, or a solvate thereof, or a pharmaceutically acceptable salt thereof, or a deuterated compound thereof is selected from the following group:

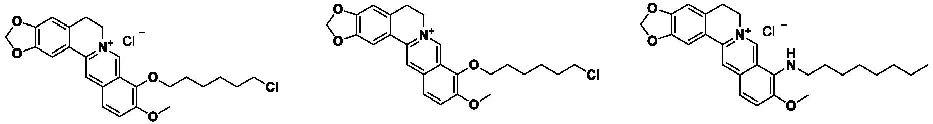

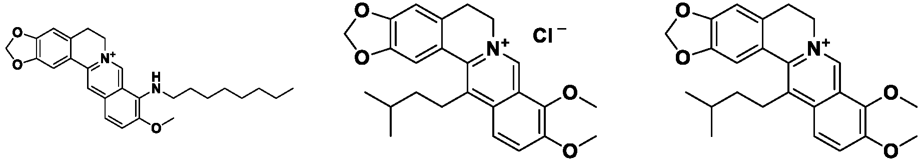

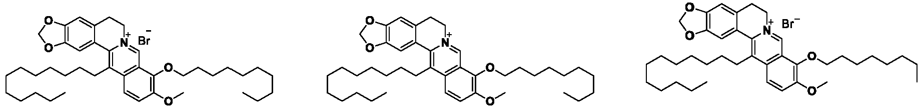

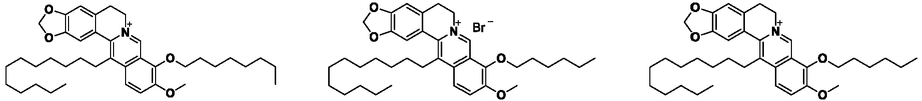

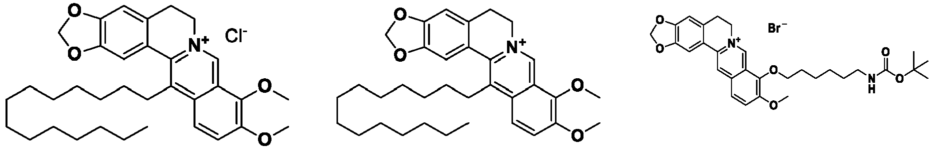

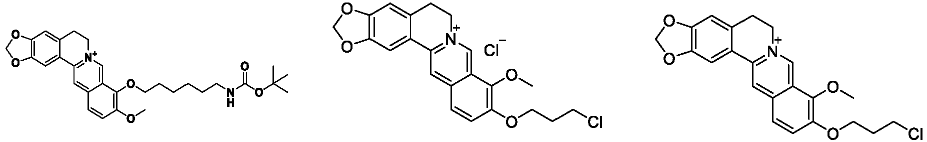

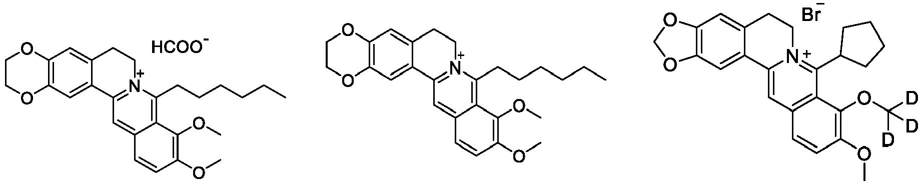

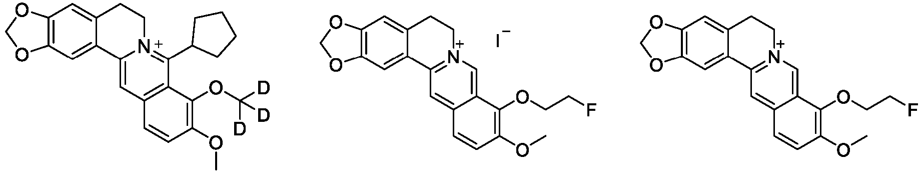

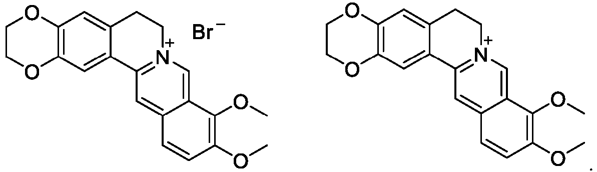

It should be understood that, in the present invention, each of the technical features specifically described above and below (such as those in the Examples) can be combined with each other, thereby constituting new or preferred technical solutions which need not be redundantly described one-by-one.

### DESCRIPTION OF THE DRAWINGS

Fig.1 is the ¹H NMR spectrum of compound AB31755.
Fig.2 is the ¹H NMR spectrum of compound AB31756.
Fig.3 is the ¹H NMR spectrum of compound AB35483.
Fig.4 is the ¹H NMR spectrum of compound AB31811.
Fig.5 shows the expression of NNMT gene in tumor cells sensitive and insensitive to the compounds prepared in the example.
Fig. 6 shows the methylation level of DNA CpG site of promoter region of NNMT gene in tumor cells sensitive and insensitive to the compounds prepared in the example.
Fig.7 shows the methylation level of DNA CpG sites from 1050 bp before the transcription start site to 499 bp after the transcription start site in NNMT gene in tumor cells sensitive and insensitive to the compounds prepared in the example.
Fig.8 shows the methylation level of DNA CpG sites from 1050 bp to 193 bp before the transcription start site in NNMT gene in tumor cells sensitive and insensitive to the compounds prepared in the example.
Fig.9 shows the methylation level of DNA CpG sites of specific NNMT gene (ie, site 114165695, site 114165730, site 114165769, site 114165804, site 114165938, site 114166050, site 114166066 on human chromosome 11) in tumor cells sensitive and insensitive to the compounds prepared in the example, black dot indicates that the relevant site is methylated, white dot indicates that the relevant site is not methylated, SST refers to the transcription starting site, and Chr11 refers to human chromosome 11 according to human genome version GCF_ 00000 1405.25 (GRCh37. p13).
Fig. 10 shows the expression of NNMT gene in brain tumor cells sensitive and insensitive to the compounds prepared in the example.

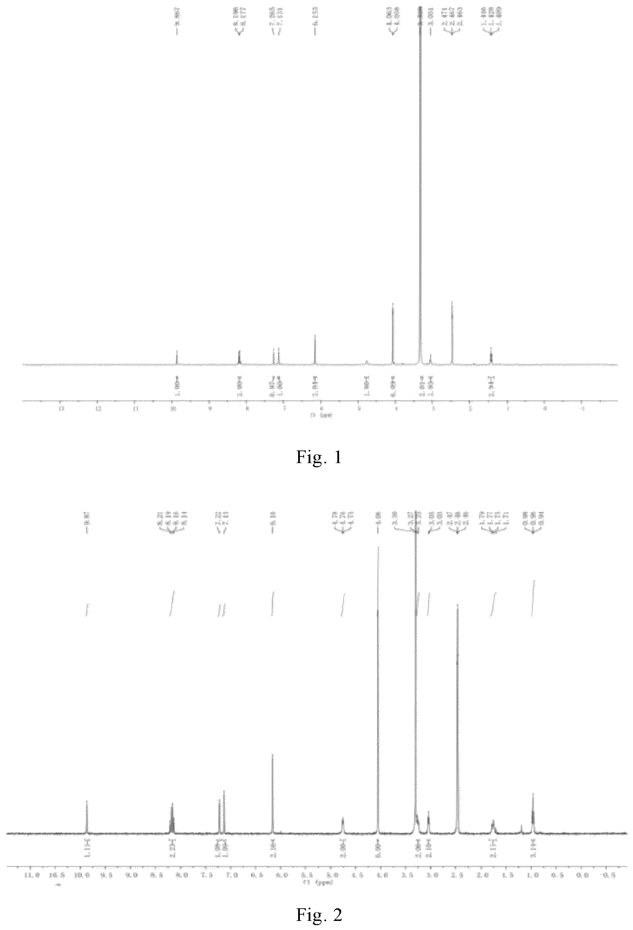

Fig. 1 1 shows the methylation level of DNA CpG site of promoter region of NNMT gene in brain tumor cells sensitive and insensitive to the compounds prepared in the example.

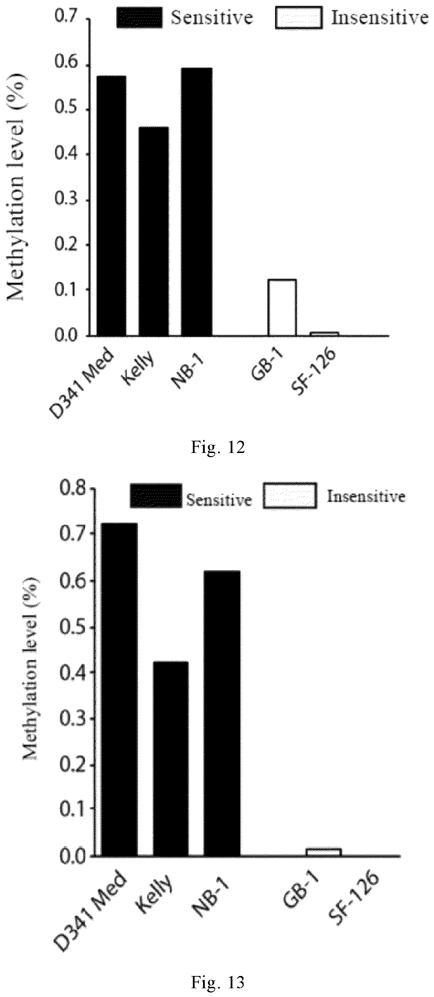

Fig. 12 shows the methylation level of DNA CpG sites from 1050 bp before the transcription start site to 499 bp after the transcription start site in NNMT gene in brain tumor cells sensitive and insensitive to the compounds prepared in the example.
Fig. 13 shows the methylation level of DNA CpG sites from 1050 bp to 193 bp before the transcription start site in NNMT gene in brain tumor cells sensitive and insensitive to the compounds prepared in the example.

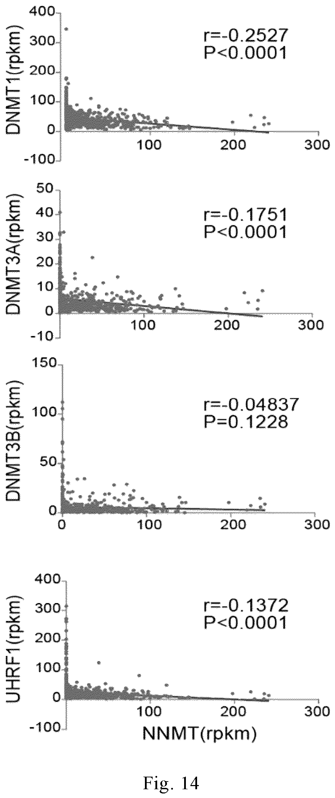

Fig.14 shows the correlation between the expression of NNMT and the expression of DNMT1, UHRF1, DNMT3a and DNMT3b in tumor cells.

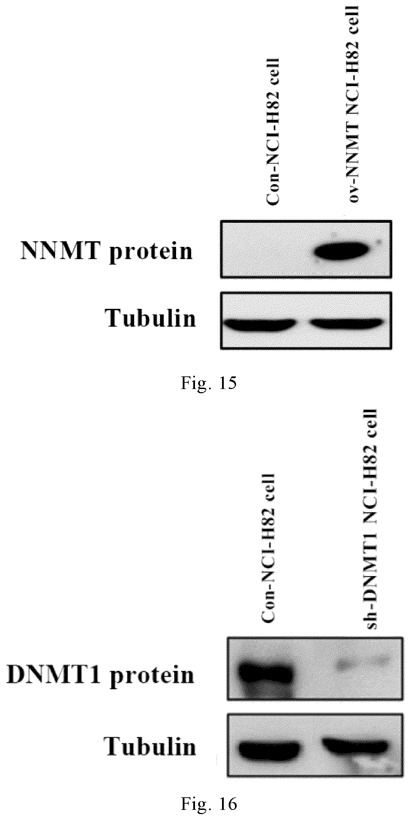

Fig. 15 shows the expression content of NNMT protein in Con-NCI-H82 cell and ov-NNMT NCI-H82 cell using Western Blot test, wherein, Con-NCI-H82 refers to the expression content of NNMT protein in NCI-H82 cell transfected with empty virus vector without carrying NNMT gene and shRNA, which is used as control; ov-NNMT NCI-H82 refers to the expression content of NNMT protein in NCI-H82 cell transfected with virus vector carrying NNMT gene.
Fig. 16 shows the expression content of DNMT1 protein in Con-NCI-H82 cell and sh-DNMT1 NCI-H82 cell using Western Blot test, wherein, Con-NCI-H82 refers to the expression content of DNMT1 protein in NCI-H82 cell transfected with empty virus vector without carrying DNMT1 gene and shRNA, which is used as control; sh-DNMT1 NCI-H82 refers to the expression content of DNMT1 protein in NCI-H82 cell transfected with virus vector carrying shRNA.

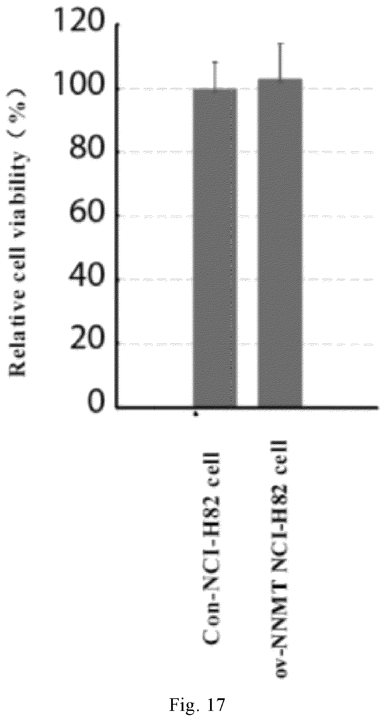

Fig. 17 shows the relative cell viability of Con-NCI-H82 cell and ov-NNMT NCI-H82 cell, wherein, Con-NCI-H82 refers to the cell viability of NCI-H82 cell transfected with empty virus vector without carrying NNMT gene and shRNA, which is used as control; ov-NNMT NCI-H82 refers to the cell viability of NCI-H82 cell transfected with virus vector carrying NNMT gene.

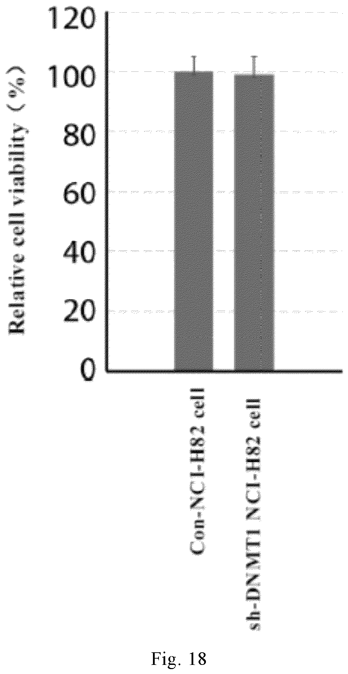

Fig. 18 shows the relative cell viability of Con-NCI-H82 cell and sh-DNMT1 NCI-H82 cell, wherein, Con-NCI-H82 refers to the cell viability of NCI-H82 cell transfected with empty virus vector without carrying NNMT gene and shRNA, which is used as control; sh-DNMT1 NCI-H82 refers to the cell viability of NCI-H82 cell transfected with virus vector carrying shRNA.

### EMBODIMENTS FOR CARRYING OUT THE INVENTION

Based on an extensive and intensive research, the inventors have unexpectedly found the compound of present invention has significant inhibitory effect on tumor with low or no expression of NNMT gene, high expression of DNA methylase, high expression of UHRFl, high methylation level of nucleotide site of NNMT gene, and/or high methylation level of DNA CpG site of NNMT gene. The expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of present invention is suitable for use in the prevention and/or treatment of patient tumor. On this basis, the inventors has completed the present invention.

### Terms

As used herein, the term "comprise", " comprising", and "containing" are used interchangeably, which not only comprise closed definitions, but also semi-closed and open definitions. In other words, the term comprises "consisting of" and "essentially consisting of".

As used herein, the term "high methylation level of DNA CpG site", "high level of DNA CpG site methylation" and "high methylation of DNA CpG site" are used interchangeably.

As used herein, the term "low methylation level of DNA CpG site", "low level of DNA CpG site methylation" and "low methylation of DNA CpG site" are used interchangeably.

As used herein, the term "high methylation level of nucleotide site", "high level of nucleotide site methylation" and "high methylation of nucleotide site" are used interchangeably.

As used herein, the term "low methylation level of nucleotide site", "low level of nucleotide site methylation" and "low methylation of nucleotide site" are used interchangeably.

As used herein, the term "IC50" and "IC₅₀" are used interchangeably, which refers to 50% inhibiting concentration, ie, the concentration of the inhibitor when 50% inhibitory effect is achieved.

As used herein, the term "methylation of CpG site", "methylation of CpG nucleotide" and "CpG methylation" are used interchangeably.

As used herein, the term "P/S" refers to adding penicillin and streptomycin into the culture medium.

As used herein, the term "a cell" refers to a cell (e.g., single tumor cell) or a group of cells containing multiple similar cells ((e.g., a tumor tissue).

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, "the expression level of NNMT gene, the expression level of DNA methylase, the expression level of UHRF1, the methylation level of nucleotide site of NNMT gene, and/or the methylation level of DNA CpG site of NNMT gene" refers to one or more of the expression level of NNMT gene, the expression level of DNA methylase, the expression level of UHRF1, the methylation level of nucleotide site of NNMT gene and the methylation level of DNA CpG site of NNMT gene.

As used herein, "the low or no expression of NNMT gene, high expression of DNA methylase, high expression of UHRFl, high methylation level of nucleotide site of NNMT gene, and/or high methylation level of DNA CpG site of NNMT gene" refers to one or more of the low or no expression of NNMT gene, high expression of DNA methylase, high expression of UHRFl, high methylation level of nucleotide site of NNMT gene and high methylation level of DNA CpG site of NNMT gene.

As used herein, "the high expression of NNMT gene, low expression of DNA methylase, low expression of UHRFl, low methylation level of nucleotide site of NNMT gene, and/or low methylation level of DNA CpG site of NNMT gene" refers to one or more of the high expression of NNMT gene, low expression of DNA methylase, low expression of UHRFl, low methylation level of nucleotide site of NNMT gene and low methylation level of DNA CpG site of NNMT gene.

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_ 000001405.25 (GRCh37. p13).

As used herein, the term "human chromosome 11" refers to human chromosome 11 according to human genome version GCF_ 000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide of site 114165695 on human chromosome 11; "site 114165730 on human chromosome 11" refers to nucleotide of site 114165730 on human chromosome 11; "site 114165769 on human chromosome 11" refers to nucleotide of site 114165769 on human chromosome 11; "site 114165804 on human chromosome 11" refers to nucleotide of site 114165804 on human chromosome 11; "site 114165938 on human chromosome 11" refers to nucleotide of site 114165938 on human chromosome 11; "site 114166050 on human chromosome 11" refers to nucleotide of site 114166050 on human chromosome 11; "site 114166066 on human chromosome 11" refers to nucleotide of site 114166066 on human chromosome 11.

As used herein, the gene expression comprises the protein expression of the gene and/or the mRNA expression of the gene.

As used herein, the term "DNMT3a" and "DNMT3A" are used interchangeably, which refers to DNA methyltransferase 3a.

As used herein, the term "DNMT3b" and "DNMT3B" are used interchangeably, which refers to DNA methyltransferase 3b.

As used herein, the term "DNMT1" refers to DNA methyltransferase 1.

As used herein, the term "UHRF1" refers to ubiquitin-like with PHD and ring finger domain 1.

As used herein, the term "MS-ESI" refers to Electrospray Ionization Mass Spectrometry.

As used herein, the term "1H NMR" and "¹H NMR" are used interchangeably, which refers to H Nuclear Magnetic Resonance Spectra.

As used herein, the term "deuterated" refers to one or more hydrogen atoms in a compound or group are substituted by deuterium. The deuterated can be mono-substituted, di-substituted, multi-substituted or fully-substituted.

As used herein, the term "solvate" refers to a complex formed by the coordination of a compound with a solvent molecule in a specific ratio.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc.

As used herein, "R₁", "R1" and "R¹" have the same meaning and can be used interchangeably, the other similar definitions have the same meaning. As used herein, " 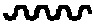 " denotes the linking site of the group.

As used herein, the term "alkyl" refers to a saturated hydrocarbon group with linear chain (ie, unbranched chain) or branched chain, or a combination of linear and branched chains, the alkyl only have carbon and hydrogen atoms. When the number of carbon atoms is limited in front of the alkyl (e.g., C1-C6 alkyl), it refers to the number of carbon atoms (e.g., 1-6) in the alkyl, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "alkylidene" refers to a group formed by removing one hydrogen on the alkyl, the alkyl is as defined above. When the number of carbon atoms is limited in front of the alkylidene (e.g., C1-C6 alkylidene), it refers to the number of carbon atoms (e.g., 1-6) in the alkylidene, for example, C1-C4 alkylidene refers to an alkylidene having 1-4 carbon atoms. Representative examples comprise but are not limited to methylidene, ethylidene, propylidene, isopropylidene, butylidene, isobutylidene, sec-butylidene, tert-butylidene, or the like.

As used herein, the term "alkenyl" refers to a hydrocarbon group formed by the loss of one hydrogen atom connected to the double bond in linear or branched alkene with one or more double bonds. When the number of carbon atoms is limited in front of the alkenyl (e.g., C2-C6 alkenyl), it refers to the number of carbon atoms (e.g., 1-6) in the alkenyl, for example, C2-C4 alkenyl refers to an alkenyl having 2-4 carbon atoms in the alkenyl. Representative examples comprise but are not limited to ethylenyl (e.g.,CH₂=CH-), butenyl (e.g.,C (CH₃) ₂=CH-), or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the haloalkyl (e.g., C1-C8 haloalkyl), it refers to the number of carbon atoms (e.g., 1-8) in the haloalkyl, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it refers to the number of ring carbon atoms (e.g., 3-12) in the cycloalkyl. In some preferred embodiments, C3-C8 cycloalkyl refers to a saturated or partially saturated monocycloalkyl or dicycloalkyl having 3-8 ring carbon atoms, comprising cyclopropyl, cyclobutyl, cyclopentyl, cycloheptyl, or the like. The term "spirocycloalkyl" refers to a bicyclic or polycyclic group that shares a carbon atom (referred to spiro-atom) between single rings, the spirocycloalkyl can contain one or more double bonds, but no ring has a fully conjugated π electron system. Fused cycloalkyl refers to all carbon bicyclic or polycyclic group in which each rings in the system shares an adjacent pair of carbon atoms with other rings, one or more rings can have one or more double bonds, but no ring has a fully conjugated π electron system. Bridged cycloalkyl refers to all carbon polycyclic group in which any two rings share two non-directly connected carbon atoms, the bridged cycloalkyl can have one or more double bonds, but no ring has a fully conjugated π electron system. The representative examples of cycloalkyl comprise but are not limited to

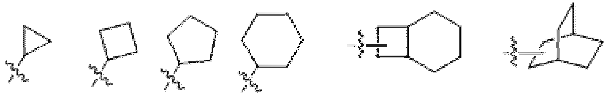

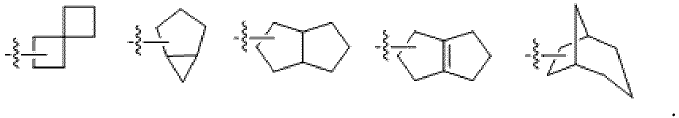

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the halocycloalkyl (e.g, C3-C8 halocycloalkyl), it refers to the number of ring carbon atoms (e.g., 3-8) in the halocycloalkyl, for example, C3-C8 halocycloalkyl refers to an halocycloalkyl having 3-8 ring carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples comprise but are not limited to monofluoromethoxyl, monofluoroethoxyl, bisfluorobutoxyl, FsC-O-, or the like.

As used herein, the term "cycloalkoxyl" refers to R-O-, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 ring carbon atoms. Representative examples of cycloalkoxyl comprise but are not limited to cyclopropyloxyl, cyclobutoxyl, or the like.

As used herein, the term "heterocycloalkane ring" refers to fully saturated or partially unsaturated ring (comprising but not limited to such as 3-7 membered monocyclic ring, 7-11 membered bicyclic ring, or 8-16 membered tricyclic ring), at least one heteroatom is present in a ring with at least one carbon atom. When the number of members is limited in front of the heterocycloalkane ring, it refers to the number of ring atoms in the heterocycloalkane ring, for example, 3-16 membered heterocycloalkane ring refers to a heterocycloalkane ring having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkane ring comprise but are not limited to azetidine ring, oxetane ring, tetrahydrofuran ring, piperidine ring, piperazine ring. Polycyclic heterocycloalkane ring comprises spiro, fused and bridged ring, the spiro, fused and bridged heterocycloalkane ring is optionally linked with other rings by single bond, or further linked with other cycloalkane ring and heterocycloalkane ring by any two or more atoms on the ring.

As used herein, the term "aryl" refers to an full carbon monocyclic ring or fused polycyclic ring (i.e., a ring that shares adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl.

As used herein, the term "heteroaryl" refers to aromatic heterocyclic ring group having one to more (preferably 1, 2, 3 or 4) heteroatoms, the heteroaryl can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms of the heteroaryl, for example, 5-12 membered heteroaryl refers to a heteroaryl having 5-12 ring atoms. Representative examples comprise but are not limited to pyrrolyl, pyrazolyl, imidazolyl, thiazolyl, furanyl, pyridyl, pyrimidinyl, etc.

As used herein, the term "aryloxyl" refers to refers to R-O-, wherein R is aryl, the aryl is as defined above. Representative examples of aryloxyl comprise but are not limited to phenoxyl, naphthoxyl, or the like.

As used herein, the term "acyl" refers to 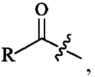 wherein R is alkyl, the alkyl is as defined above. When the number of carbon atoms is limited in front of the acyl (e.g, C2-C6 acyl), it refers to the number of carbon atoms (e.g., 2-6) in the acyl, for example, C2-C6 acyl refers to acyl having 2-6 carbon atoms. For example, the C₂-C₄ acyl refers to C₁-C₃ alkyl -C(O)-, representative examples comprises but are not limited to CH₃C(O)-, C₂H₅C (O)- , or the like.

As used herein, the term "-C(O)-" and 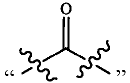 are used interchangeably.

As used herein, the term "-N(R₁₆R₁₇)" and 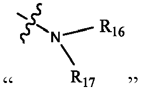 are used interchangeably.

In present invention, it should be understood that all substituents are unsubstituted, unless explicitly described herein as "substituted". The term "substituted" means that one or more hydrogen atoms on the specified group are substituted by specified substituent. Preferably, the "substituted" means that one or more (preferably 1, 2, 3, or 4) hydrogen atoms on the ring or group are each independently substituted by the substituent selected from the group consisting of C1-C6 alkyl, C2-C6 alkenyl, halogen, C3-C6 cycloalkyl, C1-C6 haloalkyl, C1-C6 alkoxyl, C1-C6 haloalkoxyl, C3-C6 cycloalkoxyl, C2-C6 acyl, C6-C12 aryl, 5-12 membered heteroaryl, C6-C12 aryl-O-, 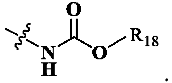 Unless otherwise specified, each substituted group can have a substituent selected from a specified group at any substituted position of the group, the substitution can be the same or different at each substituted position.

In the present invention, the term "prevention" refers to a method of preventing the occurrence of disease and/or its accompanying symptoms, or protecting a subject from getting disease. The term 'prevention' also comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100% inhibition, elimination and reversal. In some embodiments, compared to the level observed in the absence of the compound of present invention, the compound of present invention alleviates, inhibits and/or reverses the tumor and its accompanying symptoms, for example, by at least about 10%, at least about 30%, at least about 50%, at least about 80%, at least about 90%, or 100%.

### Compound

As used herein, the terms "compound of the present invention", "tetracyclic compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof, or a deuterated compound thereof. It should be understood that the term also comprises a mixture of the above components.

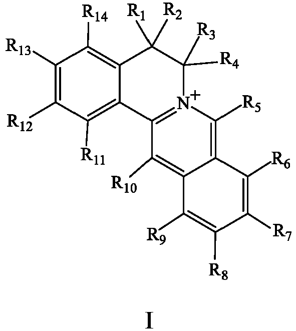

Specifically, the compound of formula I, or an optical isomer thereof, or a racemate thereof, or a solvate thereof, or a pharmaceutically acceptable salt thereof, or a deuterated compound thereof is as described above in the first aspect of the present invention.

Typically, the compound of formula I of the present invention is the compound (comprising salt thereof or free form without salt root) prepared in the Examples of the present invention.

The compound of formula I of the present invention can be prepared using the known organic synthesis method in the art.

The study of the present invention shows that the compound of the present invention has more significant inhibitory effect on tumor with low or no expression of NNMT gene, high expression of DNA methylase, high expression of UHRFl, high methylation level of nucleotide site of NNMT gene, and/or high methylation level of DNA CpG site of NNMT gene. The tumor with low or no expression of NNMT gene, high expression of DNA methylase, high expression of UHRFl, high methylation level of nucleotide site of NNMT gene, and/or high methylation level of DNA CpG site in promoter region of NNMT gene is sensitive to the compound of present invention.

The term "pharmaceutically acceptable salt" refers to a salt formed by the compound of the present invention and an acid or a base, the salt is suitable for use as a drug. Pharmaceutically acceptable salt comprises inorganic salt and organic salt. A preferred type of salt is the salt formed by the compound of the present invention and an acid. Acids suitable for salt formation comprise but are not limited to inorganic acid such as hydrochloric acid, hydrobromic acid, hydrofluoric acid, sulfuric acid, nitric acid, phosphoric acid and the like; organic acid such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid and the like; and acidic amino acid such as aspartic acid and glutamic acid. A preferred type of salt is a metal salt formed by the compound of the present invention and a base. Bases suitable for salt formation comprise but are not limited to inorganic base such as sodium hydroxide, potassium hydroxide, sodium carbonate, sodium bicarbonate, sodium phosphate and the like; and organic base such as ammonia, triethylamine, diethylamine and the like.

The compound of formula I in present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) and 499 bp after the transcription start site (non-bold section) in NNMT gene, the total length of promoter region of NNMT gene is 2500 bp, The nucleotide sequence of the promoter region of NNMT gene is as shown in SEQ ID NO: 1 as follows:

SEQ ID NO: 1:
TATCCAAGAGCTATCAGCACTCCCATGTTTATTGTAGCACTGTTCACAATAGC
CAAGATTTGGAAGTACTCTAAGTGTCCATTAGCAGATGAATGGATAAAGACAATG
TGGTAATACACATAATGGAGTACTATTCAGTCATAAAGAAGAATTAGATCCTGTC
ATTTGCAATAACATGGATGGAACTGGAGGTCATAATGTTGAGTGAAATAAACCAG
GCACAGAAAGACAAACTTTGCATGTTCTCACTTATTTATGGGAGCTAAAAACTAA
AATAACTGAACTCACAGAGATAGAGAGTAGAAGGATGGTTACGAGAGGATGGGA
AGGGTAGCGAGGTGGGTAGGGGGGATGTGGGGATCATTAATGGGTATAAAAAAT
AGTTAGAGGCCAGGCGCAGTGGCTCACGCCTGTAATCCCAGCACTTTGGGAGGC
CGAGGTAGGCGGAACACCTGAGGAGTTCAAGACCAGCCTGGCCAATATGATGAA
ACCCCGTCTCTACTAAAAATACAAAAATTAGCTGGGCGTGATGGTGTGCACCTGT
AGTCCCAGCTGCTTGGGAGGCTGAGGCAGGAGAATCGCTGGAACCCAAGAGGTG
AAGGTTGCAGTGAGCTGAGATCGCGTCACTGCACTCCAGCCTGGGTGACAGAGT
GAGACTCCACATCAAAAAAAAAAAAAAAAAGTTAGAAAGATTGAATAAGACCTAA
TATTTGCTAGCACAACAGGGTGAATATAGTAAAAAATAATTTATTTGTACCTTCA
AAAATAACTAGACAAGTATAATTGGGTTGTTTGTAACACACAAAAAATAAGTACT
TGAAGTGGTGGATACCCCATTTACCCTGATGTGATTATTTTGTATTGCAGGCCTC
TATCAGAATATCTCATGTAACCCATAAATATATACACCTACTCTGTACCCACAAAA
AGTTTTTAAAAAGAAAAATAAATAGCAACCGAAAAAAAAAGAGAGGGAGAAAAG
AAAAAAGAAAAAAAAATCAAGTGCCTGGCTGGGTAGAATAAATTCTAAGGCCAC
AATGTTACTGACCATGGGTTTTTTGGCTCTCAGTGTATAGAAATTGACACAAGGC
CAATAGTCTTCCCAAACATGCTTTACTGGAACTTACGCCCTGGCATAAGGGCCAC
AACAAAAGAGAGAGCGAATTCTCTGGCTTGCTGACTCCTTGGAAAAAACCGGTA
GGGATTTTTTTATTAGGCAAAGCACAGGAATTGACGTCAGAGGCAGGATGTGCT
GCTGGGCAAAGCATACGAGAAGTGGGGTATGCAGGTCAGCATTACTTGGTTGCA
ATGGTTATCTTGAGGAATGGGCCAACTGGTGGTCTGGCCAGTGGCAACAAGGCT
GTAAATCAATTATTCAGCATTCCTTCCCAAGGTGGGACACCCGGCAACATTGTTT
ATCTCCTAAGGCCAGTTCCTGGAATTAAGTGAAAGGATGACTAATGGACATGTTG
TCAGTGAGGTAGTGGTGTGGGTTTTGTGACCAGTGGGAATGCACGAAAGAATGC

TTTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGACA
GAGTCTTGCTCTGTCGCCCAGGCTGGAGTGCAGTGGCGCCATCTTGGCTCACTG
CAAGCTCCGCCCCCCGGGTTCATGCCATTCTCCTGCCTCAGCCTCCCAAGTAGCT
GGGACTGCAGGTGCCTACCACCAAGCCCAGCTAATTTTTTGTATTTTTTTTTTAG
TAGAGACGGGGTTTCACCATGTTAGCCAGGATGGTCTCGATCTCCTGACCTCGTG
ATCTGCCCGCCTTAGCCTCCCAAAGTGCTGGGATTACAGGCATGAGCCTCCGCC
CCCGGCCTTAAATAATTCTTAAAGGAAGTAAAGTTAACTTTGAAAGAACTATCAG
GATTTGGATTGACTGAAAGGAGTGGGGAAGCTTAGGGAGGAGGTGCTTGCCAGAC
ACTGGGTCATGGCAGTGGTCGGTGAAGCTGCAGTTGCCTAGGGCAGGGATGGAGAGA
GAGTCTGGGCATGAGGAGAGGGTCTCGGGATGTTTGGCTGGACTAGATTTTACAGAA
AGCCTTATCCAGGCTTTTAAAATTACTCTTTCCAGACTTCATCTGAGACTCCTTCTTCA
GCCAACATTCCTTAGCCCTGAATACATTTCCTATCCTCATCTTTCCCTTCTTTTTTTTCC
TTTCTTTTACATGTTTAAATTTAAACCATTCTTCGTGACCCCTTTTCTTGGGAGATTCAT
GGCAAGAACGAGAAGAATGATGGTGCTTGTTAGGGGATGTCCTGTCTCTCTGAACTTT
GGGGTCCTATGCATTAAATAATTTTCCTGACGAGCTCAAGTGCTCCCTCTGGTCTACAA
TCCCTGGCGGCTGGCCTTCATCCCTTGGGCAAGCATTGCATACAGCTCATGGCCCTCCC
TCTACCATACCC.

In the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

In the present invention, the nucleotide sites from 840 bp to 469 bp before the transcription start site in NNMT gene is sites 1161-1532 of nucleotide sequence as shown in SEQ ID NO: 1.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Corresponding to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plants and animals. DNA methylation, as a relatively stable modification state, can be passed on to new generations of DNA during DNA replication process under the action of DNA methyltransferase, which is an important epigenetic mechanism.

There are two types of DNA methylation reactions. One type is that the two unmethylated strands in the DNA are methylated, which is called denovo methylation; the other type is that the unmethylated strand of double-stranded DNA with one methylated strand and one unmethylated strand is methylated, which is called maintenance methylation.

Typically, DNA methylation is the methylation of DNA CpG site. The distribution of CpG binucleotide is very uneven in the human genome, while CpG remains or is higher than normal level in some regions of the genome. The CpG site rich region (also known as CpG island) is mainly located in the promoter region and exon regions of the gene, which is a region rich in CpG dinucleotide. About 60% of the promoters of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of the gene. Compared to the regulation of gene expression by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is an ideal biomarker.

### Tumor

The studies of the present invention shows the compound of present invention can be used for preventing and/or treating tumor.

As used herein, the term "tumor" and "cancer" are used interchangeably.

In a preferred embodiment of the present invention, the tumor comprises tumor with low or no expression of NNMT gene. Typically, the tumor with low or no expression of NNMT gene is as described above in the first aspect of the present invention.

In a preferred embodiment of the present invention, the tumor comprises tumor with high expression of DNA methylase. Typically, the tumor with high expression of DNA methylase is as described above in the first aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the first aspect of the present invention.

In a preferred embodiment of the present invention, the tumor comprises tumor with high expression of DNMT3a. Typically, the tumor with high expression of DNMT3a is as described above in the first aspect of the present invention.

In a preferred embodiment of the present invention, the tumor comprises tumor with high expression of DNMT3b. Typically, the tumor with high expression of DNMT3b is as described above in the first aspect of the present invention.

In a preferred embodiment of the present invention, the tumor comprises tumor with high expression of UHRF1(ubiquitin-like with PHD and ring finger domain 1). Typically, the tumor with high expression of UHRF1 is as described above in the first aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the first aspect of the present invention.

In a preferred embodiment of the present invention, the tumor comprises tumor with high methylation level of DNA CpG site of NNMT gene. Typically, the tumor with high methylation level of DNA CpG site of NNMT gene is as described above in the first aspect of the present invention.

Specifically, the tumor of present invention is as described above in the first aspect of the present invention.

In present invention, the type of tumors corresponding to tumor cell lines are shown in Table 2

**Table 2**

| Tumor cell line | The corresponding type of tumor |
|---|---|
| NCI-H82 | Human small cell lung cancer cell |
| G-401 | Human renal carcinoma Wilms cell |
| MDA-MB-453 | Breast cancer cell |
| SW48 | Human colon adenocarcinoma cell |
| GB-1 | Human brain glioblastoma cell |
| CFPAC-1 | Human pancreatic cancer cell |
| SF126 | Human glioblastoma multiforme cell |
| 786-O | Clear cell renal cell adenocarcinoma cell |
| D341 Med | Cerebellar medulloblastoma cell |
| Kelly | Brain neuroblastoma cell |
| NB-1 | Brain neuroblastoma cell |

### Anti-tumor drug

The anti-tumor drug can be the compound of formula I, or an optical isomer thereof, or a racemate thereof, or a solvate thereof, or a pharmaceutically acceptable salt thereof, or a deuterated compound thereof of the present invention.

### Use

A use of the compound of formula I of the present invention for preventing and/or treating tumor is provided.

Specifically, the compound of present invention has remarkable and excellent prevention and treatment effect on tumor with low or no expression of NNMT gene, high expression of DNA methylase, high expression of UHRFl, high methylation level of nucleotide site of NNMT gene, and/or high methylation level of DNA CpG site of NNMT gene. That is, the tumor with low or no expression of NNMT gene, high expression of DNA methylase, high expression of UHRFl, high methylation level of nucleotide site of NNMT gene, and/or high methylation level of DNA CpG site of NNMT gene is sensitive to the compound of the present invention. The expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of the present invention is suitable for use in the precision treatment on tumor, the biomarker can be used to effectively identify patient tumor sensitive to the compound of the present invention, improve treatment effects and avoid administrating the compound of the present invention to patient tumor insensitive to the compound of the present invention, thereby achieving precision treatment on tumor.

The present invention further provides a method for preventing and/or treating tumor, which comprises administering the compound of the present invention to a subject in need.

The compound of present invention has more remarkable and excellent prevention and treatment effect on tumor with low or no expression of NNMT gene, high expression of DNA methylase, high expression of UHRFl, high methylation level of nucleotide site of NNMT gene, and/or high methylation level of DNA CpG site of NNMT gene, therefore, during the prevention and/or treatment of tumor, firstly administering NNMT gene inhibitor, DNA methylase promoter, UHRF1 promoter, methylation promoter of nucleotide site of NNMT gene, and/or methylation promoter of DNA CpG site of NNMT gene to achieve low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of the present invention to prevent and/or treat tumor. Therefore, the present invention develops a compound that can significantly enhance anti-tumor effects when combined with NNMT gene inhibitor, DNA methylase promoter, UHRF1 promoter, methylation promoter of nucleotide site of NNMT gene, and/or methylation promoter of DNA CpG site of NNMT gene. The compound of the present invention can be combined with NNMT gene inhibitor, DNA methylase promoter, UHRF1 promoter, methylation promoter of nucleotide site of NNMT gene, and/or methylation promoter of DNA CpG site of NNMT gene to significantly enhance the treatment effect of the compound on tumor.

In another preferred embodiment, the subject is human and non-human mammals (rodent, rabbit, monkey, livestock, dog, cat, etc.).

In the present invention, there are no special limitations to the method for achieving low or no expression of NNMT gene, high expression of DNA methylase, high expression of UHRFl, high methylation level of nucleotide site of NNMT gene, and/or high methylation level of DNA CpG site of NNMT gene in the tumor. For example, the low or no expression of NNMT gene, high expression of DNA methylase, high expression of UHRFl, high methylation level of nucleotide site of NNMT gene, and/or high methylation level of DNA CpG site of NNMT gene in the tumor can be specifically achieved using methods such as gene insertion, gene knockout, or gene silencing (e.g, shRNA transfection), etc.

### Marker

The present invention provides a marker for determining whether the compound of present invention is suitable for use in the prevention and/or treatment of patient tumor, the marker comprises the expression level of NNMT gene, the expression level of DNA methylase, the expression level of UHRF1, the methylation level of nucleotide site of NNMT gene, and/or the methylation level of DNA CpG site of NNMT gene.

In a preferred embodiment, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether the compound of present invention is suitable for use in the prevention and/or treatment of patient tumor, the method comprises as follows:
the compound of present invention is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene; and/or
the compound of present invention is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

Specifically, the tumor with low or no expression of NNMT gene, high expression of DNA methylase (e.g, NNMT1), high expression of UHRFl, high methylation level of nucleotide site of NNMT gene, and/or high methylation level of DNA CpG site of NNMT gene is as described above in the first aspect of the present invention.

In a preferred embodiment, the tumor with high expression of NNMT gene, low expression of DNA methylase (e.g, NNMT1), low expression of UHRFl, low methylation level of nucleotide site of NNMT gene, and/or low methylation level of DNA CpG site of NNMT gene is as described above in the second aspect of the present invention.

The present invention further provides a use of the marker (or the expression level of marker) or its detection reagent in the preparation of reagent kit for determining whether the compound of the present invention is suitable for use in the prevention and/or treatment of patient tumor.

### Composition or preparation, active ingredient combination, medical kit and administration method

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

Typically, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 10 µg/kg.bw to 1 mg/kg.bw. Of course, the specific dose should also take into account the route of administration, the patient's health and other factors, which are within the skill range of skilled doctors.

### The main advantages of the present invention comprise:

1. The present invention has unexpectedly developed a compound that can have excellent precision treatment effect on tumor with low or no expression of NNMT gene, high expression of DNA methylase, high expression of UHRFl, high methylation level of nucleotide site of NNMT gene, and/or high methylation level of DNA CpG site of NNMT gene. The tumor with low or no expression of NNMT gene, high expression of DNA methylase, high expression of UHRFl, high methylation level of nucleotide site of NNMT gene, and/or high methylation level of DNA CpG site of NNMT gene is highly sensitive to the compound of present invention, ie, the compound of present invention has more remarkable and excellent treatment effect on tumor with low or no expression of NNMT gene, high expression of DNA methylase, high expression of UHRFl, high methylation level of nucleotide site of NNMT gene, and/or high methylation level of DNA CpG site of NNMT gene. Therefore, the compound of present invention can realize precise treatment on tumor with low or no expression of NNMT gene, high expression of DNA methylase, high expression of UHRFl, high methylation level of nucleotide site of NNMT gene, and/or high methylation level of DNA CpG site of NNMT gene, thus improving the treatment effect of the compound of present invention on tumor and avoiding administrating the compound of present invention to patient tumor insensitive to such anti-tumor drug. Therefore, the precision treatment of the compound of the present invention on tumor with low or no expression of NNMT gene, high expression of DNA methylase, high expression of UHRFl, high methylation level of nucleotide site of NNMT gene, and/or high methylation level of DNA CpG site of NNMT gene has advantages such a more excellent prevention and treatment effect on tumors, low drug dose, and minimal side effects, which improve the precision prevention and treatment effect on tumor, reduce the side effects and improve patient compliance.
2. The compound of present invention has excellent druggability.

The present invention will be further illustrated below with reference to the specific examples. It should be understood that these examples are only to illustrate the invention but are not to limit the scope of the invention. The experimental methods with no specific conditions described in the following examples are generally performed under the conventional conditions, or according to the manufacturer's instructions. Unless indicated otherwise, parts and percentage are calculated by weight.

### Example

DNMT3a refers to DNA methyltransferase 3a, NCBI entrez gene: 1788; Uniprotkb/Swiss-port: Q9Y6K1.

DNMT3b refers to DNA methyltransferase 3b, NCBI entrez gene: 1789; Uniprotkb/Swiss-port: Q9UBC3.

DNMT1 refers to DNA methyltransferase 1, NCBI entrez gene: 1786; Uniprotkb/Swiss-port: P26358.

UHRF1 refers to ubiquitin-like with PHD and ring finger domain 1, NCBI entrez gene: 29128; Uniprotkb/Swis s-port: Q96T8 8.

NNMT refers to Nicotinamide N-Methyltransferase.

The nucleotide sequence of the promoter region of NNMT gene was as shown in SEQ ID NO: 1.

The nucleotide sites from 1050 bp before the transcription start site to 499 bp after the transcription start site in NNMT gene was sites 951-2500 of nucleotide sequence as shown in SEQ ID NO: 1.

The nucleotide sites from 1050 bp to 193 bp before the transcription start site in NNMT gene was sites 951-1808 of nucleotide sequence as shown in SEQ ID NO: 1.

The nucleotide sites from 840 bp to 469 bp before the transcription start site in NNMT gene was sites 1161-1532 of nucleotide sequence as shown in SEQ ID NO: 1.

### Example 1 Synthesis of compound AB31706

The structure of compound AB31706 was as follows:

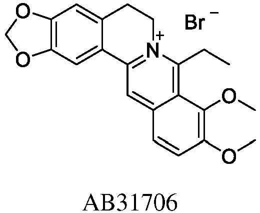

### Step 1):

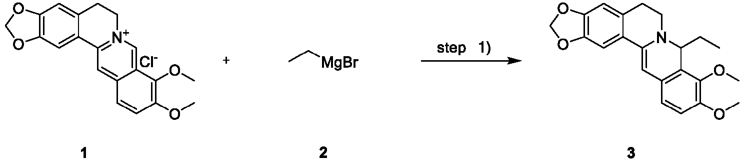

Compound 1 (337 mg, 1 mmol) was dissolved in tetrahydrofuran (20 mL), and compound 2 (2 mL, 3 mmol/L) was added, the mixture was stirred at room temperature for overnight, new spot was detected on a plate. The mixture was quenched with water and then filtered with diatomite, the filtrate was concentrated under reduced pressure to remove organic solvent, the remaining aqueous phase was filtered to afford solid compound 3 (190 mg, yield: 36.55%).

### Step 2):

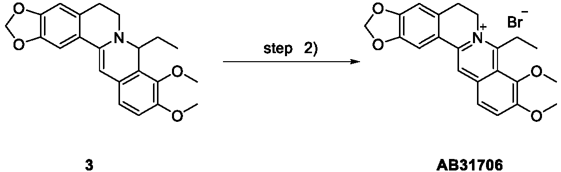

Compound 3 (190 mg, 0.52 mmol) was dissolved in acetic acid (510 mL), and heated to 120 °C, a mixture solution of liquid bromine (83.2 mg, 0.52 mmol) in acetic acid (1 mL) was then added. The mixture was stirred for 1 h, new spot was detected on a plate. The mixture was quenched with water and then filtered with diatomite, the filtrate was concentrated under reduced pressure to remove organic solvent, the remaining aqueous phase was then filtered to afford solid compound AB31706 (68.2 mg).

MS-ESI: Calculated: [M-Br⁻]⁺ : 364.42, Found: 364.10.

¹H NMR (400 MHz, DMSO-d6) : δ 8.78 (s, 1H), 8.16 (d, J = 9.1 Hz, 1H), 7.99 (d, J = 9.1 Hz, 1H), 7.71 (s, 1H), 7.08 (s, 1H), 6.14 (s, 2H), 4.84 - 4.76 (m, 2H), 4.03 (d, J = 3.9 Hz, 6H), 3.78 (s, 2H), 3.14 (d, J = 5.3 Hz, 2H), 1.43 (s, 3H).

### Example 2 Synthesis of compound AB31707

The structure of compound AB31707 was as follows:

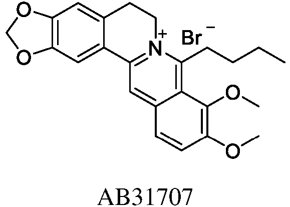

### Step 1):

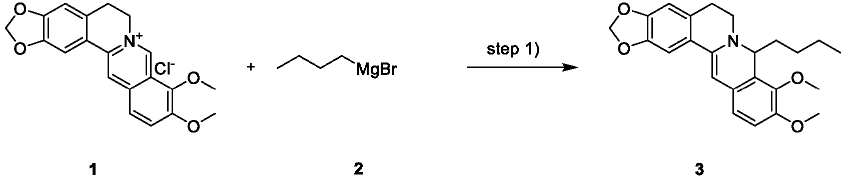

Compound 1 (337 mg, 1 mmol) was dissolved in tetrahydrofuran (20 mL), and compound 2 (2 mL, 3 mmol/L) was added, the mixture was stirred at room temperature for overnight, new spot was detected on a plate. The mixture was quenched with water and then filtered with diatomite, the filtrate was concentrated under reduced pressure to remove organic solvent, the remaining aqueous phase was filtered to afford solid compound 3 (230 mg).

### Step 2):

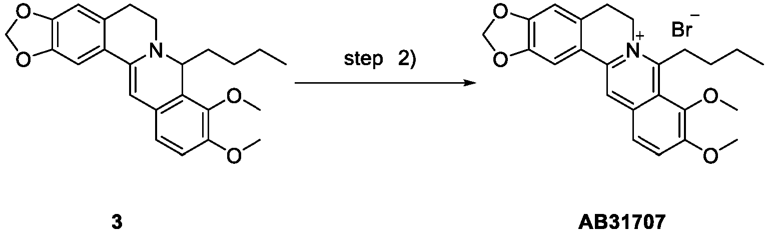

Compound 3 (50 mg, 0.12 mmol) was dissolved in acetic acid (5 mL), and heated to 120 °C, a mixture solution of liquid bromine (20 mg, 0.12 mmol) in acetic acid (1 mL) was then added. The mixture was stirred for 1 h, new spot was detected on a plate. The mixture was quenched with water and then filtered with diatomite, the filtrate was concentrated under reduced pressure to remove organic solvent, the remaining aqueous phase was filtered to afford solid compound AB31707 (68.2 mg).

MS-ESI: Calculated: [M-Br⁻]⁺ : 392.47, Found: 392.05.

¹H NMR (400 MHz, DMSO-d6) : δ 8.77 (s, 1H), 8.16 (d, J = 9.1 Hz, 1H), 7.98 (d, J = 9.1 Hz, 1H), 7.70 (s, 1H), 7.08 (s, 1H), 6.13 (s, 2H), 4.78 (s, 1H), 4.02 (d, J = 11.2 Hz, 6H), 3.73 (d, J = 9.9 Hz, 2H), 3.13 (s, 3H), 1.75 (s, 2H), 1.57 (d, J = 7.3 Hz, 2H), 1.20 (s, 2H), 0.98 (t, J = 7.3 Hz, 3H).

### Example 3 Synthesis of compound AB31711

The structure of compound AB31711 was as follows:

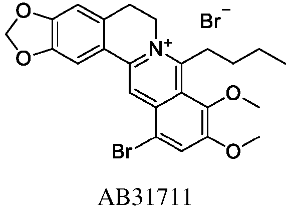

### Step 1):

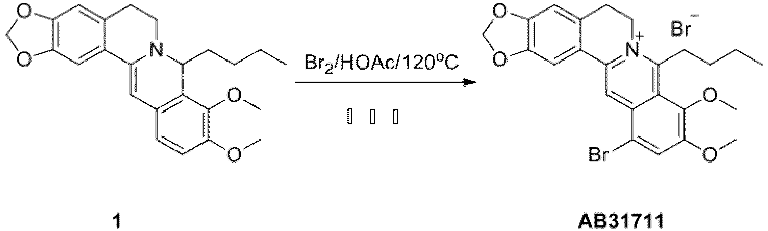

Compound 1 (200 mg, 0.508 mmol) was dissolved in acetic acid (10 mL), and heated to 120 °C, a mixture solution of liquid bromine (488 mg, 3.05 mmol) in acetic acid (1 mL) was then added. The mixture was stirred for 1 h, new spot was detected on a plate. The mixture was quenched with water and then filtered with diatomite, the filtrate was concentrated under reduced pressure to remove organic solvent, the remaining aqueous phase was then filtered to afford solid compound AB31711.

MS-ESI: Calculated: [M-Br-]+ : 471.37, Found: 472.00.

1H NMR (400 MHz, DMSO-d6) δ 8.52 (s, 1H), 8.41 (s, 1H), 7.74 (s, 1H), 7.11 (s, 1H), 6.15 (s, 2H), 4.81 (t, J = 7.4 Hz, 2H), 4.07 (s, 3H), 4.02 (s, 3H), 3.71 (s, 2H), 3.12 (s, 2H), 1.75 (s, 2H), 1.57 (d, J = 7.1 Hz, 2H), 0.98 (t, J = 7.3 Hz, 3H).

### Example 4 Synthesis of compound AB31716

The structure of compound AB31716 was as follows:

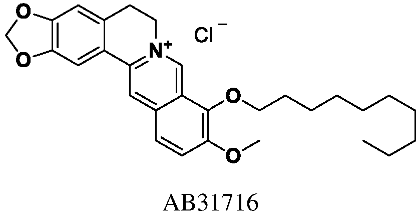

### Step 1):

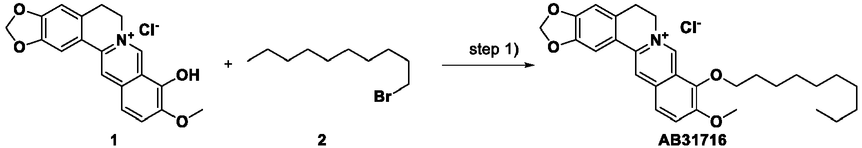

Compound 1 (322 mg, 1 mmol) was dissolved in N, N-dimethylacetamide (8 mL), and compound 2 (884.4 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20: 1) to afford solid compound AB31716 (122.9 mg).
MS-ESI: Calculated: [M-Cl⁻]⁺ : 462.60, Found: 462.25
1H NMR (400 MHz, DMSO-d6) : δ 9.72 (s, 1H), 8.90 (s, 1H), 8.17 (d, J = 9.1 Hz, 1H), 7.97 (s, 1H), 7.77 (s, 1H), 7.07 (s, 1H), 6.15 (s, 2H), 4.91 (s, 2H), 4.25 (t, J = 6.7 Hz, 2H), 4.02 (s, 3H), 3.18 (s, 2H), 1.89 - 1.79 (m, 2H), 1.44 (s, 2H), 1.23 (s, 12H), 0.82 (d, J = 7.0 Hz, 3H).

### Example 5 Synthesis of compound AB31717

The structure of compound AB31717 was as follows:

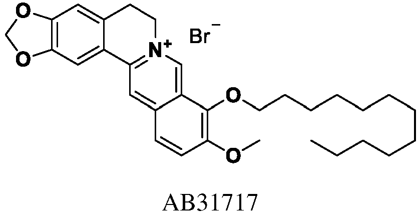

### Step 1):

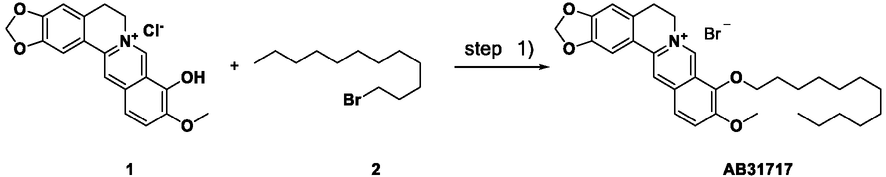

Compound 1 (322 mg, 1 mmol) was dissolved in N, N-dimethylacetamide (8 mL), and compound 2 (996.92 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31717 (144.5 mg).
MS-ESI: Calculated: [M-Br-]+ : 490.65, Found: 490.25
1H NMR (400 MHz, DMSO-d6) : δ 9.71 (s, 1H), 8.90 (s, 1H), 8.16 (d, J = 8.9 Hz, 1H), 7.95 (d, J = 9.3 Hz, 1H), 7.76 (s, 1H), 7.06 (s, 1H), 6.14 (s, 2H), 4.91 (s, 2H), 4.25 (s, 2H), 4.02 (s, 3H), 3.17 (s, 2H), 1.84 (s, 2H), 1.44 (s, 2H), 1.21 (s, 16H), 0.81 (d, J = 6.8 Hz, 3H).

### Example 6 Synthesis of compound AB31718

The structure of compound AB31718 was as follows:

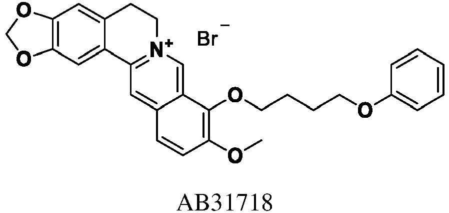

### Step 1):

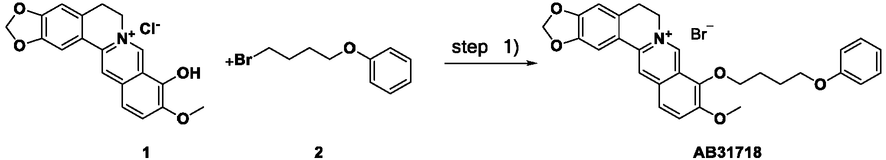

Compound 1 (322 mg, 1 mmol) was dissolved in N, N-dimethylacetamide (8 mL), and compound 2 (916.44 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31718 (83.9 mg).
MS-ESI: Calculated: [M-Br⁻]⁺ : 470.54, Found: 470.10
1H NMR (400 MHz, DMSO-d6) δ 9.74 (s, 1H), 8.90 (s, 1H), 8.16 (s, 1H), 7.96 (d, J = 9.0 Hz, 1H), 7.77 (s, 1H), 7.25 (s, 2H), 7.06 (s, 1H), 6.90 (d, J = 8.2 Hz, 3H), 6.14 (s, 2H), 4.90 (s, 2H), 4.33 (s, 2H), 4.05 (s, 2H), 4.01 (s, 3H), 3.17 (s, 2H), 1.97 (d, J = 22.8 Hz, 4H).

### Example 7 Synthesis of compound AB31721

The structure of compound AB31721 was as follows:

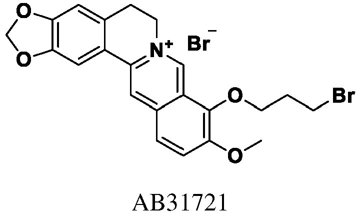

### Step 1):

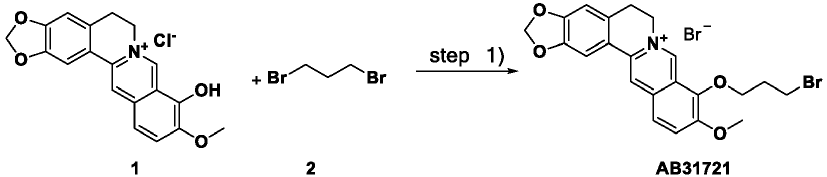

Compound 1 (161 mg, 0.5 mmol) was dissolved in N, N-dimethylacetamide (8 mL), and compound 2 (400 mg, 2 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20: 1) to afford solid compound AB31721 (122.9 mg).
MS-ESI: Calculated: [M-Br⁻]⁺ : 443.31, Found: 444.10
1H NMR (400 MHz, DMSO-d6): δ 9.76 (s, 1H), 8.90 (s, 1H), 8.17 (d, J = 9.2 Hz, 1H), 7.98 (d, J = 9.2 Hz, 1H), 7.76 (s, 1H), 7.06 (s, 1H), 6.14 (s, 2H), 4.91 (s, 2H), 4.38 (t, J = 6.1 Hz, 2H), 4.03 (s, 4H), 3.79 (t, J = 6.6 Hz, 3H), 3.19 (d, J = 5.5 Hz, 3H), 2.42 - 2.35 (m, 2H).

### Example 8 Synthesis of compound AB31722

The structure of compound AB31722 was as follows:

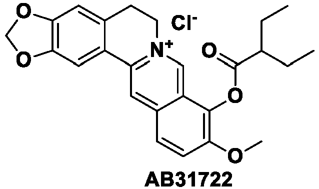

### Step 1):

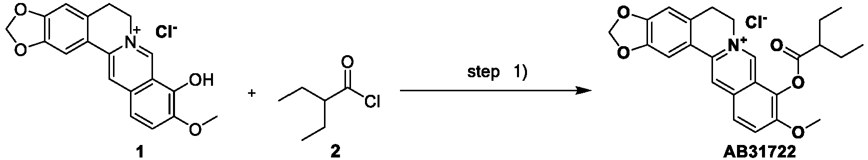

Compound 1 (100 mg, 0.3 mmol) was dissolved in acetonitrile (10 mL), and compound 2 (100 mL, 3 mmol/L) was added at room temperature, the mixture was stirred at room temperature for 2 h, new spot was detected on a plate. The mixture was quenched with methanol (2 ml) and then concentrated, the concentrate was triturated with dichloromethane to afford solid compound AB31722 (70 mg).
MS-ESI: Calculated: [M-Cl⁻]⁺: 420.48, Found: 420.10..
1H NMR (400 MHz,DMSO-d6) δ 9.60 (s, 1H), 9.01 (s, 1H), 8.27 (d, J = 9.4 Hz, 1H), 8.18 (d, J = 9.1 Hz, 1H), 7.79 (s, 1H), 7.07 (s, 1H), 6.15 (s, 2H), 4.90 (s, 2H), 3.99 (s, 3H), 3.19 (s, 2H), 2.78 (s, 1H), 1.88 - 1.79 (m, 2H), 1.75 (d, J = 6.8 Hz, 2H), 1.03 (t, J = 7.4 Hz, 6H).

### Example 9 Synthesis of compound AB31723

The structure of compound AB31723 was as follows:

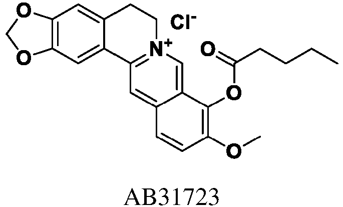

### Step 1):

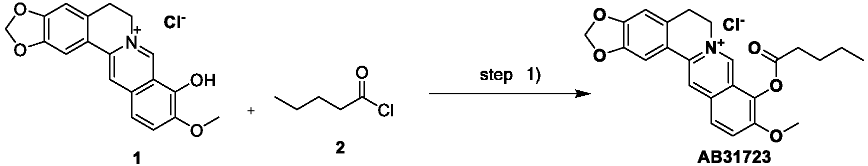

Compound 1 (322 mg, 1 mmol) was dissolved in acetonitrile (10 mL), and compound 2 (360 mL, 3 mmol/L) was added at room temperature, the mixture was stirred at room temperature for 2 h, new spot was detected on a plate. The mixture was quenched with methanol (2 ml) and then concentrated, the concentrate was triturated with dichloromethane to afford solid compound AB31723 (204.1 mg).

MS-ESI: Calculated: [M-Cl⁻]⁺ : 406.45, Found: 406.10.

1H NMR (400 MHz, DMSO-d6) δ 9.80 (s, 1H), 8.97 (s, 1H), 8.23 (s, 1H), 8.19 (s, 1H), 7.77 (s, 1H), 7.06 (s, 1H), 6.14 (s, 2H), 4.88 (s, 2H), 3.99 (s, 3H), 3.19 (s, 2H), 2.82 (t, J = 7.4 Hz, 2H), 1.70 (s, 2H), 1.44 (d, J = 7.5 Hz, 2H), 0.94 (t, J = 7.4 Hz, 3H).

### Example 10 Synthesis of compound AB31731

The structure of compound AB31731 was as follows:

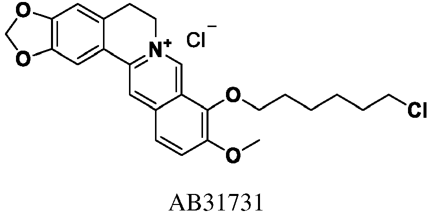

### Step 1):

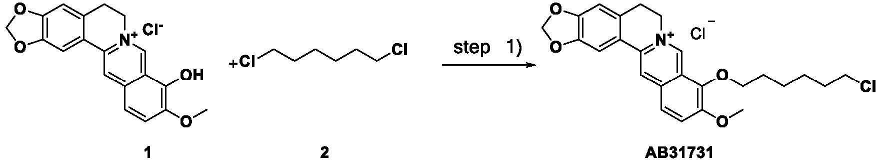

Compound 1 (322 mg, 1 mmol) was dissolved in N, N-dimethylacetamide (8 mL), and compound 2 (620.28 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31731 (35.4 mg).
MS-ESI: Calculated: [M-Cl⁻]⁺ : 440.94, Found: 440.15
1H NMR (400 MHz, DMSO-d6) : δ 9.71 (s, 1H), 8.90 (s, 1H), 8.16 (d, J = 9.1 Hz, 1H), 7.95 (d, J = 9.4 Hz, 1H), 7.76 (s, 1H), 7.06 (s, 1H), 6.14 (s, 2H), 4.91 (s, 2H), 4.26 (t, J = 6.8 Hz, 2H), 4.02 (s, 3H), 3.63 (t, J = 6.6 Hz, 2H), 3.17 (s, 2H), 1.85 (s, 2H), 1.74 (s, 2H), 1.48 (s, 4H).

### Example 11 Synthesis of compound AB31737

The structure of compound AB31737 was as follows:

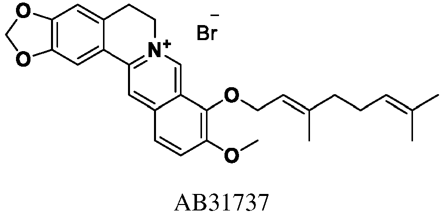

### Step 1):

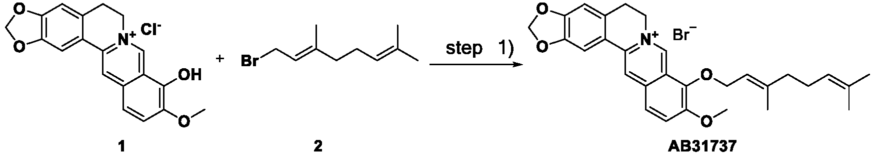

Compound 1 (322 mg, 1 mmol) was dissolved in N, N-dimethylacetamide (8 mL), and compound 2 (868.6 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31737 (31.1 mg).
MS-ESI: Calculated: [M-Br-]⁺: 458.58, Found: 458.20
1H NMR (400 MHz, DMSO-d6) : δ 9.78 (s, 1H), 8.91 (s, 1H), 8.16 (d, J = 9.1 Hz, 1H), 7.96 (d, J = 8.8 Hz, 1H), 7.77 (s, 1H), 7.06 (s, 1H), 6.15 (s, 2H), 4.92 (s, 3H), 4.85 (d, J = 7.2 Hz, 2H), 4.04 (s, 3H), 3.17 (s, 2H), 1.94 (s, 4H), 1.61 (s, 3H), 1.54 (s, 3H), 1.46 (s, 3H), 1.20 (s, 2H).

### Example 12 Synthesis of compound AB31739

The structure of compound AB31739 was as follows:

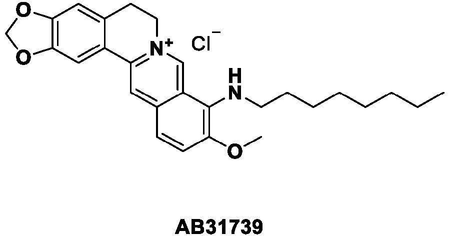

### Step 1):

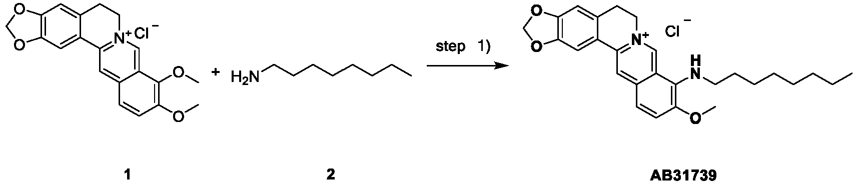

Compound 1 (300 mg, 0.89 mmol) was dissolved in ethanol (20 mL), and compound 2 (4.17 g, 32.27 mmol) was added. The mixture was stirred at 78 °C for overnight, and the reaction was detected to be performed completely using LCMS. The reaction mixture was diluted with water and then extracted with ethyl acetate, the combined organic phase was filtered and concentrated under reduced pressure to remove organic solvent, the residue was purified by preparative column to afford solid compound AB31739 (31.8 mg).
MS-ESI: Calculated: [M-Br-]⁺: 433.25, Found: 433.40
1H NMR (399 MHz, cd3od) δ 9.60 (s, 1H), 8.52 (s, 1H), 8.47 (s, 1H), 7.84 (d, J = 8.8 Hz, 1H), 7.58 (s, 2H), 6.91 (s, 1H), 6.07 (s, 2H), 4.03 (s, 4H), 3.60 (t, J = 7.3 Hz, 3H), 3.21 (s, 3H), 1.68 (s, 3H), 1.41 (s, 3H), 1.28 (d, J = 11.4 Hz, 10H), 0.87 (d, J = 4.4 Hz, 4H).

### Example 13 Synthesis of compound AB31740

The structure of compound AB31740 was as follows:

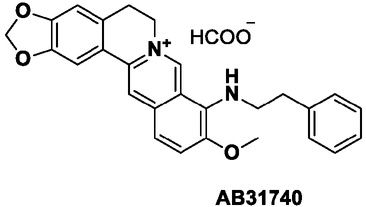

### Step 1):

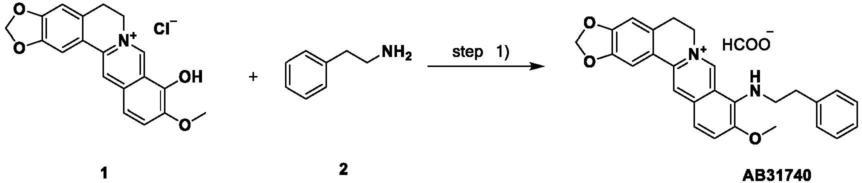

Compound 1 (200 mg, 0.6 mmol) was dissolved in ethanol (20 mL), and compound 2 (726 mg, 6 mmol) was added. The mixture was stirred at 78 °C for overnight, new spot was detected on a plate. The reaction mixture was diluted with water and then extracted with ethyl acetate, the combined organic phase was filtered and concentrated under reduced pressure to remove organic solvent, the residue was purified by preparative column to afford solid compound AB31740 (7.3 mg).

MS-ESI: Calculated: [M-Br-]+ : 425.19, Found: 425.15.

1H NMR (400 MHz, cd3od) δ 9.33 (s, 1H), 8.48 (s, 1H), 7.84 (d, J = 8.7 Hz, 1H), 7.61 (d, J = 10.2 Hz, 2H), 7.26 -7.16 (m, 5H), 6.92 (s, 1H), 6.08 (s, 2H), 4.71- 4.67 (m, 2H), 4.58 (s, 1H), 4.00 (s, 3H), 3.86 (t, J = 6.8 Hz, 2H), 3.22 - 3.18 (m, 2H), 2.95 (t, J = 6.8 Hz, 2H). 8

### Example 14 Synthesis of compound AB31748

The structure of compound AB31748 was as follows:

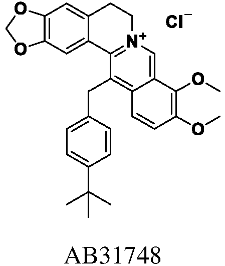

### Step 1):

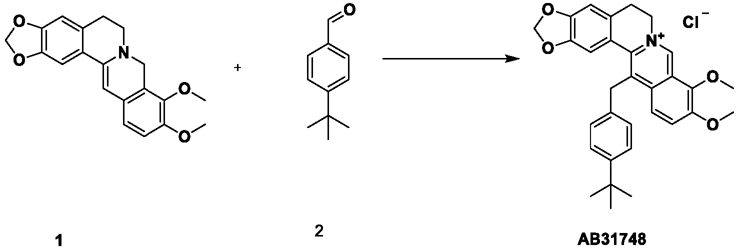

Compound 1 (337 mg, 1 mmol) was dissolved in 80% ethanol (5 mL), and acetic acid (4 mL) and compound 2 (1.20 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography to afford solid compound AB31748.

MS-ESI: Calculated: [M-Cl⁻]⁺ 482.59, Found: 482.30.

1H NMR (400 MHz, DMSO-6) : δ 9.96 (s, 1H), 8.06 (d, J = 9.6 Hz, 1H), 7.75 (d, J = 9.4 Hz, 1H), 7.34 (d, J = 8.4 Hz, 2H), 7.13 (s, 1H), 7.05 (d, J = 7.7 Hz, 2H), 6.96 (s, 1H), 6.04 (s, 2H), 4.83 (s, 2H), 4.66 (s, 2H), 4.08 (s, 3H), 3.98 (s, 3H), 3.12 (s, 2H), 1.23 (s, 9H).

### Example 15 Synthesis of compound AB31757

The structure of compound AB31757 was as follows:

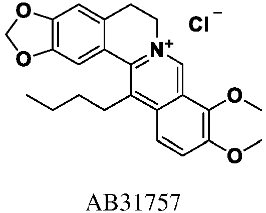

### Step 1):

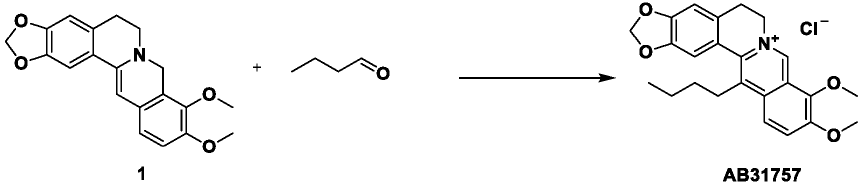

Compound 1 (200 mg, 0.59 mmol) was dissolved in ethanol (5 mL), and acetic acid (4 mL) and butyraldehyde (424.8 mg, 5.9 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography to afford solid compound AB31757 (23.8 mg).
MS-ESI: Calculated: [M-Cl-]+ : 392.47, Found: 392.65
1H NMR (400 MHz, DMSO-6) : δ 9.86 (s, 1H), 8.17 (s, 2H), 7.26 (s, 1H), 7.13 (s, 1H), 6.15 (s, 2H), 4.76 (s, 2H), 4.06 (d, J = 2.6 Hz, 6H), 3.05 (s, 2H), 1.72 (s, 2H), 1.38 (d, J = 6.7 Hz, 2H), 1.13 (s, 2H), 0.88 (t, J = 7.1 Hz, 3H).

### Example 16 Synthesis of compound AB31758

The structure of compound AB31758 was as follows:

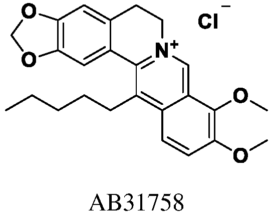

### Step 1):

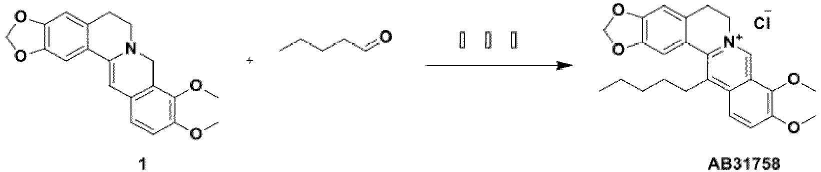

Compound 1 (200 mg, 0.59 mmol) was dissolved in 80% ethanol (5 mL), and pentaaldehyde (507.4 mg, 5.9 mmol) and ice acetic acid (4mL) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by preparative column (parameters: device: Shimadzu PREP-HPLC system; column: Sharpsil-U C18 (250 mm x 30 x 10 um, 100 Å); mobile phase: 0.1% hydrochloric acid aqueous solution/acetonitrile; 1-20 min, 5-25% acetonitrile, 20-50 min, 25-35% acetonitrile; column temperature: 25 °C; detection wavelength: 224/254 nm; flow rate: 30 mL/min; injection volume: 2.0 mL; sample concentration: 25 mg/mL) to afford solid compound AB31758.

MS-ESI: Calculated: [M-Cl⁻]⁺: 406.49, Found: 406.10.

1H NMR (400 MHz, DMSO-6) δ 9.85 (s, 1H), 8.17 (s, 2H), 7.24 (s, 1H), 7.13 (s, 1H), 6.15 (s, 2H), 4.76 (s, 2H), 4.06 (d, J = 3.1 Hz, 6H), 3.31 - 3.28 (m, 2H), 3.05 (s, 2H), 1.80 - 1.64 (m, 2H), 1.38 - 1.24 (m, 4H), 0.83 (t, J = 7.0 Hz, 3H).

### Example 17 Synthesis of compound AB31759

The structure of compound AB31759 was as follows:

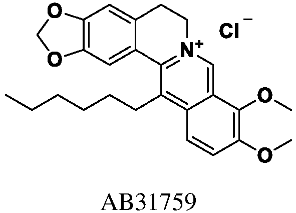

### Step 1):

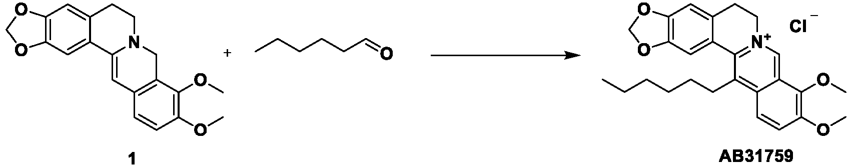

Compound 1 (200 mg, 0.59 mmol) was dissolved in ethanol (5 mL), and acetic acid (4 mL) and hexanal (590 mg, 5.9 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography to afford solid compound AB31759 (56.9 mg).

MS-ESI: Calculated: [M-Cl⁻]⁺ : 420.52, Found: 420.20.

1H NMR (400 MHz, DMSO-6) δ 9.85 (s, 1H), 8.17 (s, 2H), 7.25 (s, 1H), 7.13 (s, 1H), 6.15 (s, 2H), 4.75 (s, 2H), 4.06 (d, J = 2.4 Hz, 6H), 3.28 - 3.26 (m, 2H), 3.04 (s, 2H), 1.72 (s, 2H), 1.35 (s, 2H), 1.22 (s, 4H), 0.83 (d, J = 6.8 Hz, 3H).

### Example 18 Synthesis of compound AB31760

The structure of compound AB31760 was as follows:

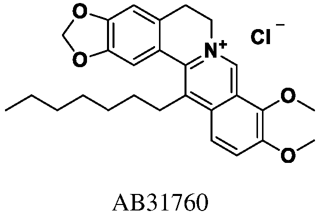

### Step 1):

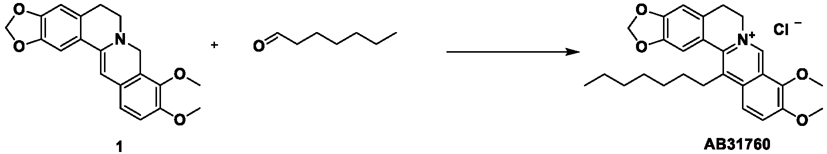

Compound 1 (200 mg, 0.59 mmol) was dissolved in ethanol (5 mL), and acetic acid (4 mL) and heptanal (590 mg, 5.9 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography to afford solid compound AB31760 (15.6 mg).

MS-ESI: Calculated: [M-Cl⁻]⁺: 434.55, Found: 434.30.

1H NMR (400 MHz, DMSO-6) δ 9.86 (s, 1H), 8.17 (s, 2H), 7.26 (s, 1H), 7.13 (s, 1H), 6.15 (s, 2H), 4.76 (s, 2H), 4.06 (d, J = 2.0 Hz, 6H), 3.05 (s, 2H), 1.72 (s, 2H), 1.34 (s, 2H), 1.20 (s, 8H), 0.82 (t, J = 6.7 Hz, 3H).

### Example 19 Synthesis of compound AB31761

The structure of compound AB31761 was as follows:

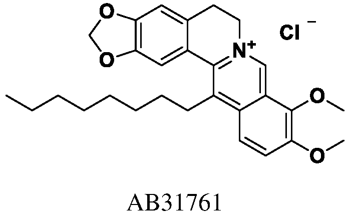

### Step 1):

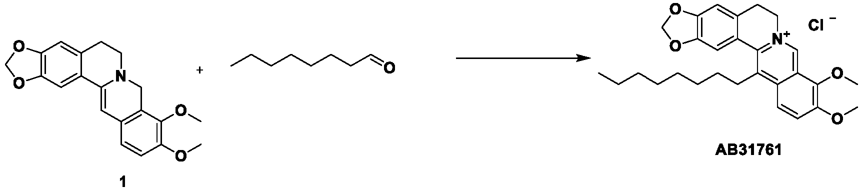

Compound 1 (337 mg, 1 mmol) was dissolved in 80% ethanol (5 mL), and acetic acid (4 mL) and octanal (1.56 mg,10 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31761 (30.3 mg).

MS-ESI: Calculated: [M-Cl⁻]⁺ : 448.57, Found: 448.45.

1H NMR (400 MHz, DMSO-6) δ 9.86 (s, 1H), 8.17 (s, 2H), 7.26 (s, 1H), 7.13 (s, 1H), 6.16 (s, 2H), 4.76 (s, 2H), 4.06 (d, J = 2.1 Hz, 6H), 3.05 (s, 2H), 1.71 (s, 2H), 1.34 (s, 2H), 1.20 (s, 10H), 0.82 (t, J = 6.7 Hz, 3H).

### Example 20 Synthesis of compound AB31762

The structure of compound AB31762 was as follows:

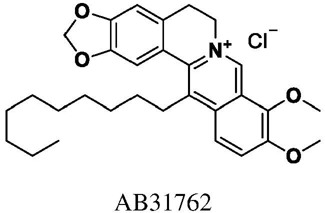

### Step 1):

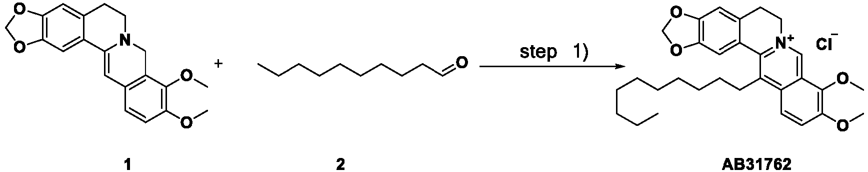

Compound 1 (338 mg, 1 mmol) was dissolved in 80% ethanol (5 mL), and acetic acid (4 mL) and compound 2 (1.56 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31762 (110.9 mg).

MS-ESI: Calculated: [M-Cl⁻]⁺ : 476.63, Found: 476.20.

1H NMR (400 MHz, DMSO-d6) δ 9.86 (s, 1H), 8.16 (s, 2H), 7.26 (s, 1H), 7.13 (s, 1H), 6.15 (s, 2H), 4.76 (s, 2H), 4.06 (d, J = 2.3 Hz, 6H), 3.04 (s, 2H), 1.19 (s, 18H), 0.81 (d, J = 6.9 Hz, 3H)

### Example 21 Synthesis of compound AB31763

The structure of compound AB31763 was as follows:

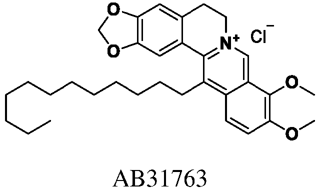

### Step 1):

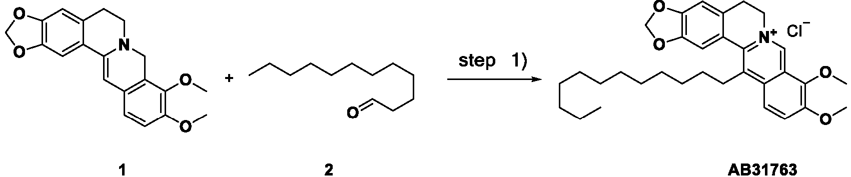

Compound 1 (337 mg, 1 mmol) was dissolved in 80% ethanol (5 mL), and acetic acid (4 mL) and compound 2 (1.86 g, 10 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31763 (36.7 mg).

MS-ESI: Calculated: [M-Cl⁻]⁺ : 504.68, Found: 504.40.

1H NMR (400 MHz, DMSO-d6) δ 9.85 (s, 1H), 8.17 (s, 2H), 7.26 (s, 1H), 7.13 (s, 1H), 6.15 (s, 2H), 4.75 (s, 2H), 4.06 (d, J = 2.0 Hz, 6H), 3.05 (s, 2H), 1.20 (s, 22H), 0.82 (s, 3H).

### Example 22 Synthesis of compound AB31765

The structure of compound AB31765 was as follows:

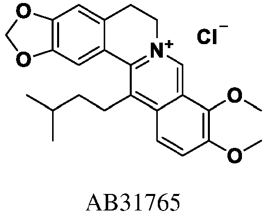

### Step 1):

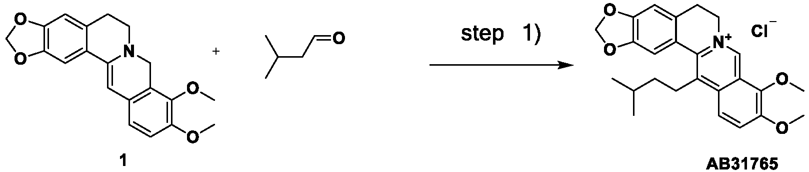

Compound 1 (200 mg, 0.59 mmol) was dissolved in 80% ethanol (5 mL), and isovaleraldehyde (507.4 mg, 5.9 mmol) and ice acetic acid (4 mL) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by preparative column to afford solid compound AB31765 (23.2 mg).

MS-ESI: Calculated: [M-Cl⁻]⁺ : 406.49, Found: 406.15.

1H NMR (399 MHz, DMSO-6) δ 9.87 (s, 1H), 8.16 (q, J = 9.6 Hz, 2H), 7.30 (s, 1H), 7.13 (s, 1H), 6.15 (s, 2H), 4.76 (s, 2H), 4.06 (d, J = 3.5 Hz, 6H), 3.29 (s, 2H), 3.05 (s, 2H), 1.67 (s, 2H), 1.07 (d, J = 6.8 Hz, 1H), 0.91 (d, J = 5.9 Hz, 6H).

### Example 23 Synthesis of compound AB31766

The structure of compound AB31766 was as follows:

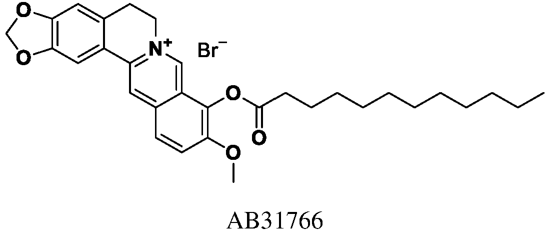

### Step 1):

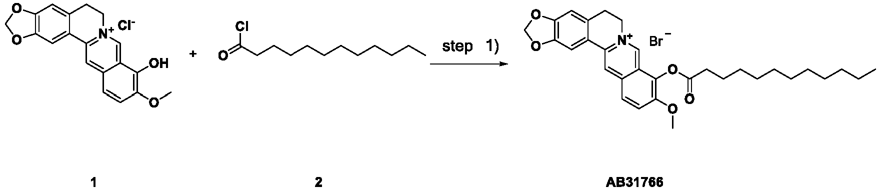

Compound 1 (322 mg, 1 mmol) was dissolved in N, N-dimethylacetamide (8 mL), and compound 2 (875.04 mg, 4 mmol) was added. The mixture was stirred at 80 °C for 16 h, and the reaction was detected to be performed completely using LCMS. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31766 (100 mg).

MS-ESI: Calculated: [M-Br-]+ : 504.64, Found: 504.30.

1H NMR (400 MHz,DMSO-d6) δ 9.82 (s, 1H), 8.99 (s, 1H), 8.26 (d, J = 9.2 Hz, 1H), 8.17 (d, J = 9.0 Hz, 1H), 7.78 (s, 1H), 7.08 (s, 1H), 6.15 (s, 2H), 4.89 (t, J = 6.6 Hz, 2H), 4.00 (s, 3H), 3.18 (d, J = 7.1 Hz, 2H), 2.82 (t, J = 7.4 Hz, 2H), 1.71 (t, J = 8.5 Hz, 2H), 1.47 - 1.36 (m, 2H), 1.25 (dd, J = 24.0, 12.7 Hz, 14H), 0.83 (t, J = 6.7 Hz, 3H).

### Example 24 Synthesis of compound AB31767

The structure of compound AB31767 was as follows:

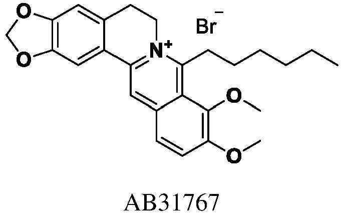

### Step 1):

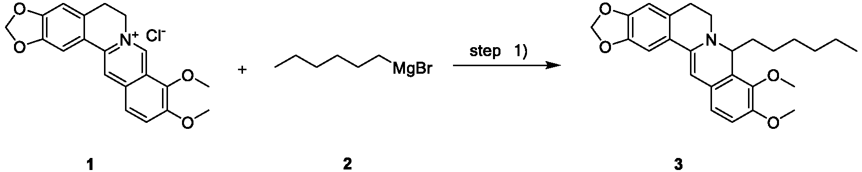

Compound 1 (100 mg, 0.31 mmol) was dissolved in tetrahydrofuran (20 mL), and compound 2 (1 mL, 1 mmol/L) was added, the mixture was stirred at room temperature for overnight, new spot was detected on a plate. The mixture was quenched with water and then filtered with diatomite, the filtrate was concentrated under reduced pressure to remove organic solvent, the remaining aqueous phase was filtered to afford solid compound 3 (100 mg).

### Step 2):

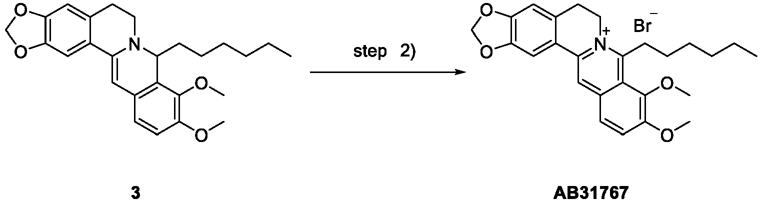

Compound 3 (100 mg, 0.31 mmol) was dissolved in acetic acid (2 mL), and heated to 120 °C, a mixture solution of liquid bromine (50 mg, 0.31 mmol) in acetic acid (1 mL) was then added. The mixture was stirred for 1 h, new spot was detected on a plate. The mixture was quenched with water and then filtered with diatomite, the filtrate was concentrated under reduced pressure to remove organic solvent, the remaining aqueous phase was then filtered to afford solid compound AB31767 (27mg).

MS-ESI: Calculated: [M-Br⁻]⁺ : 420.53, Found: 420.15.

1H NMR (400 MHz,DMSO-d6) δ 8.77 (s, 1H), 8.16 (s, 1H), 8.00 (s, 1H), 7.71 (s, 1H), 7.09 (s, 1H), 6.15 (s, 2H), 4.79 (s, 2H), 4.03 (s, 6H), 3.74 (s, 2H), 3.17 - 3.12 (m, 2H), 1.76 (s, 2H), 1.56 (s, 2H), 1.36 (s, 4H), 0.90 (s, 3H).

### Example 25 Synthesis of compound AB31771

The structure of compound AB31771 was as follows:

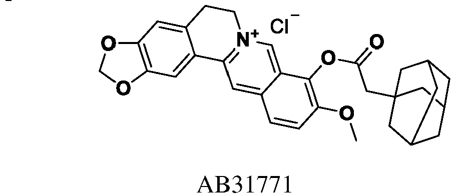

### Step 1):

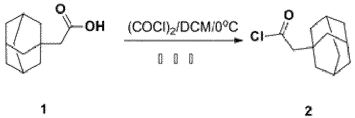

Compound 1 (190 mg, 1 mmol) was dissolved in dichloromethane (10 mL), oxaloyl chloride (254 mg, 2 mmol) was added at 0 °C, and two drops of N, N-dimethylformamide were added. The mixture was stirred at room temperature for 2 h, and concentrated under reduced pressure to remove organic solvent to afford solid compound 2 (210 mg).

### Step 2):

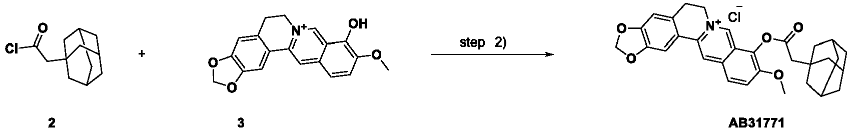

Compound 2 (210 mg, 1 mmol) was dissolved in acetonitrile (10 mL) and compound 3 (100 mg, 0.31 mmol) was added. The mixture was stirred at room temperature for 2 h, and concentrated under reduced pressure to remove organic solvent, the residue was purified by column chromatography (dichloromethane: methanol=30:1) to afford solid compound AB31771.

MS-ESI: Calculated: [M-Cl⁻]⁺ : 498.60, Found: 498.20.

1HNMR (400 MHz, DMSO) δ 9.85 (s, 1H), 9.00 (s, 1H), 8.26 (d, J = 9.2 Hz, 1H), 8.17 (d, J = 9.3 Hz, 1H), 7.78 (s, 1H), 7.07 (s, 1H), 6.15 (s, 2H), 4.90 (s, 2H), 4.01 (s, 3H), 3.19 (s, 2H), 2.54 (s, 2H), 1.99 (s, 3H), 1.76 (s, 4H), 1.70 - 1.63 (m, 6H), 1.54 (s, 2H).

### Example 26 Synthesis of compound AB31780

The structure of compound AB31780 was as follows:

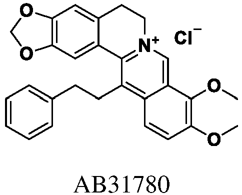

### Step 1):

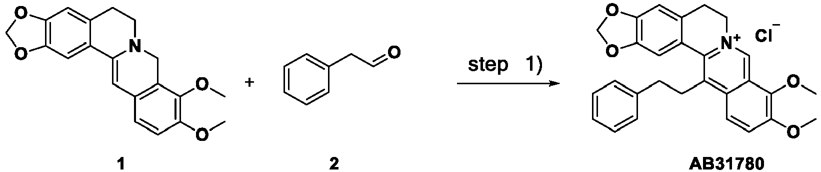

Compound 1 (337 mg, 1 mmol) was dissolved in 80% ethanol (5 mL), and acetic acid (4 mL) and compound 2 (1.20 g, 10 mmol) were added. The mixture was stirred at 90 °C for 4 h, new spot was detected on a plate. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31780 (64.2 mg).

MS-ESI: Calculated: [M-Cl⁻]⁺ : 440.51, Found: 440.35.

1H NMR (400 MHz, DMSO-d6) : δ 9.83 (s, 1H), 8.34 (d, J = 9.3 Hz, 1H), 8.20 (d, J = 9.4 Hz, 1H), 7.39 (s, 1H), 7.16 (d, J = 7.2 Hz, 3H), 7.02 (d, J = 17.1 Hz, 3H), 6.13 (s, 2H), 4.69 (s, 2H), 4.08 (s, 6H), 3.72 (s, 2H), 2.98 (s, 2H), 2.83 (s, 2H).

### Example 27 Synthesis of compound AB31781

The structure of compound AB31781 was as follows:

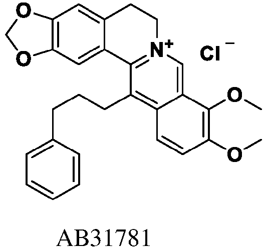

### Step 1):

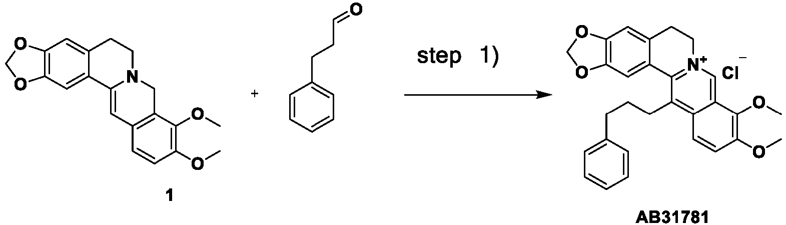

Compound 1 (337 mg, 1 mmol) was dissolved in 80% ethanol (5 mL), and acetic acid (4 mL) and phenylpropionaldehyde (1.2 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31781 (215.4 mg).

MS-ESI: Calculated: [M-Cl⁻]⁺ : 454.54, Found: 454.30.

1HNMR (400 MHz, DMSO-6) δ 9.85 (s, 1H), 8.13 (s, 2H), 7.25-7.19 (m, 3H), 7.14 (dd, J = 12.6, 7.2 Hz, 4H), 6.14 (s, 2H), 4.75 (s, 2H), 4.05 (d, J = 1.2 Hz, 6H), 3.32 (s, 2H), 3.07 -3.02 (m, 2H), 2.66 (t, J = 7.0 Hz, 2H), 2.08 - 1.96 (m, 2H).

### Example 28 Synthesis of compound AB31787

The structure of compound AB31787 was as follows:

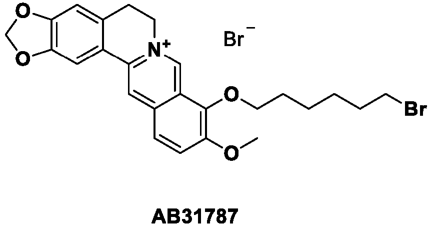

### Step 1):

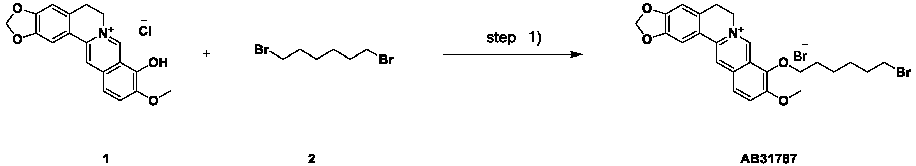

Compound 1 (322 mg, 1 mmol) was dissolved in N, N-dimethylformamide (10 mL), and compound 2 (975 mg, 4.0 mmol) was added. The mixture was stirred at 80 °C for overnight, new spot was detected on a plate. The reaction mixture was diluted with water and then extracted with ethyl acetate, the combined organic phase was filtered and concentrated under reduced pressure to remove organic solvent, the residue was purified by column chromatography and triturated with ethyl acetate to afford solid compound AB31787 (27.3 mg, 0.05 mmol, yield: 5.6%).

MS-ESI: Calculated: [M-Br-]+ : 485.39, Found: 486.05.

1H NMR (400 MHz, DMSO-d6) δ 9.70 (s, 1H), 8.89 (s, 1H), 8.16 (d, J = 9.1 Hz, 1H), 7.96 (d, J = 8.9 Hz, 1H), 7.76 (s, 1H), 7.06 (s, 1H), 6.14 (s, 3H), 4.90 (s, 2H), 4.25 (t, J = 6.6 Hz, 2H), 4.02 (s, 3H), 3.17 (s, 3H), 1.83 (s, 4H), 1.47 (s, 4H).

### Example 29 Synthesis of compound AB31788

The structure of compound AB31788 was as follows:

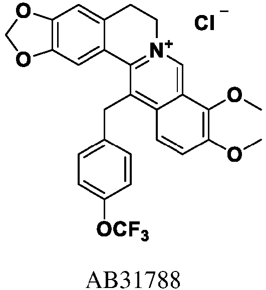

### Step 1):

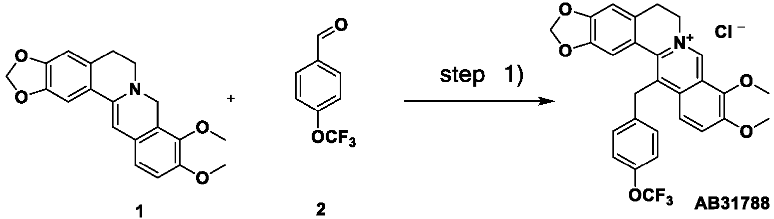

Compound 1 (337 mg, 1 mmol) was dissolved in 80% ethanol (5 mL), and acetic acid (4 mL) and compound 2 (1.20 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31788 (17.5 mg).

MS-ESI: Calculated: [M-Cl⁻]⁺ : 510.48, Found: 510.25.

1H NMR (400 MHz, dmso) δ 10.00 (s, 1H), 8.07 (d, J = 9.2 Hz, 1H), 7.75 (d, J = 9.0 Hz, 1H), 7.33 (d, J = 8.2 Hz, 2H), 7.27 (s, 2H), 7.14 (s, 1H), 6.87 (s, 1H), 6.05 (s, 2H), 4.79 (d, J = 29.0 Hz, 4H), 4.08 (s, 3H), 3.99 (s, 3H), 3.12 (s, 2H).

### Example 30 Synthesis of compound AB31789

The structure of compound AB31789 was as follows:

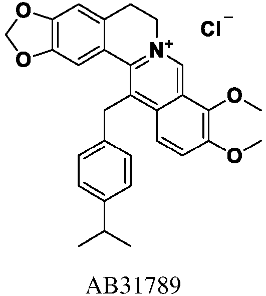

### Step 1):

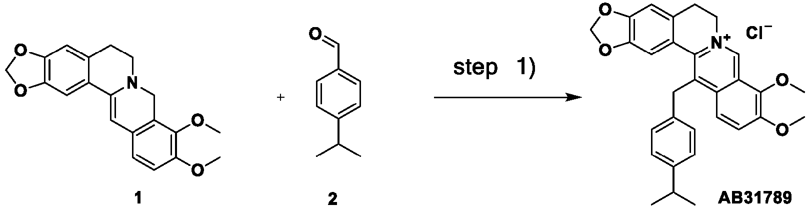

Compound 1 (337 mg, 1 mmol) was dissolved in 80% ethanol (10 mL), and acetic acid (8 mL) and compound 2 (1.20 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31789.

MS-ESI: Calculated: [M-Cl⁻]⁺: 468.56, Found: 468.35.

1H NMR (400MHz, DMSO-6) δ 9.97 (s, 1H), 8.06 (d, J = 9.4 Hz, 1H), 7.75 (d, J = 9.5 Hz, 1H), 7.20 (d, J = 8.1 Hz, 2H), 7.13 (s, 1H), 7.04 (d, J = 7.7 Hz, 2H), 6.96 (s, 1H), 6.04 (s, 2H), 4.83 (s, 2H), 4.66 (s, 2H), 4.08 (s, 3H), 3.99 (s, 3H), 3.11 (s, 2H), 2.84 (s, 1H), 1.16 (s, 3H), 1.14 (s, 3H).

### Example 31 Synthesis of compound AB31790

The structure of compound AB31790 was as follows:

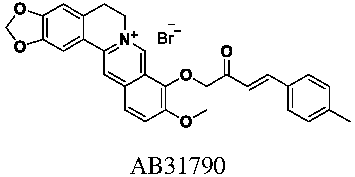

### Step 1):

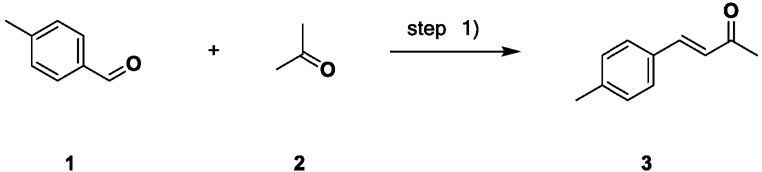

Compound 1 (5 g, 41 mmol) was dissolved in sodium hydroxide aqueous solution (10 mL, 2 mol/L, and compound 2 (10 mL) was added. The mixture was stirred at 60 °C for 2 h, new spot was detected on a plate, the reaction was completed, appropriate amount of water and ethyl acetate were added, the mixture was extracted, the combined organic phase was filtered and concentrated under reduced pressure to remove organic solvent to afford compound 3 (6 g).

### Step 2):

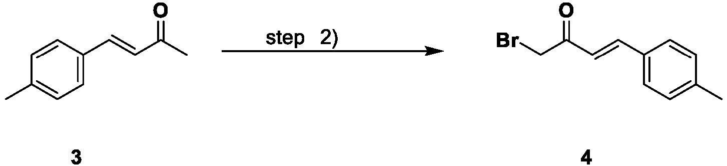

Compound 3 (1 g, 6.25 mmol) was dissolved in tetrahydrofuran (40 mL), and pyrrolidone tribromide (2.2 g, 12.5 mmol) was added. The mixture was stirred at room temperature for 16 h, new spot was detected on a plate, the reaction was completed, appropriate amount of water and ethyl acetate were added, the mixture was extracted, the combined organic phase was filtered and concentrated under reduced pressure to remove organic, the residue was purified by column chromatography (dichloromethane: methanol=30:1) to afford solid compound 4 (440 mg).

### Step 3) :

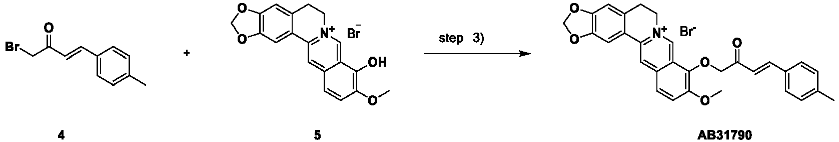

Compound 4 (470 mg, 2 mmol) was dissolved in acetonitrile (20 mL), and compound 5 (322 g, 1 mmol) was added. The mixture was stirred at 80 °C for 16 h, new spot was detected on a plate, the mixture was filtered to afford solid compound AB31790 (356 mg).

MS-ESI: Calculated: [M-Br-]+ : 480.53, Found: 480.30.

1H NMR (400 MHz, DMSO-d6) δ 9.99 (s, 1H), 8.91 (s, 1H), 8.15 (d, J = 9.0 Hz, 1H), 7.96 (d, J = 9.1 Hz, 1H), 7.78 (s, 1H), 7.63 (dd, J = 19.8, 12.1 Hz, 3H), 7.24 (d, J = 8.2 Hz, 2H), 7.09 - 6.97 (m, 2H), 6.15 (s, 2H), 5.39 (s, 2H), 4.91 (s, 2H), 3.98 (s, 3H), 3.19 (s, 2H), 2.31 (s, 3H).

### Example 32 Synthesis of compound AB31794

The structure of compound AB31794 was as follows:

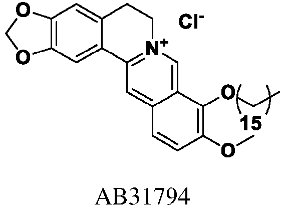

### Step 1):

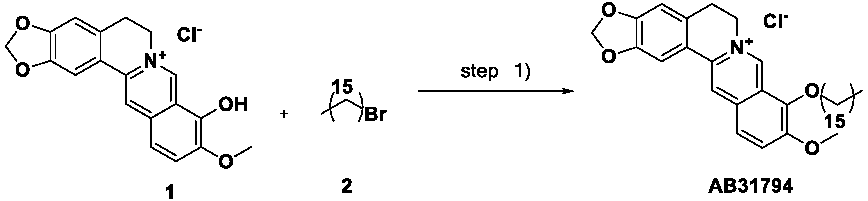

Compound 1 (200 mg, 0.6 mmol) was dissolved in DMF (5 mL), and compound 2 (759 mg, 2.5 mmol) was added. The mixture was stirred at 90 °C for 4 h, new spot was detected on a plate, the reaction was completed, appropriate amount of water and ethyl acetate were added, the mixture was extracted, the combined organic phase was filtered and concentrated under reduced pressure to remove organic solvent, the residue was purified by column chromatography (dichloromethane: methanol=30:1) to afford solid compound AB31794 (213.1 mg).

MS-ESI: Calculated: [M-Cl⁻]⁺ :546.76, Found:546.50.

1H NMR (400 MHz, DMSO-d6) δ 9.72 (s, 1H), 8.90 (s, 1H), 8.17 (d, J = 9.1 Hz, 1H), 7.95 (d, J = 9.2 Hz, 1H), 7.77 (s, 1H), 7.06 (s, 1H), 6.15 (s, 2H), 4.91 (s, 2H), 4.25 (t, J = 6.8 Hz, 2H), 4.02 (s, 3H), 3.17 (s, 2H), 1.88-1.79 (m, 2H), 1.44 (s, 2H), 1.20 (s, 24H), 0.82 (t, J = 6.8 Hz, 3H).

### Example 33 Synthesis of compound AB31795

The structure of compound AB31795 was as follows:

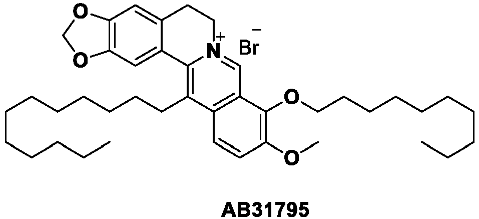

### Step 1):

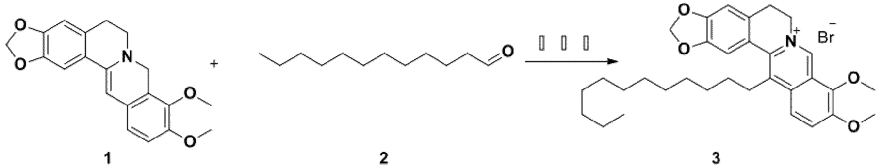

Compound 1 (3.38 g, 10 mmol) was dissolved in 80% ethanol (50 mL), and acetic acid (40 mL) and compound 2 (18.40 g, 100 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound 3 (3 g, 5.95mmol).
MS-ESI: Calculated: [M-Br-]+ : 504.68, Found: 504.69
step2)

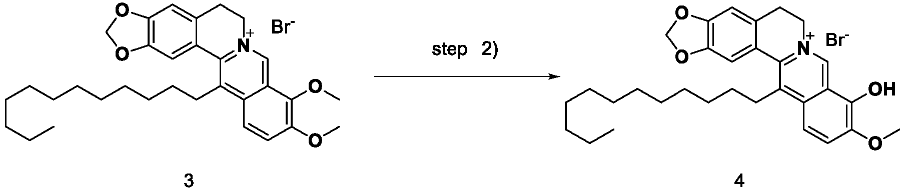

Compound 3 (3 g, 5.95 mmol) was heated to 190 °C, the reaction was performed for 1 h to afford solid compound 4 (2.5 g, 5.1 mmol).
MS-ESI: Calculated: [M-Br-]+ : 490.66, Found: 490.66

### Step 3)

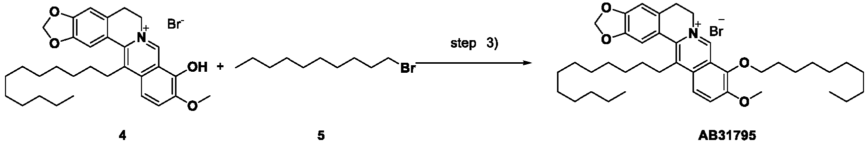

Compound 1 (490 mg, 1 mmol) was dissolved in N, N-dimethylformamide (10 mL), and compound 5 (2.2g, 10 mmol) was added. The mixture was replaced with N₂ three times and stirred at 80 °C for 4 h, and the reaction was detected to be performed completely using LCMS. The reaction mixture was diluted with water and then extracted with dichloromethane three times, the combined organic phase was washed with saturated brine, dried, filtered and concentrated, the concentrate was purified by column chromatography (dichloromethane: methanol=20:1) to afford solid compound AB31795 (164.8 mg).

MS-ESI: Calculated: [M-Br-]+ : 630.93, Found: 630.45.

1H NMR (400 MHz, DMSO-d6) δ 9.73 (s, 1H), 8.16 (s, 2H), 7.26 (s, 1H), 7.14 (s, 1H), 6.16 (s, 2H), 4.78 (s, 2H), 4.24 (d, J = 6.5 Hz, 2H), 4.04 (s, 4H), 3.05 (s, 3H), 1.83 (d, J = 7.4 Hz, 2H), 1.72 (s, 2H), 1.44 (s, 2H), 1.20 (s, 27H), 0.83 (s, 6H).

### Example 34 Synthesis of compound AB31796

The structure of compound AB31796 was as follows:

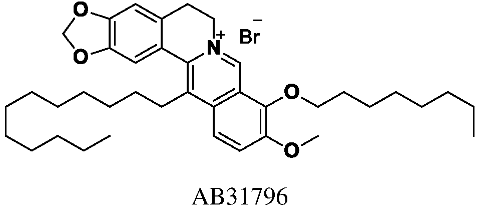

### Step 1):

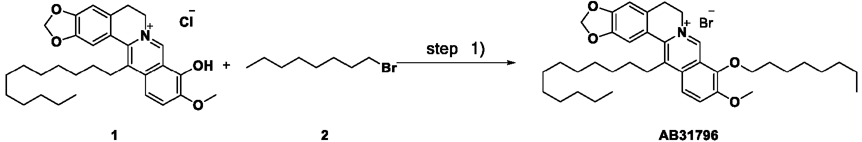

Compound 1 (490 mg, 1 mmol) was dissolved in N, N-dimethylformamide (10 mL), and compound 2 (800 mg, 4 mmol) was added. The mixture was replaced with N₂ three times and stirred at 80 °C for 16 h, and the reaction was detected to be performed completely using LCMS. The reaction mixture was diluted with water and then extracted with dichloromethane three times, the combined organic phase was washed with saturated brine, dried, filtered and concentrated, the concentrate was purified by column chromatography (dichloromethane: methanol=50:1) to afford solid compound AB31796 (322 mg).

MS-ESI: Calculated: [M-Br-]+ : 602.88, Found: 602.45.

1H NMR (400MHz, DMSO-d6) δ 9.72 (s, 1H), 8.15 (s, 2H), 7.26 (s, 1H), 7.13 (s, 1H), 6.15 (s, 2H), 4.77 (s, 2H), 4.24 (t, J = 6.8 Hz, 2H), 4.03 (s, 3H), 3.04 (s, 2H), 1.89 - 1.78 (m, 2H), 1.72 (s, 2H), 1.44 (s, 2H), 1.34 - 1.14 (m, 28H), 0.82 (t, J = 6.6 Hz, 6H).

### Example 35 Synthesis of compound AB31797

The structure of compound AB31797 was as follows:

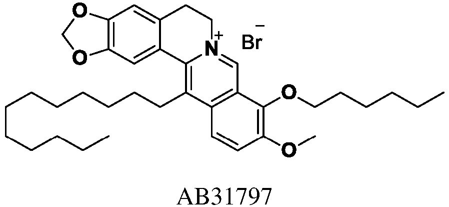

### Step 1):

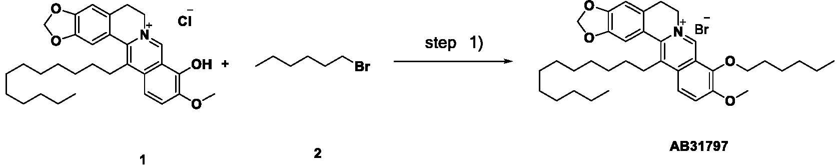

Compound 1 (490 mg, 1 mmol) was dissolved in N, N-dimethylformamide (10 mL), and compound 2 (700 mg, 4 mmol) was added. The mixture was replaced with N₂ three times and stirred at 80 °C for 16 h, and the reaction was detected to be performed completely using LCMS. The reaction mixture was diluted with water and then extracted with dichloromethane three times, the combined organic phase was washed with saturated brine, dried, filtered and concentrated, the concentrate was purified by column chromatography (dichloromethane: methanol=20:1) to afford solid compound AB31797 (354 mg, yield: 61.67%).

MS-ESI: Calculated: [M-Br-]+ : 574.83, Found: 574.40.

1H NMR (400 MHz, DMSO-d6) δ 9.73 (s, 1H), 8.16 (s, 2H), 7.26 (s, 1H), 7.13 (s, 1H), 6.15 (s, 2H), 4.78 (s, 2H), 4.25 (t, J = 6.8 Hz, 2H), 4.04 (s, 3H), 3.05 (s, 2H), 1.89 -1.79 (m, 2H), 1.72 (s, 2H), 1.45 (s, 2H), 1.32 (s, 6H), 1.20 (s, 18H), 0.83 (dd, J = 15.7, 9.6 Hz, 6H).

### Example 36 Synthesis of compound AB31800

The structure of compound AB31800 was as follows:

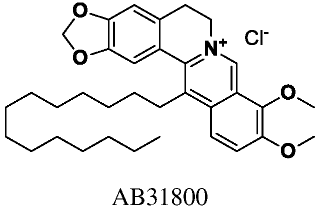

### Step 1):

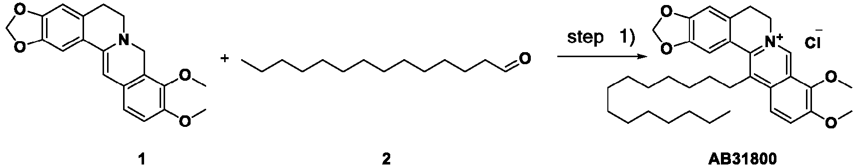

Compound 1 (338 mg, 1 mmol) was dissolved in 80% ethanol (5 mL), and acetic acid (4 mL) and compound 2 (2.12 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31800 (204 mg).

MS-ESI: Calculated: [M-Cl⁻]⁺ : 532.74, Found: 532.60.

1H NMR (400 MHz, DMSO-d6) δ 9.87 (s, 1H), 8.17 (s, 2H), 7.26 (s, 1H), 7.13 (s, 1H), 6.15 (s, 2H), 4.76 (s, 2H), 4.07 (s, 6H), 3.05 (s, 2H), 1.71 (s, 2H), 1.34 (s, 2H), 1.20 (s, 22H), 0.81 (d, J = 6.9 Hz, 3H).

### Example 37 Synthesis of compound AB31805

The structure of compound AB31805 was as follows:

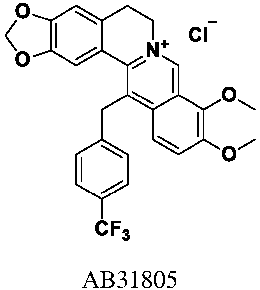

### Step 1):

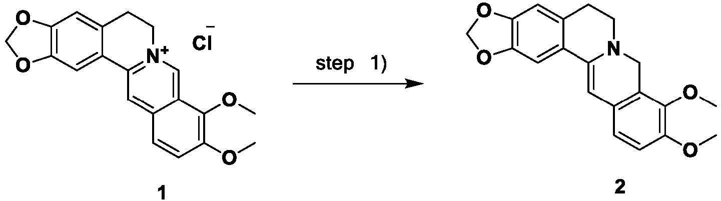

Compound 1 (10 g, 26.88 mmol) was dissolved in methanol (100 mL), and potassium carbonate (11.13 g, 80.64 mmol) and sodium borohydride (326.86 mg, 8.6 mmol) were added. The mixture was stirred at room temperature for 1 h, new spot was detected on a plate. The reaction mixture was diluted with water and then extracted with ethyl acetate, the combined organic phase was filtered and concentrated under reduced pressure to remove organic solvent to afford solid compound 2 (8.5 g).

### Step 2):

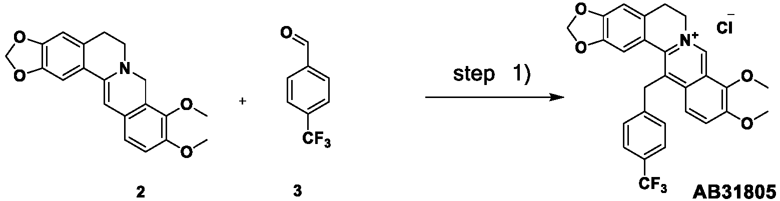

Compound 2 (337 mg, 1 mmol) was dissolved in 80% ethanol (5 mL), and acetic acid (4 mL) and compound 3 (1.2 g, 10 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31805 (28.2 mg).

MS-ESI: Calculated: [M-Cl⁻]⁺ : 494.48, Found: 494.60.

1H NMR (400 MHz, DMSO-6) δ 10.04 (s, 1H), 8.07 (d, J = 9.5 Hz, 1H), 7.75-7.68 (m, 3H), 7.39 (s, 2H), 7.15 (s, 1H), 6.84 (s, 1H), 6.06 (s, 2H), 4.83 (s, 4H), 4.09 (s, 3H), 4.00 (s, 3H), 3.13 (s, 2H).

### Example 38 Synthesis of compound AB31808

The structure of compound AB31808 was as follows:

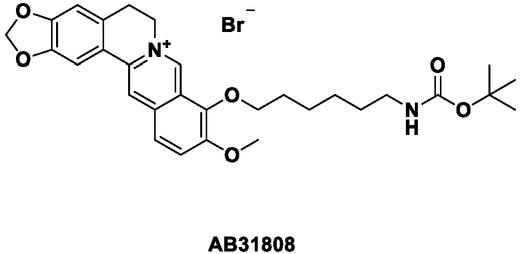

### Step 1):

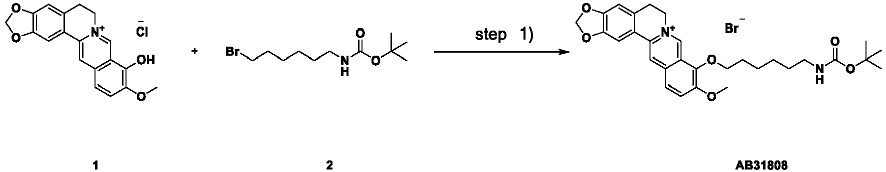

Compound 1 (200 mg, 0.6 mmol) was dissolved in N, N-dimethylformamide (10 mL), and compound 2 (348 mg, 1.2 mmol) was added. The mixture was stirred at 80 °C for overnight, new spot was detected on a plate. The reaction mixture was diluted with water and then extracted with ethyl acetate, the combined organic phase was filtered and concentrated under reduced pressure to remove organic solvent, the residue was purified by column chromatography to afford solid compound AB31808 (73.8 mg, 0.1 mmol).

MS-ESI: Calculated: [M-Br-]+ : 521.62, Found: 521.50.

1H NMR (400 MHz, DMSO-d6) δ 9.72 (s, 1H), 8.91 (s, 1H), 8.17 (d, J = 9.4 Hz, 1H), 7.96 (d, J = 9.2 Hz, 1H), 7.77 (s, 1H), 7.06 (s, 1H), 6.76 (s, 1H), 6.14 (s, 2H), 4.92 (s, 2H), 4.25 (s, 2H), 4.02 (s, 3H), 3.18 (s, 2H), 2.89 (d, J = 5.9 Hz, 2H), 1.83 (s, 2H), 1.42 (d, J = 22.7 Hz, 4H), 1.33 (s, 9H), 1.20 (s, 2H).

### Example 39 Synthesis of compound AB31809

The structure of compound AB31809 was as follows:

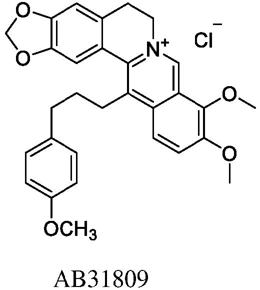

### Step 1):

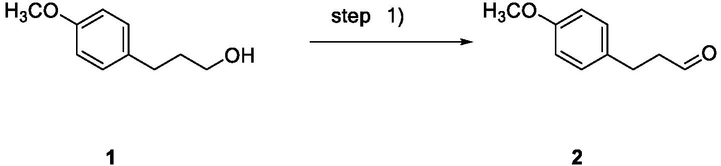

Compound 1 (2.0g, 12.03mmol) was dissolved in dichloromethane (10 mL), and Dess-Martin periodinane (6.12 g, 14.53 mmol) was added under ice bath condition. The mixture was stirred at 0 °C for 2 h, new spot was detected on a plate, the reaction was completed. The reaction mixture was filtered, the filtrate was quenched with sodium thiosulfate solution, the mixture was extracted, the organic solvent was spin dried, the residue was purified by column chromatography (petroleum ether: ethyl acetate=50:1) to afford compound 2 (1.7 g).

### Step 2):

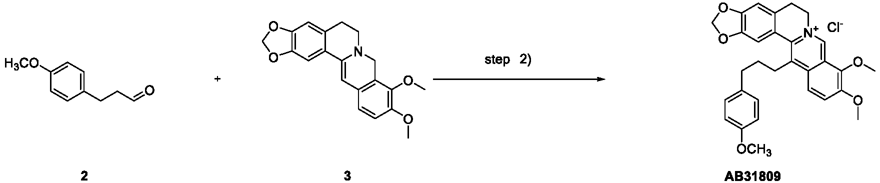

Compound 2 (1.7 g, 10.36 mmol) was dissolved in 80% ethanol (5 mL), and acetic acid (4 mL) and compound 3 (381 mg, 1.036 mmol) were added. The mixture was stirred at 90 °C for 4 h, new spot was detected on a plate, the reaction was completed, appropriate amount of hydrochloric acid was added dropwise. The reaction mixture was diluted with water and then extracted with ethyl acetate, the combined organic phase was filtered and concentrated under reduced pressure to remove organic solvent, the residue was purified by column chromatography (dichloromethane: methanol=20:1) to afford solid compound AB31809 (220 mg).

MS-ESI: Calculated: [M-Cl⁻]⁺ : 484.57, Found: 484.90.

1H NMR (400 MHz,DMSO-d6) δ 9.91 (s, 1H), 8.13 (d, J = 3.4 Hz, 2H), 7.14 (d, J = 16.0 Hz, 2H), 7.03 (d, J = 8.1 Hz, 2H), 6.77 (d, J = 8.2 Hz, 2H), 6.13 (s, 2H), 4.78 (s, 2H), 4.06 (d, J = 2.5 Hz, 6H), 3.67 (s, 3H), 3.29 (s, 2H), 3.05 (s, 2H), 2.59 (s, 2H), 1.98 (s, 2H).

### Example 40 Synthesis of compound AB35511

The structure of compound AB35511 was as follows:

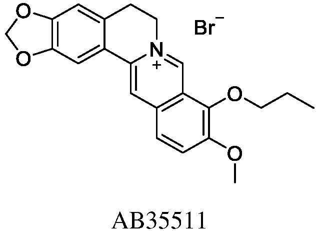

### Step 1):

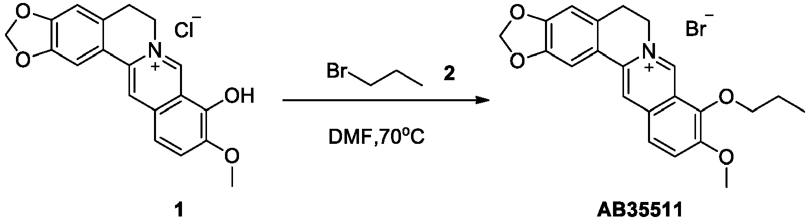

Compound 1 (1 g, 2.79 mmol, 1 eq) was dissolved in N, N-dimethylformamide (20 mL), and compound 2 (1.7 g, 13.95 mmol, 5 eq) was added. The mixture was stirred at 70 °C for overnight. The reaction mixture was diluted with water and then extracted with dichloromethane twice, the combined organic phase was washed with brine, dried over anhydrous sodium sulfate and filtered, the filtrate was spin dried. The crude product was purified by fast chromatography (DCM/MeOH=50/1 to 30/1) to afford compound AB 35511 (136.5 mg) as a yellow solid.

MS-ESI: Calculated [M]⁺: 364.15; Found [M]⁺: 364.10.

¹H NMR (400 MHz, CDCl₃) δ 10.18 (s, 1H), 8.31 (s, 1H), 7.85-7.73 (m, 2H), 7.34 (s, 1H), 6.77 (s, 1H), 6.05 (s, 2H), 5.27-5.24 (m, 2H), 4.45-4.42 (m, 2H), 4.01 (s, 3H), 3.34-3.30 (m, 2H), 2.07-2.01 (m, 2H), 1.12-1.08 (m, 3H).

### Example 41 Synthesis of compound AB36412

The structure of compound AB36412 was as follows:

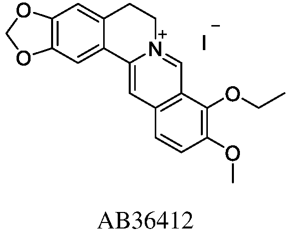

### Step 1):

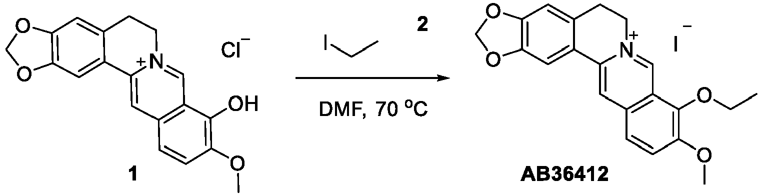

Compound 1 (500 mg, 1.4 mmol, 1.0 eq) was dissolved in N, N-dimethylformamide (10 mL), and compound 2 (1.9 g, 11.2 mmol, 8.0 eq) was added. The mixture was stirred at 70 °C for 16 h. The reaction mixture was concentrated, the crude product was purified by fast chromatography (dichloromethane/methanol=20:1) to afford compound AB 36412 (78.5 mg) as a yellow solid.

MS-ESI: Calculated [M]⁺: 350.14, Found: 350.05.

¹H NMR (400 MHz, DMSO-d6) δ 9.77 (s, 1H), 8.89 (s, 1H), 8.16 (d, *J=* 4.0 Hz, 1H), 7.95 (d, *J=* 4.0 Hz, 1H), 7.76 (s, 1H), 7.06 (s, 1H), 6.14 (s, 2H), 4.93-4.90 (m, 2H), 4.35-4.30 (m, 2H), 4.02 (s, 3H), 3.19-3.16 (m, 2H), 1.43-1.40 (m, 3H).

### Example 42 Synthesis of compound AB31755

Compound AB31755 was prepared by conventional method, the structure of compound AB31755 was as follows:

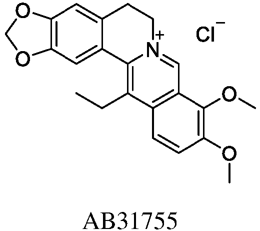

The ¹H NMR (400 MHz, DMSO-d6) spectrum of compound AB31755 was shown in Fig. 1.

### Example 43 Synthesis of compound AB31756

Compound AB31756 was prepared by conventional method, the structure of compound AB31756 was as follows:

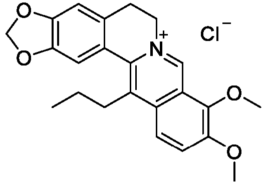

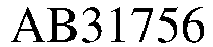

The ¹H NMR (400 MHz, DMSO-d6) spectrum of compound AB31756 was shown in Fig. 2.

### Example 44 Synthesis of compound AB35595

The structure of compound AB35595 was as follows:

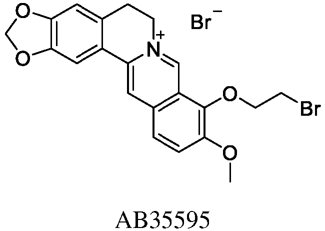

### Step 1):

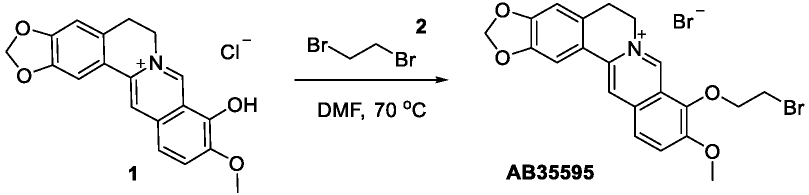

Compound 1 (500 mg, 1.4 mmol, 1.0 eq) was dissolved in N, N-dimethylformamide (10 mL), and compound 2 (2.1 g, 11.2 mmol, 8.0 eq) was added. The mixture was stirred at 70 °C for 16 h. The reaction mixture was concentrated, the crude product was purified by fast chromatography (dichloromethane/methanol=20:1) to afford compound AB 35595 (33.1 mg) as a yellow solid.

MS-ESI: Calculated [M+H]⁺: 428.05, Found: 428.15.

¹H NMR (400 MHz, DMSO-d6) δ 9.85 (s, 1H), 8.95 (s, 1H), 8.20 (d, *J=* 8.0 Hz, 1H), 8.02 (d, *J=* 8.0 Hz, 1H), 7.79 (s, 1H), 7.09 (s, 1H), 6.17 (s, 2H), 4.95-4.92 (m, 2H), 4.62-4.59 (m, 2H), 4.06 (s, 3H), 3.98-3.95 (m, 2H), 3.23-3.20 (m, 2H).

### Example 45 Synthesis of compound AB36404

The structure of compound AB36404 was as follows:

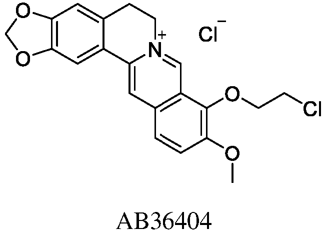

### Step 1):

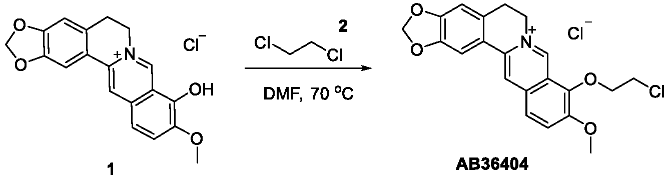

Compound 1 (1 g, 2.79 mmol, 1.0 eq) was dissolved in N, N-dimethylformamide (30 mL), and compound 2 (2.2 g, 22.36 mmol, 8.0 eq) was added. The mixture was stirred at 70 °C for 16 h. The reaction mixture was concentrated, the crude product was purified by fast chromatography (dichloromethane/methanol=20:1) to afford compound AB 36404 (35.5 mg) as a yellow solid.

MS-ESI: Calculated [M+H]⁺: 384.10, Found: 384.00.

¹H NMR (400 MHz, DMSO-d6) δ 9.82 (s, 1H), 8.94 (s, 1H), 8.22-8.01 (m, 2H), 7.78 (s, 1H), 7.09 (s, 1H), 6.17 (s, 2H), 4.98-4.89 (m, 2H), 4.57-4.55 (m, 2H), 4.13-4.10 (m, 2H), 4.07 (s, 3H), 3.26-3.17 (m, 2H).

### Example 46 Synthesis of compound AB35483

The structure of compound AB35483 was as follows:

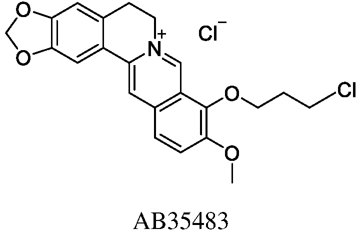

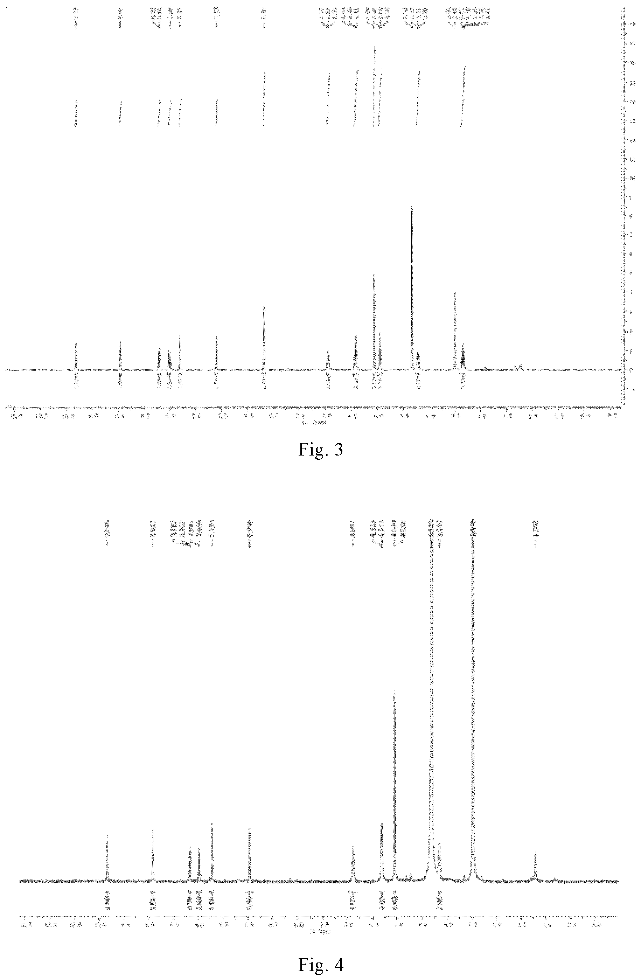

The ¹H NMR (400 MHz, DMSO-d6) spectrum of compound AB35483 was shown in Fig. 3.

### Example 47 Synthesis of compound AB35532

The structure of compound AB35532 was as follows:

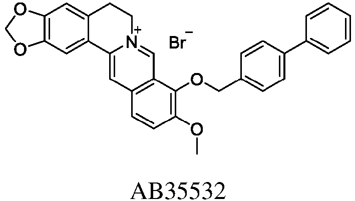

The synthesis route was as follows:

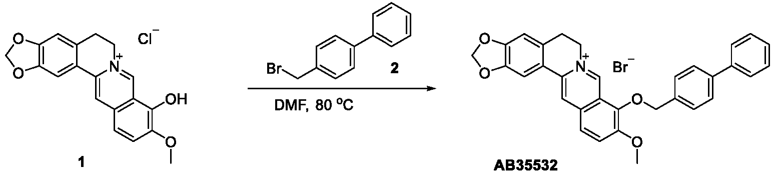

Compound 1 (200 mg, 0.56 mmol, 1.0 eq) was dissolved in N, N-dimethylformamide (10 mL), and compound 2 (692 mg, 2.8 mmol, 5 eq) was added. The mixture was stirred at 80 °C for overnight. The reaction mixture was diluted with water and then extracted with dichloromethane twice, the combined organic phase was washed with brine, dried over anhydrous sodium sulfate and filtered, the filtrate was spin dried. The crude product was purified by fast chromatography (DCM/MeOH=50/1 to 30/1) to afford compound AB 35532 (18.1 mg) as a yellow solid.

MS-ESI: Calculated [M]⁺: 488.19; Found [M]⁺: 488.15.

¹H NMR (400 MHz, DMSO-d6) δ 9.76 (s, 1H), 8.91 (s, 1H), 8.21 (d, *J=* 8.0 Hz, 1H), 7.97 (d, *J=* 8.0 Hz, 1H), 7.75 (s, 1H), 7.64-7.62 (m, 6H), 7.48-7.33 (m, 3H), 7.06 (s, 1H), 6.15 (s, 2H), 5.38 (s, 2H), 4.92-4.89 (m, 2H),4.08 (s, 3H), 3.18-3.14 (m, 2H).

### Example 48 Synthesis of compound AB36425

The structure of compound AB36425 was as follows:

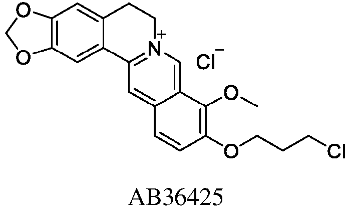

The synthesis route was as follows:

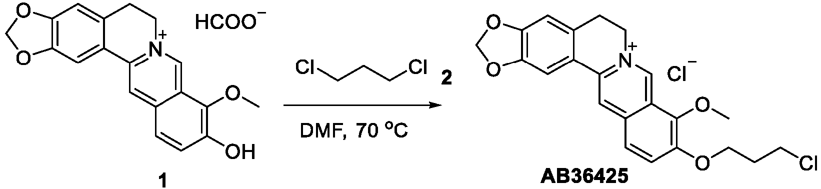

Compound 1 (160 mg, 0.43 mmol, 1 eq) was dissolved in N, N-dimethylformamide (5 mL), and compound 2 (511 mg, 2.18 mmol, 5 eq) was added. The mixture was stirred at room temperature for 16 h under N₂. The reaction mixture was cooled and spin dried, the crude product was purified by fast chromatography (dichloromethane : methanol=50: 1) to afford compound AB 36425 (39 mg) as a yellow solid.

MS-ESI: Calculated [M]⁺: 398.12; Found [M]⁺: 398.20.

¹H NMR (400 MHz, DMSO-d6) δ 9.90 (s, 1H), 8.95 (s, 1H), 8.23-7.97 (m, 2H), 7.80 (s, 1H), 7.09 (s, 1H), 6.18 (s, 2H), 4.95-4.92 (m, 2H), 4.43-4.40 (m, 2H), 4.12 (s, 3H), 3.90-3.87 (m, 2H), 3.22-3.19 (m, 2H), 2.33-2.27 (m, 2H).

### Example 49 Synthesis of compound AB31815

The structure of compound AB31815 was as follows:

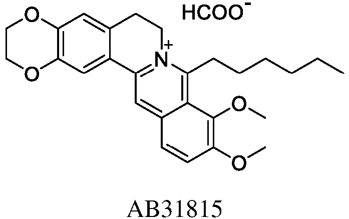

The synthesis route was as follows:

### Step (1)

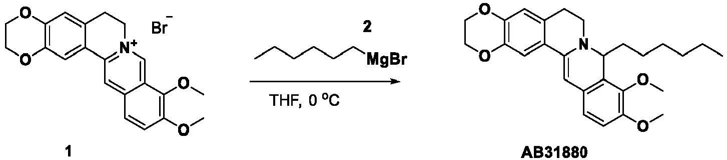

Compound 1 (300 mg, 0.70 mmol, 1 eq) was dissolved in anhydrous tetrahydrofuran (10 mL), and hexylmagnesium bromide (3.5 mL, 3.50 mmol, 5 eq, 1M/L) was slowly added dropwise under N₂ at 0 °C.The mixture was heated to room temperature and reacted for 0.5 h , and the reaction was detected to be performed completely using TLC and LCMS. The mixture was quenched with saturated ammonium chloride solution, and then extracted with ethyl acetate twice, the organic phase was dried over anhydrous sodium sulfate and spin dried, the crude product was purified by thin layer chromatography (dichloromethane: methanol=50:1) to afford compound AB 31880 (160 mg) as a yellow solid.

MS-ESI: Calculated [M+H]⁺: 436.24, Found: 436.20.

¹H NMR (400 MHz, CDCl₃) δ 7.19 (s, 1H), 6.75-6.68 (m, 2H), 6.62 (s, 1H), 5.81 (s, 1H), 4.70-4.67 (m, 1H), 4.26 (s, 4H), 3.87 (s, 3H), 3.84 (s, 3H), 3.46-3.45 (m, 1H), 3.31-3.29 (m, 1H), 2.87-2.78 (m, 2H), 1.75-1.50 (m, 4H), 1.38-1.26 (m, 6H), 0.82-0.79 (m, 3H).

### Step (2):

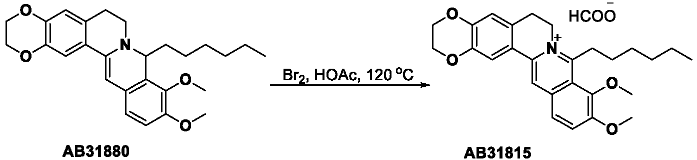

Compound AB31880 (80 mg, 0.18 mmol, 1 eq) was dissolved in acetic acid (3 mL), and three drops of bromine was added. The mixture was stirred at 120 °C for 1 h, the reaction was detected to be performed completely using LCMS. The reaction mixture was spin dried, the crude product was purified by reversed phase preparative chromatography to afford compound AB 31815 (3.6 mg) as a yellow solid.

MS-ESI: Calculated [M]⁺: 434.23, Found: 434.20.

¹H NMR (400 MHz, CDCl₃) δ 7.19 (s, 1H), 6.75-6.70 (m, 2H), 6.62 (s, 1H), 5.81 (s, 1H), 4.70-4.67 (m, 1H), 4.26 (s, 4H), 3.87 (s, 3H), 3.84 (s, 3H), 3.46-3.44 (m, 1H), 3.31-3.28 (m, 1H), 2.87-2.81 (m, 2H), 1.81 (s, 4H), 1.65 (s, 2H), 1.37-1.26 (m, 4H), 0.82-0.79 (m, 3H).

### Example 50 Synthesis of compound AB36508

The structure of compound AB36508 was as follows:

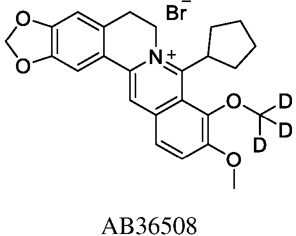

The synthesis route was as follows:

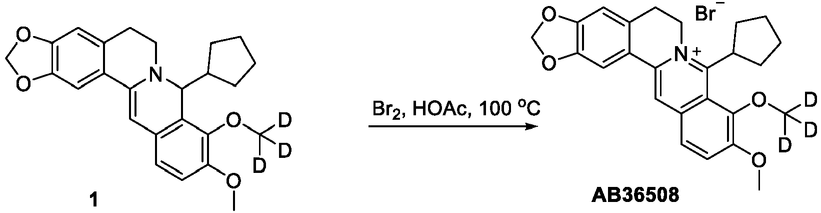

Compound 1 (200 mg, 0.49 mmol, 1.0 eq) was dissolved in acetic acid (10 mL), and bromine (118 mg, 0.74 mmol, 1.5 eq) was added. The mixture was stirred at 100 °C for 1 h. The reaction mixture was spin dried, the crude product was purified by reversed phase preparative chromatography (acetonitrile/water+0.1% formic acid) to afford compound AB 36508 (24 mg) as a yellow solid.

MS-ESI: Calculated [M]⁺: 407.20; Found: 407.25.

¹H NMR (400 MHz, DMSO-d6) δ 8.81 (s, 1H), 8.18 (d, *J=* 12.0 Hz, 1H), 8.00 (d, *J=* 8.0 Hz, 1H), 7.77 (s, 1H), 7.11 (s, 1H), 6.17 (s, 2H), 5.04-4.99 (m, 1H), 4.67-4.64 (m, 2H), 3.18-3.16 (m, 2H), 2.23-2.16 (m, 4H), 2.08-2.04 (m, 2H), 1.88-1.81 (m, 2H).

### Example 51 Synthesis of compound AB36484

The structure of compound AB36484 was as follows:

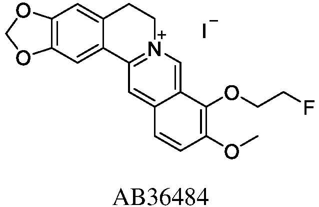

The synthesis route was as follows:
step (1) :

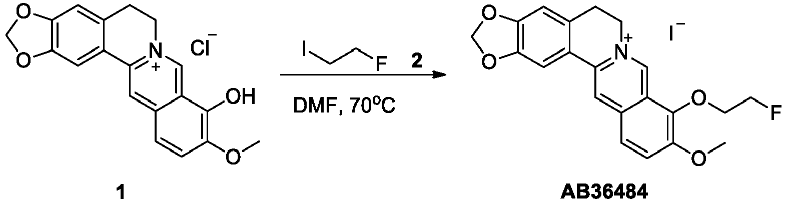

To a mixture of compound 1 (1g, 2.79 mmol, 1 eq) in DMF (20 mL) was added compound 2 (2.4 g, 13.97 mmol, 5 eq). The mixture was stirred at 70 °C for 16 h, concentrated. The mixture was concentrated and purified by FCC (DCM/ MeOH = 50/1) to get AB36484 (98.4 mg) as a yellow solid.

MS-ESI: Calculated [M]⁺: 368.13; Found[M]⁺: 368.00.

¹H NMR (400 MHz, DMSO-d6) δ 9.79 (s, 1H), 8.94 (s, 1H), 8.23-8.00 (m, 2H), 7.80 (s, 1H), 7.09 (s, 1H), 6.17 (s, 2H), 4.95-4.92 (m, 3H), 4.84-4.82 (m, 1H), 4.61-4.59 (m, 1H), 4.53-4.51 (m, 1H), 4.07 (s, 3H), 3.22-3.19 (m, 2H).

### Example 52 Synthesis of compound AB31811

The structure of compound AB31811 was as follows:

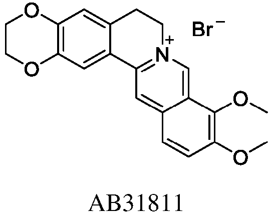

The ¹H NMR (400 MHz, DMSO-d6) spectrum of compound AB31811 was shown in Fig. 4.

### Example 53

The inhibitory effect of the compounds prepared in the Examples of the present invention on various tumor cell lines was detected using cell viability assay reagent.

### Experimental background:

Cell viability was detected using the Promega CellTiter-Glo kit, cell viability was determined by directly measuring intracellular ATP content. In the experiment, the IC₅₀ value of the compounds prepared in the Examples of the present invention on various tumor cell lines was detected.

### Experimental method and result:

Each tumor cell was cultured in relevant medium. After cell passage, the gradient diluted compounds prepared in the Examples of the present invention was added. After 3 days of culture, the relevant IC₅₀ (50% inhibiting concentration) was measured. The name, source and culture conditions of each tumor cell line were as follows:
Cell line NCI-H82 (ATCC, No. HTB-175) was cultured in 10% fetal bovine serum-containing RPMI1640 medium (+P/S).
Cell line G-401 (ATCC, No. CRL-1441) was cultured in 10% fetal bovine serum-containing McCoy's 5a medium (+P/S).
Cell line MDA-MB-453 (ATCC, No. HTB-131) was cultured in 10% fetal bovine serum-containing Leibovitz's L-15 medium (+P/S).
Cell line SW48 (ATCC, No. CCL-231) was cultured in 10% fetal bovine serum-containing Leibovitz's L-15 medium (+P/S).
Cell line CFPAC-1 (ATCC, No. CRL-1918) was cultured in 10% fetal bovine serum-containing IMDM medium (+P/S).
Cell line 786-0 (ATCC, No. CRL-1932) was cultured in 10% fetal bovine serum-containing RPMI1640 medium (+P/S).
Cell line GB-1 (JCRB, No. IFO50489) was cultured in 10% fetal bovine serum-containing DMEM medium (+P/S).
Cell line SF126 (JCRB, No. IFO50286) was cultured in 10% fetal bovine serum-containing EMEM medium (+P/S).

The experiment result was shown in Table 3 below:

**Table 3 inhibitory effect of the compounds prepared in the Examples of the present invention on different cell lines (IC₅₀, µM)**

| Compound no. | NCI-H82 | SW48 | G-401 | MDA-MB-453 | CFPAC-1 | 786-O | SF-126 | GB-1 |
|---|---|---|---|---|---|---|---|---|
| compound AB31706 | 1.48 | 1.24 | 1.12 | 0.62 | 21.94 | 16.57 | 6.49 | 12.92 |
| compound AB31707 | 1.17 | 1.42 | 1.12 | 0.62 | 13.05 | 7.63 | 10.18 | 8.27 |
| compound AB31711 | 0.65 | 0.82 | 0.72 | 0.50 | 9.47 | 3.97 | 5.21 | 5.32 |
| compound AB31718 | 0.29 | 0.25 | 0.37 | 0.09 | 5.29 | 2.69 | 4.25 | 3.41 |
| compound AB31721 | 0.40 | 0.80 | 1.38 | 0.43 | 7.17 | 9.16 | 10.83 | 10.05 |
| compound AB31722 | 2.81 | 2.06 | 3.94 | 1.41 | 24.42 | 26.18 | 20.55 | 40.45 |
| compound AB31723 | 3.94 | 2.56 | 5.31 | 3.45 | 22.57 | 21.54 | 19.28 | >50 |
| compound AB31731 | 0.52 | 0.62 | 0.77 | 0.22 | 8.98 | 3.76 | 6.19 | 6.64 |
| compound AB31737 | 0.24 | 0.20 | 0.17 | 0.09 | 4.60 | 2.34 | 4.14 | 2.66 |
| compound AB31739 | 0.45 | 0.43 | 0.53 | 0.37 | 6.07 | 3.05 | 3.57 | 3.97 |
| compound AB31740 | 0.49 | 0.34 | 0.93 | 0.20 | 12.40 | 5.13 | 7.77 | 7.74 |
| compound AB31748 | 0.32 | 0.42 | 0.32 | 0.32 | 4.95 | 3.93 | 3.53 | 3.21 |
| compound AB31757 | 0.88 | 0.65 | 0.74 | 0.68 | 29.92 | 15.17 | 16.03 | 17.50 |
| compound AB31758 | 0.36 | 0.49 | 0.42 | 0.40 | 8.32 | 5.41 | 5.14 | 6.47 |
| compound AB31759 | 0.27 | 0.31 | 0.31 | 0.31 | 8.15 | 3.18 | 3.94 | 3.47 |
| compound AB31760 | 0.15 | 0.21 | 0.15 | 0.25 | 2.71 | 1.60 | 3.89 | 3.67 |
| compound AB31765 | 1.34 | 1.16 | 1.27 | 1.07 | 21.66 | 9.09 | 9.80 | 14.83 |
| compound AB31766 | 2.47 | 1.21 | 2.89 | 1.25 | 15.22 | 12.95 | 12.93 | 9.12 |
| compound AB31767 | 0.26 | 0.37 | 0.18 | 0.27 | 4.31 | 2.10 | 3.70 | 3.61 |
| compound AB31771 | 1.87 | 1.37 | 2.04 | 0.35 | 17.12 | 20.76 | 15.71 | 11.23 |
| compound AB31780 | 0.66 | 0.79 | 0.88 | 0.90 | 17.87 | 8.05 | 9.75 | 12.55 |
| compound AB31781 | 0.33 | 0.40 | 0.37 | 0.76 | 11.28 | 5.34 | 7.48 | 8.60 |
| compound AB31787 | 0.34 | 0.48 | 0.49 | 0.28 | 6.63 | 4.09 | 4.29 | 4.23 |
| compound AB31788 | 0.71 | 0.69 | 0.63 | 1.03 | 16.85 | 10.80 | 10.89 | 16.46 |
| compound AB31789 | 0.30 | 0.56 | 0.26 | 0.31 | 4.51 | 2.98 | 3.77 | 3.22 |
| compound AB31790 | 0.43 | 0.42 | 0.82 | 0.91 | 7.82 | 4.75 | 3.26 | 3.73 |
| compound AB31805 | 2.11 | 1.35 | 1.84 | 4.11 | >50 | 25.82 | >50 | >50 |
| compound AB31808 | 0.33 | 0.39 | 0.48 | 0.28 | 7.32 | 3.90 | 4.58 | 5.56 |
| compound AB31809 | 0.44 | 0.56 | 0.53 | 0.87 | 15.51 | 7.88 | 10.09 | 11.83 |

| | | | | | | | | |
|---|---|---|---|---|---|---|---|---|
| Note: IC₅₀ refered to 50% inhibiting concentration, ie, the concentration of the compound when 50% inhibitory effect was achieved. | | | | | | | | |

The Table 3 showed the sensitivity of different tumor cells to the compounds prepared in the Examples of the present invention, NCI-H82 (human small cell lung cancer cell), G-401 (human renal carcinoma Wilms cell), MDA-MB-453 (breast cancer cell) and SW48 (human colon adenocarcinoma cell) were sensitive to the compounds prepared in the Examples of the present invention with low IC₅₀ value, while 786-O (clear cell renal cell adenocarcinoma cell), CFPAC-1 (human pancreatic cancer cell), GB-1 (human brain glioblastoma cell) and SF126 (human glioblastoma multiforme cell) were not sensitive to the compounds prepared in the Examples of the present invention with high IC₅₀ value.

### Example 54

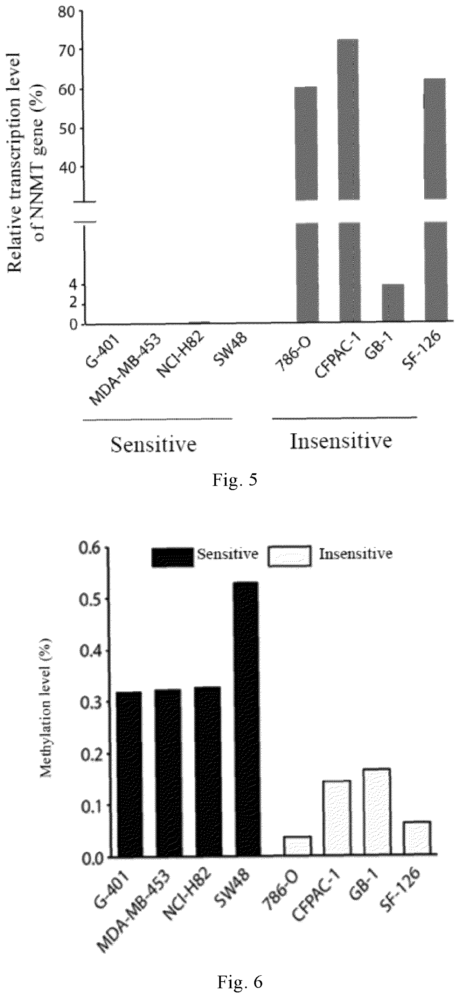

The mRNA transcription level of NNMT gene in four tumor cell lines sensitive to the compounds prepared in the Examples of the present invention and four tumor cell lines insensitive to the compounds prepared in the Examples of the present invention was measured using RT-qPCR gene expression analysis test, and the expression of NNMT gene in the tumor cell lines was measured respectively. The results were shown in Fig.5.

As shown in Fig.5, the mRNA transcription level of NNMT gene in four tumor cell lines (NCI-H82, G-401, MDA-MB-453 and SW48) sensitive to the compounds prepared in the Examples of the present invention and four tumor cell lines (786-O, CFPAC-1, GB-1, and SF126) insensitive to the compounds prepared in the Examples of the present invention was measured using RT-qPCR gene expression analysis test, the results showed the expression of NNMT gene was low in sensitive cell lines (NCI-H82, G-401, MDA-MB-453 and SW48) , and the expression of NNMT gene was high in insensitive cell lines (786-O, CFPAC-1, GB-1 and SF126).

Therefore, the Fig.5 showed that compared with tumor cell lines with high expression of NNMT gene, the inhibitory effect of the compounds prepared in the Examples of the present invention on tumor cell lines with low expression of NNMT gene was significantly enhanced, i.e, the expression of NNMT gene in tumor cell was negatively correlated with the sensitivity of tumor cell to the compounds prepared in the Examples of the present invention. Therefor, the tumor with low expression of NNMT gene was highly sensitive to the compounds prepared in the Examples of the present invention, the compounds prepared in the Examples of the present invention has excellent precision treatment on the tumor with low expression of NNMT gene.

### Example 55

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in four tumor cell lines (NCI-H82, G-401, MDA-MB-453 and SW48) sensitive to the compounds prepared in the Examples of the present invention and four tumor cell lines (786-O, CFPAC-1, GB-1 and SF126) insensitive to the compounds prepared in the Examples of the present invention. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.6, Fig.7, and Fig.8.

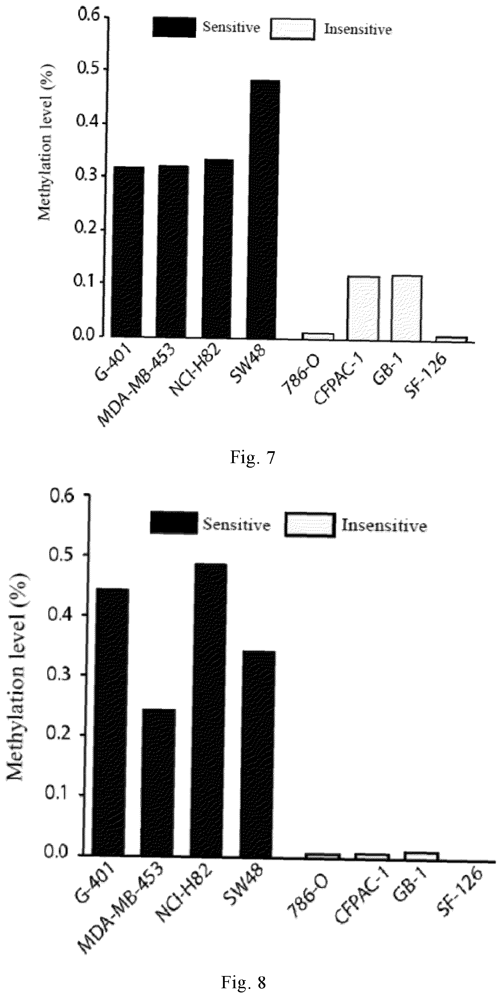

As shown in Fig.6 (the promoter region of NNMT gene), Fig.7 (the region from 1050 bp before the transcription start site to 499 bp after the transcription start site in NNMT gene) and Fig.8 (the region from 1050 bp to 193 bp before the transcription start site in NNMT gene), the compounds prepared in the Examples of the present invention had significantly stronger inhibitory effect on tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, while the compounds prepared in the Examples of the present invention had significantly weaker inhibitory effect on tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499bp after the transcription start site in NNMT gene and the region from 1050 bp to 193 bp before the transcription start site in NNMT gene was positively correlated with the sensitivity of tumor cell to the compounds prepared in the Examples of the present invention. Therefore, the tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene were highly sensitive to the compounds prepared in the Examples of the present invention, the compounds prepared in the Examples of the present invention has excellent precision treatment on the tumor with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene.

### Example 56

The methylation of specific DNA CpG sites from 840 bp (i.e., site 114165695 on human chromosome 11) to 469 bp (i.e., site 114166066 on human chromosome 11) before the transcription start site in NNMT gene in three tumor cell lines (NCI-H82, G-401 and SW48) sensitive to the compounds prepared in the Examples of the present invention and three tumor cell lines (786-O, CFPAC-1 and SF126) insensitive to the compounds prepared in the Examples of the present invention was studied.

Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, then PCR amplification and sequencing analysis were performed on the region using corresponding primers to measure the methylation level of CpG site in the DNA region.

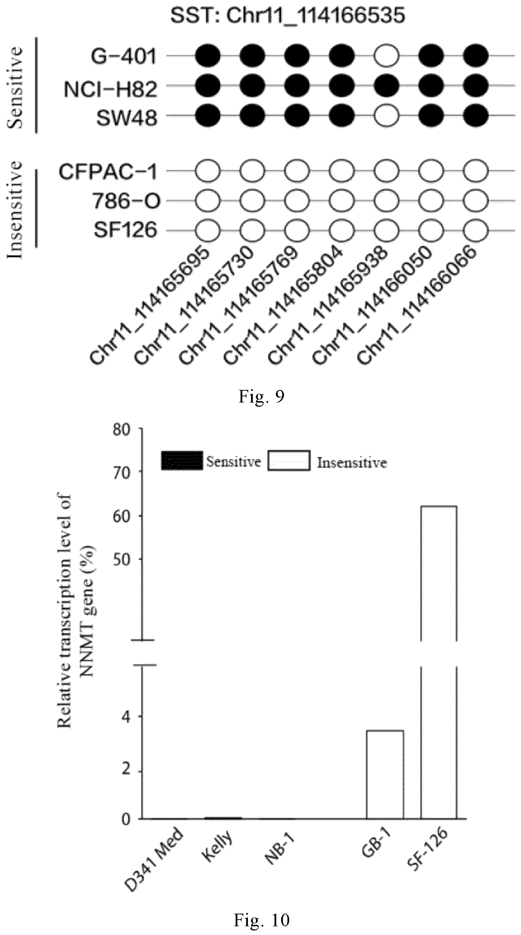

The study showed that almost all of the seven CpG sites (site 114165695, site 114165730, site 114165769, site 114165804, site 114165938, site 114166050 and site 114166066 on the human chromosome 11) were methylated in cell lines (NCI-H82, G-401 and SW48) sensitive to the compounds prepared in the Examples of the present invention, while none of the above seven CpG sites were methylated in cell lines ((786-O, CFPAC-1 and SF126) insensitive to the compounds prepared in the Examples of the present invention, the methylation of related sites was shown in Fig.9.

The sites of the nucleotide sequence in SEQ ID NO: 1 corresponding to the site 114165695, site 114165730, site 114165769, site 114165804, site 114165938, site 114166050 and site 114166066 on the human chromosome 11 were as follows:

| The site on the human chromosome 11 | Corresponding to the sites of the nucleotide sequence in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### Example 57

The inhibitory effect of the compounds prepared in the Examples of the present invention on various brain tumor cell lines was detected using cell viability assay reagent.

### Experimental background:

Cell viability was detected using the Promega CellTiter-Glo kit, cell viability was determined by directly measuring intracellular ATP content. In the experiment, the IC₅₀ value of the compounds prepared in the Examples of the present invention on various brain tumor cell lines was detected.

### Experimental method and result:

Each tumor cell was cultured in relevant medium. After cell passage, the gradient diluted compounds prepared in the Examples of the present invention was added. After 3 days of culture, the relevant IC₅₀ (50% inhibiting concentration) was measured. The name, source and culture conditions of each tumor cell line were as follows:
Cell line D341 Med (ATCC, No. HTB-187) was cultured in 20% fetal bovine serum-containing EMEM medium (+P/S).
Cell line Kelly (ECACC, No. 92110411) was cultured in 10% fetal bovine serum and 2 mM glutamine-containing RPMI1640 medium.
Cell line NB-1 (ATCC, No. HTB-131) was cultured in 10% fetal bovine serum-containing Leibovitz's L-15 medium (+P/S).

The experiment result was shown in Table 4 below:

**Table 4 inhibitory effect of the compounds prepared in the Examples of the present invention on different brain cell lines (IC₅₀, µM)**

| No, | Kelly | NB-1 | D341 Med | SF126 | GB-1 |
|---|---|---|---|---|---|
| compound AB31706 | 1.51 | 0.30 | 1.18 | 6.49 | 12.92 |
| compound AB31707 | 1.24 | 0.29 | 1.37 | 10.18 | 8.27 |
| compound AB31711 | 0.88 | 0.24 | 0.92 | 5.21 | 5.32 |
| compound AB31718 | 0.66 | 0.02 | 0.18 | 4.25 | 3.41 |
| compound AB31721 | 1.26 | 0.14 | 0.36 | 10.83 | 10.05 |
| compound AB31722 | 2.52 | 0.98 | 2.89 | 20.55 | 40.45 |
| compound AB31731 | 0.86 | 0.07 | 0.34 | 6.19 | 6.64 |
| compound AB31737 | 0.36 | 0.02 | 0.40 | 4.14 | 2.66 |
| compound AB31739 | 0.64 | 0.09 | 0.86 | 3.57 | 3.97 |
| compound AB31740 | 0.84 | 0.08 | 0.53 | 7.77 | 7.74 |
| compound AB31748 | 0.46 | 0.21 | 0.67 | 3.53 | 3.21 |
| compound AB31757 | 1.50 | 0.15 | 2.82 | 16.03 | 17.50 |
| compound AB31758 | 0.59 | 0.12 | 0.98 | 5.14 | 6.47 |
| compound AB31759 | 0.41 | 0.12 | 0.85 | 3.94 | 3.47 |
| compound AB31760 | 0.23 | 0.09 | 0.70 | 3.89 | 3.67 |
| compound AB31765 | 1.29 | 0.30 | 1.63 | 9.80 | 14.83 |
| compound AB31767 | 0.30 | 0.12 | 0.57 | 3.70 | 3.61 |
| compound AB31771 | 2.21 | 1.04 | 1.66 | 15.71 | 11.23 |
| compound AB31780 | 1.16 | 0.23 | 1.88 | 9.75 | 12.55 |
| compound AB31781 | 0.73 | 0.14 | 0.82 | 7.48 | 8.60 |
| compound AB31787 | 0.69 | 0.08 | 0.33 | 4.29 | 4.23 |
| compound AB31788 | 1.30 | 0.31 | 1.88 | 10.89 | 16.46 |
| compound AB31789 | 0.46 | 0.21 | 0.51 | 3.77 | 3.22 |
| compound AB31790 | 0.27 | 0.23 | 0.26 | 3.26 | 3.73 |
| compound AB31805 | 4.82 | 1.03 | 4.80 | >50 | >50 |
| compound AB31808 | 1.05 | 0.06 | 0.43 | 4.58 | 5.56 |
| compound AB31809 | 0.99 | 0.16 | 1.92 | 10.09 | 11.83 |

| | | | | | |
|---|---|---|---|---|---|
| Note: IC₅₀ refered to 50% inhibiting concentration, ie, the concentration of the compound when 50% inhibitory effect was achieved. | | | | | |

The Table 4 showed the sensitivity of different brain tumor cells to the compounds prepared in the Examples of the present invention, D341 Med (cerebellar medulloblastoma cell), Kelly (brain neuroblastoma cell) and NB-1 (brain neuroblastoma cell) were sensitive to the compounds prepared in the Examples of the present invention with low IC₅₀ value, while GB-1 (human brain glioblastoma cell) and SF-126 (human glioblastoma multiforme cell) were not sensitive to the compounds prepared in the Examples of the present invention with high IC₅₀ value.

### Example 58

The mRNA transcription level of NNMT gene in three brain tumor cell lines (D341 Med, Kelly and NB-1) sensitive to the compounds prepared in the Examples of the present invention and two brain tumor cell lines (GB-1 and SF126) insensitive to the compounds prepared in the Examples of the present invention was measured using RT-qPCR gene expression analysis test, and the expression of NNMT gene in the tumor cell lines was measured respectively. The results were shown in Fig.10.

As shown in Fig.10, the mRNA transcription level of NNMT gene in three brain tumor cell lines (D341 Med, Kelly and NB-1) sensitive to the compounds prepared in the Examples of the present invention and two brain tumor cell lines (GB-1 and SF126) insensitive to the compounds prepared in the Examples of the present invention was measured using RT-qPCR gene expression analysis test, the results showed the expression of NNMT gene was low in sensitive cell lines (D341 Med, Kelly and NB-1), and the expression of NNMT gene was high in insensitive cell lines (GB-1 and SF126).

Therefore, the Fig. 10 showed that compared with brain tumor cell lines with high expression of NNMT gene, the inhibitory effect of the compounds prepared in the Examples of the present invention on brain tumor cell lines with low expression of NNMT gene was significantly enhanced, i.e, the expression of NNMT gene in brain tumor cell was negatively correlated with the sensitivity of brain tumor cell to the compounds prepared in the Examples of the present invention, therefor, the brain tumor with low expression of NNMT gene was highly sensitive to the compounds prepared in the Examples of the present invention, the compounds prepared in the Examples of the present invention has excellent precision treatment on the brain tumor with low expression of NNMT gene.

### Example 59

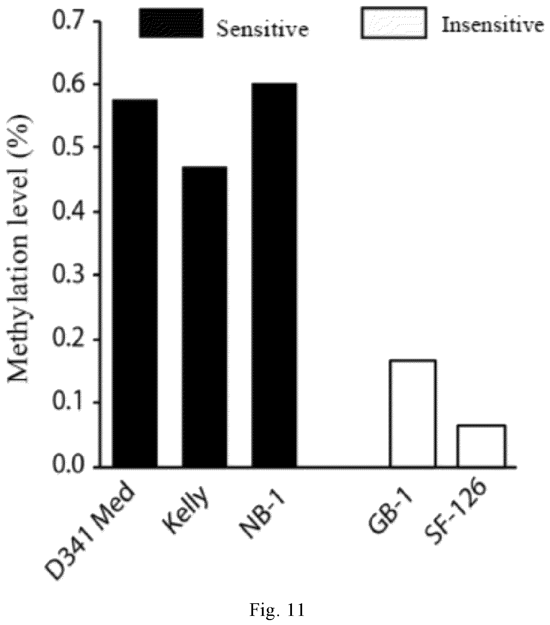

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in three brain tumor cell lines (D341 Med, Kelly and NB-1) sensitive to the compounds prepared in the Examples of the present invention and two brain tumor cell lines (GB-1 and SF126) insensitive to the compounds prepared in the Examples of the present invention. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.11, Fig.12, and Fig.13.

As shown in Fig.11 (the promoter region of NNMT gene), Fig.12 (the region from 1050 bp before the transcription start site to 499 bp after the transcription start site in NNMT gene) and Fig.13 (the region from 1050 bp to 193 bp before the transcription start site in NNMT gene), the compounds prepared in the Examples of the present invention had significantly stronger inhibitory effect on brain tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, while the compounds prepared in the Examples of the present invention had significantly weaker inhibitory effect on brain tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499bp after the transcription start site in NNMT gene and the region from 1050 bp to 193 bp before the transcription start site in NNMT gene was positively correlated with the sensitivity of brain tumor cell to the compounds prepared in the Examples of the present invention. Therefore, the brain tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene were highly sensitive to the compounds prepared in the Examples of the present invention, the compounds prepared in the Examples of the present invention has excellent precision treatment on the tumor with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene.

### Example 60

The methylation level of DNA in cell was maintained by DNA methylation enzymes DNMT3a, DNMT3b and DNMT1. The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumor was determined in the Example.

### Experiment method and result:

The expression of NNMT gene, DNMT1, UHRF1, DNMT3a and DNMT3b in various cells were obtained from a public database (Cancer Cell Line Encyclopedia, CCLE, 1019 cells in total). Then, the correlation between expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.14.

The Fig. 14 showed the expression of NNMT was negatively correlated with the expression of DNA methylase and UHRF1 in each cell. Therefore, the tumor with high expression of DNA methylase and UHRF1 was highly sensitive to the compounds prepared in the Examples of the present invention, the compounds prepared in the Examples of the present invention has excellent precision treatment on the tumor with high expression of DNA methylase and UHRF1.

### Example 61

The role of expression level of NNMT gene and DNMT1 in the sensitivity of NCI-H82 cell to the compounds prepared in the Examples of the present invention was determined

### Experiment method and result:

The expression of NNMT protein in NCI-H82 cell was overexpressed by inserting the NNMT gene into NCI-H82 cell using a viral vector, and the NNMT protein-overexpressing NCI-H82 cell (ov-NNMT NCI-H82 cell) was obtained. The expression of DNMT1 in NCI-H82 cell was knocked down by transfecting shRNA (the nucleotide sequence of shRNA was GATCCGGGATGAGTCCATCAAGGAAGATTCAAGAGATCTTCCTTGATGGACTCATCCT TTTTTG (SEQ ID No: 2)) using a viral vector, and the NCI-H82 cell with low expression of DNMT1 protein (sh-DNMT1 NCI-H82 cell) was obtained. The control NCI-H82 cell (Con-NCI-H82 cell) was obtained by transfecting NCI-H82 cell with empty virus vector without carrying NNMT gene and shRNA. The expressing content of NNMT protein and DNMT1 protein in the Con-NCI-H82 cell, the expressing content of NNMT protein in the ov-NNMT NCI-H82 cell, and the expressing content of DNMT1 protein in the sh-DNMT1 NCI-H82 cell were detected using Western Blot assay (as shown in Fig.15 and Fig.16 ), it could be seen from Fig.15 and Fig.16 that the NNMT protein was overexpressed in the ov-NNMT NCI-H82 cell and the expression of DNMT1 protein was knocked down (i.e, low expression) in the sh-DNMT1 NCI-H82 cell compared with the expression level of NNMT protein and DNMT protein in the Con-NCI-H82 cell.

The cell viability of Con-NCI-H82 cell, ov-NNMT NCI-H82 cell and sh-DNMT1 NCI-H82 cell was detected using the Promega CellTiter-Glo kit which reflected cell viability by measuring intracellular ATP content (as shown in Fig.17 and Fig.18) , it could be seen from Fig.17 and Fig.18 that the cell viability of Con-NCI-H82 cell, ov-NNMT NCI-H82 cell and sh-DNMT1 NCI-H82 cell was almost the same, and the difference in cell viability was not statistically significant.

The inhibitory effect (IC₅₀ value) of the compounds prepared in the Examples of the present invention on Con-NCI-H82 cell, ov-NNMT NCI-H82 cell and sh-DNMT1 NCI-H82 cell was detected using Promega CellTiter-Glo kit which directly measured intracellular ATP content, the result was shown in Table 5 below:

**Table 5 the inhibitory effect of the compounds prepared in the Examples of the present invention on Con-NCI-H82 cell, ov-NNMT NCI-H82 cell and sh-DNMT1 NCI-H82 cell (IC₅₀, µM)**

| Compound no. | Con-NCI-H82 (µM) | ov-NNMT NCI-H82 (µM) | sh-DNMT1 NCI-H82 (µM) |
|---|---|---|---|
| compound AB31706 | 1.48 | 3.15 | 4.64 |
| compound AB31707 | 1.17 | 2.96 | 3.41 |
| compound AB31711 | 0.65 | 1.43 | 1.9 |
| compound AB31716 | 0.07 | 0.38 | 0.35 |
| compound AB31717 | 0.11 | 0.25 | 0.36 |
| compound AB31718 | 0.29 | 0.68 | 1.36 |
| compound AB31721 | 0.4 | 0.92 | 1.31 |
| compound AB31722 | 2.81 | 5.98 | 8.93 |
| compound AB31723 | 3.94 | 8.28 | 8.08 |
| compound AB31731 | 0.52 | 1.32 | 1.66 |
| compound AB31737 | 0.24 | 0.73 | 0.94 |
| compound AB31739 | 0.45 | 0.95 | 1.55 |
| compound AB31740 | 0.49 | 1.55 | 1.57 |
| compound AB31748 | 0.32 | 0.78 | 0.91 |
| compound AB31757 | 0.88 | 3.32 | 3.82 |
| compound AB31758 | 0.36 | 0.92 | 1.38 |
| compound AB31759 | 0.27 | 0.64 | 1.18 |
| compound AB31760 | 0.15 | 0.35 | 0.71 |
| compound AB31761 | 0.27 | 0.74 | 0.98 |
| compound AB31762 | 0.13 | 0.32 | 0.49 |
| compound AB31763 | 0.08 | 0.31 | 0.42 |
| compound AB31765 | 1.34 | 2.88 | 4.05 |
| compound AB31766 | 2.47 | 4.95 | 4.84 |
| compound AB31767 | 0.26 | 0.65 | 0.98 |
| compound AB31771 | 1.87 | 3.94 | 5.81 |
| compound AB31780 | 0.66 | 1.52 | 1.98 |
| compound AB31781 | 0.33 | 1.16 | 1.42 |
| compound AB31787 | 0.34 | 0.88 | 1.31 |
| compound AB31788 | 0.71 | 2.42 | 2.95 |
| compound AB31789 | 0.30 | 0.85 | 1.05 |
| compound AB31790 | 0.43 | 1.52 | 1.39 |
| compound AB31794 | 0.14 | 0.61 | 0.55 |
| compound AB31795 | 0.24 | 0.68 | 0.88 |
| compound AB31796 | 0.27 | 0.64 | 0.76 |
| compound AB31797 | 0.12 | 0.54 | 0.48 |
| compound AB31800 | 0.22 | 0.65 | 0.58 |
| compound AB31805 | 2.11 | 4.82 | 6.04 |
| compound AB31808 | 0.33 | 0.86 | 1.04 |
| compound AB31809 | 0.44 | 1.38 | 1.86 |
| compound AB35511 | 0.61 | 1.38 | 2.56 |
| compound AB36412 | 1.17 | 2.58 | 4.69 |
| compound AB31755 | 2.36 | 4.82 | 7.68 |
| compound AB31756 | 1.01 | 2.38 | 4.36 |
| compound AB35595 | 0.68 | 1.54 | 3.15 |
| compound AB36404 | 1.06 | 2.54 | 4.86 |
| compound AB35483 | 1.26 | 2.88 | 5.32 |
| compound AB35532 | 0.34 | 0.96 | 2.56 |
| compound AB36425 | 0.85 | 1.92 | 3.88 |
| compound AB31815 | 0.65 | 1.35 | 2.18 |
| compound AB36508 | 0.56 | 1.34 | 2.82 |
| compound AB36484 | 1.84 | 3.85 | 8.32 |
| compound AB31811 | 3.12 | 7.34 | 12.45 |

| | | | |
|---|---|---|---|
| Note: IC₅₀ refered to 50% inhibiting concentration, ie, the concentration of the compound when 50% inhibitory effect was achieved. Con-NCI-H82 refered to the NCI-H82 cell transfected with empty virus vector without carrying NNMT gene and shRNA, which was used as control; ov-NNMT NCI-H82 refered to the NCI-H82 cell transfected with virus vector carrying NNMT gene, the NNMT protein was overexpressed in the ov-NNMT NCI-H82 cell; sh-DNMT1 NCI-H82 refered to the NCI-H82 cell transfected with virus vector carrying shRNA; the expression of DNMT1 was knocked down (i.e, low expression) in the sh-DNMT1 NCI-H82 cell. | | | |

As was shown in Table 5, the NNMT protein in NCI-H82 cell was overexpressed and the expression of DNMT1 in NCI-H82 cell was knocked down in the Example, the results further confirmed that the compounds prepared in the Examples of the present invention had significant inhibitory effect on tumor cell with low or no expression of NNMT gene and high expression of DNMT1 while the compounds prepared in the Examples of the present invention had weak inhibitory effect on tumor cells with high expression of NNMT gene and low expression of DNMT1. Decreasing the expression of NNMT gene and increasing the expression of DNMT1 in tumor could significantly improve the inhibitory effect of the compounds prepared in the Examples of the present invention on tumor on tumor, the expression level of NNMT gene in tumor cell was significantly negative correlation with the sensitivity of tumor cell to the compounds prepared in the Examples of the present invention, while the expression level of DNMT1 in tumor cell was significantly positive correlation with the sensitivity of tumor cell to the compounds prepared in the Examples of the present invention. Therefore, the compounds prepared in the Examples of the present invention had more significant inhibitory effect on tumor cell with low or no expression of NNMT gene and high expression of DNMT1, the tumor cell with low or no expression of NNMT gene and high expression of DNMT1 were highly sensitive to the compounds prepared in the Examples of the present invention. Therefore, the compounds prepared in the Examples of the present invention has excellent precision treatment on the tumor cell with low or no expression of NNMT gene and high expression of DNMT1.

All documents mentioned in the present invention are incorporated herein by reference, as if each document is individually cited for reference. It should be understood that those skilled in the art will be able to make various changes or modifications to the present invention after reading the teachings of the present invention, which also fall within the scope of the claims appended hereto.

## Claims

1. A use of a compound of formula I, or an optical isomer thereof, or a racemate thereof, or a solvate thereof, or a pharmaceutically acceptable salt thereof, or a deuterated compound thereof in the preparation of a composition or a preparation for preventing and/or treating tumor; 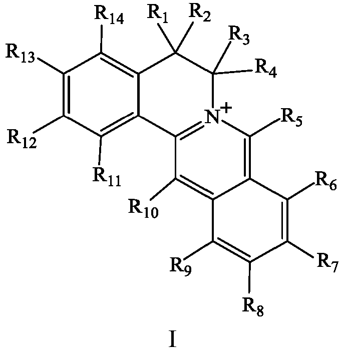 wherein,
R₁, R₂, R₃ and R₄ are each independently hydrogen, substituted or unsubstituted C1-C6 alkyl;
R₅ is hydrogen, substituted or unsubstituted C1-C10 alkyl, substituted or unsubstituted C3-C10 cycloalkyl;
R₆ is -O-R₁₅ or -N (R₁₆R₁₇);
R₇ is hydrogen, substituted or unsubstituted C1-C6 alkoxyl, substituted or unsubstituted C1-C8 haloalkoxyl;
R₈ is hydrogen, substituted or unsubstituted C1-C6 alkyl, halogen;
R₉ is hydrogen, substituted or unsubstituted C1-C6 alkyl, halogen;
R₁₀ is hydrogen, substituted or unsubstituted C1-C16 alkyl, substituted or unsubstituted C6-C12 aryl-substituted or unsubstituted C1-C6 alkyl-, substituted or unsubstituted C1-C6 alkyl-substituted or unsubstituted C6-C12 aryl-C1-C6 alkyl-, substituted or unsubstituted C1-C6 haloalkoxyl-substituted or unsubstituted C6-C12 aryl-substituted or unsubstituted C1-C6 alkyl-, substituted or unsubstituted C1-C6 haloalkyl-substituted or unsubstituted C6-C12 aryl-substituted or unsubstituted C1-C6 alkyl-, substituted or unsubstituted C1-C6 alkoxyl-substituted or unsubstituted C6-C12 aryl-substituted or unsubstituted C1-C6 alkyl-;
R₁₁ and R₁₄ are each independently hydrogen, substituted or unsubstituted C1-C6 alkyl;
R₁₂ and R₁₃ are connected to form a substituted or unsubstituted 3-10 membered heterocycloalkane ring;
R₁₅ is substituted or unsubstituted C1-C18 alkyl, deuterated C1-C18 alkyl, substituted or unsubstituted C6-C12 aryl, substituted or unsubstituted C6-C12 aryl-substituted or unsubstituted C6-C12 aryl-substituted or unsubstituted C1-C8 alkyl-, substituted or unsubstituted C6-C12 aryl-O-substituted or unsubstituted C1-C6 alkyl-, substituted or unsubstituted C1-C10 haloalkyl, substituted or unsubstituted C2-C16 acyl, substituted or unsubstituted C2-C8 alkenyl-substituted or unsubstituted C1-C4 alkyl-substituted or unsubstituted C2-C8 alkenyl-substituted or unsubstituted C1-C4 alkyl-, substituted or unsubstituted C3-C14 cycloalkyl-substituted or unsubstituted C2-C6 acyl-, substituted or unsubstituted C6-C12 aryl-substituted or unsubstituted C2-C8 alkenyl-C(O)-substituted or unsubstituted C1-C4 alkyl-, or 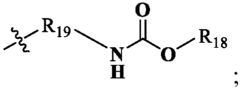
R₁₆ and R₁₇ are each independently hydrogen, substituted or unsubstituted C1-C12 alkyl, substituted or unsubstituted C6-C12 aryl-substituted or unsubstituted C1-C4 alkyl-;
R₁₈ is substituted or unsubstituted C1-C8 alkyl;
R₁₉ is substituted or unsubstituted C1-C10 alkylidene;
the heterocyclic ring of the heterocycloalkane ring and heteroaryl has 1-4 (preferably 1, 2 or 3) heteroatoms independently selected from the group consisting of N, O and S;
the "substituted" means that one or more (preferably 1, 2, 3, or 4) hydrogen atoms on the ring or group are each independently substituted by the substituent selected from the group consisting of C1-C6 alkyl, C2-C6 alkenyl, halogen, C3-C6 cycloalkyl, C1-C6 haloalkyl, C1-C6 alkoxyl, C1-C6 haloalkoxyl, C3-C6 cycloalkoxyl, C2-C6 acyl, C6-C12 aryl, 5-12 membered heteroaryl, C6-C12 aryl-O-,

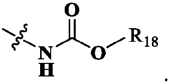

2. The use of claim 1, wherein the compound of formula I, or an optical isomer thereof, or a racemate thereof, or a solvate thereof, or a pharmaceutically acceptable salt thereof, or a deuterated compound thereof is selected from the following group:

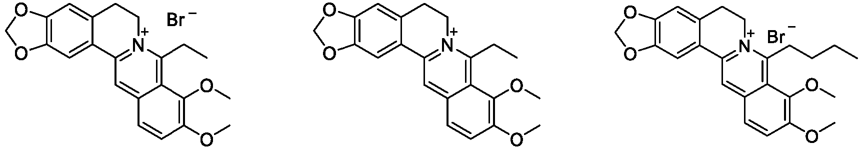

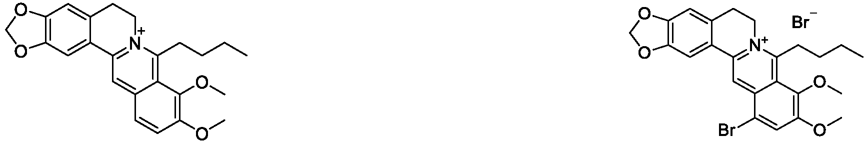

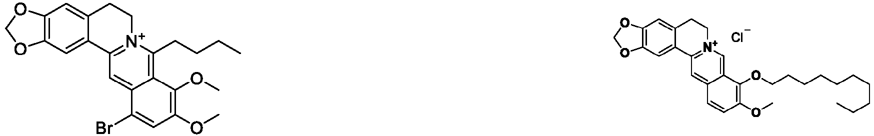

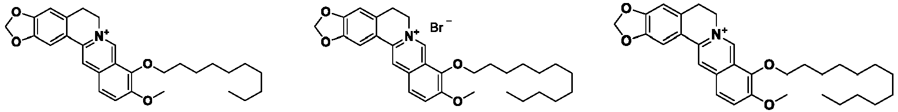

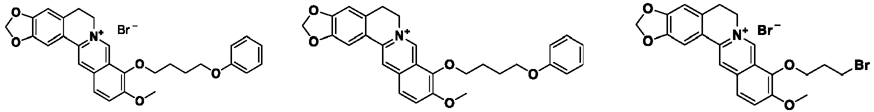

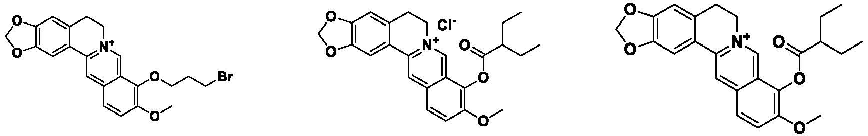

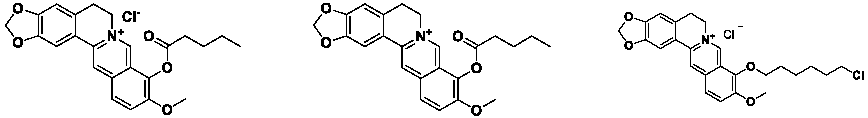

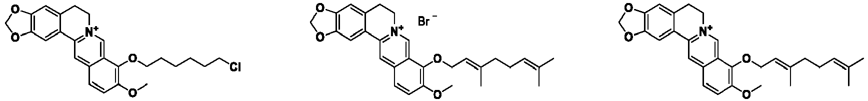

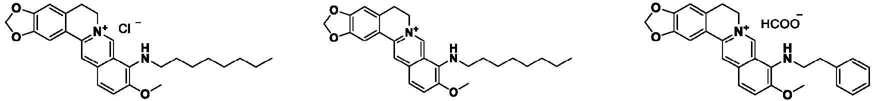

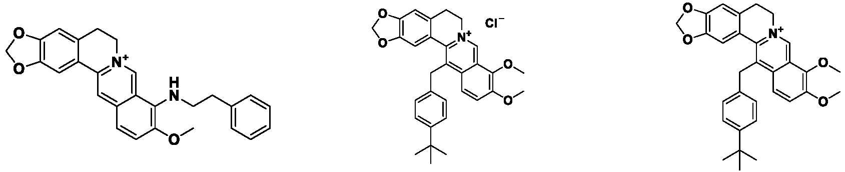

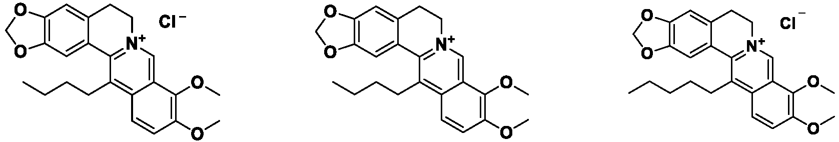

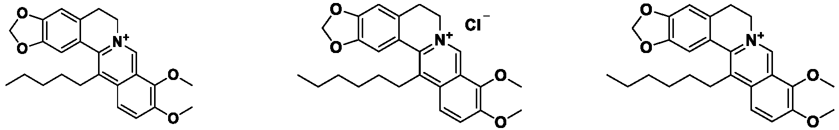

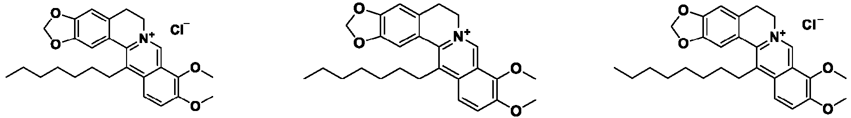

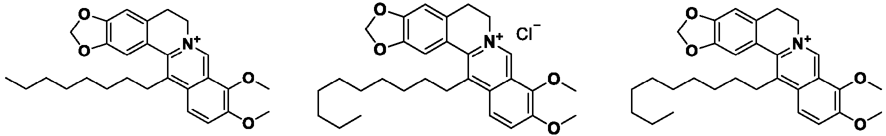

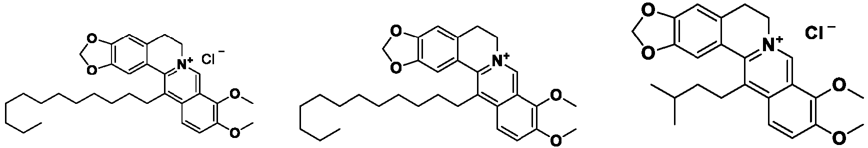

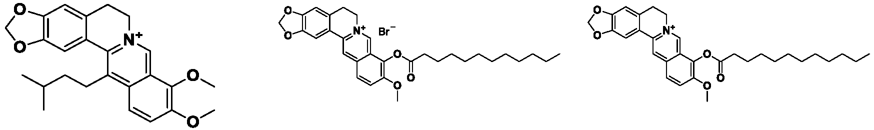

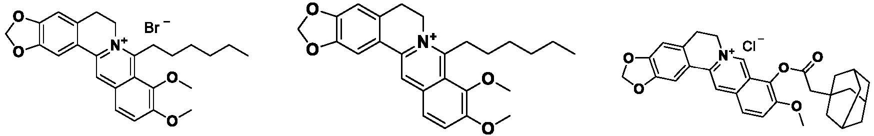

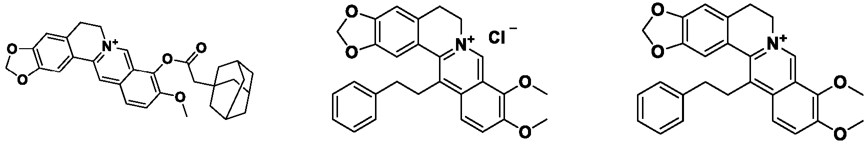

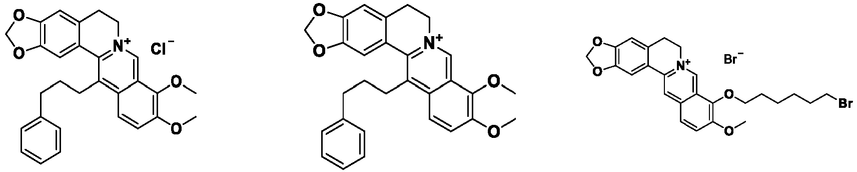

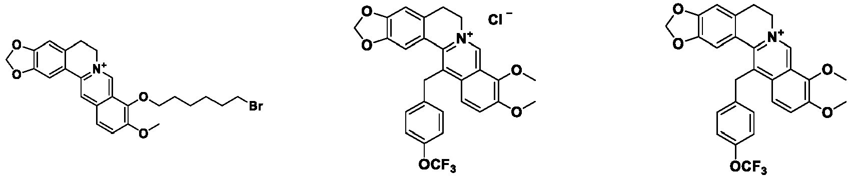

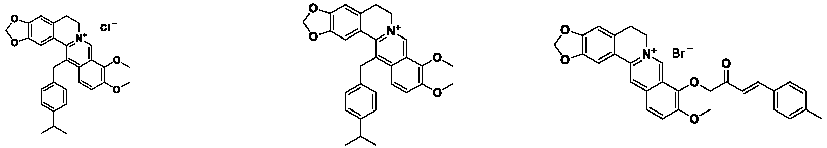

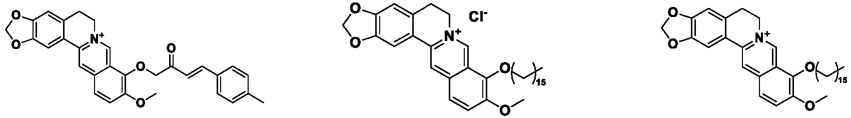

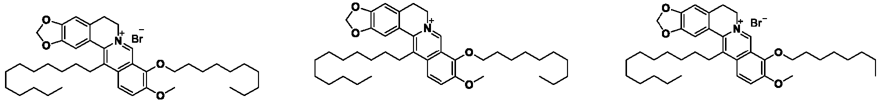

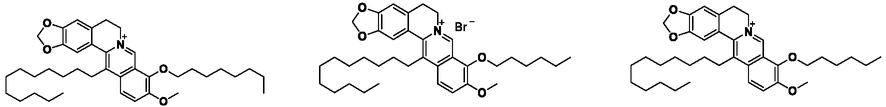

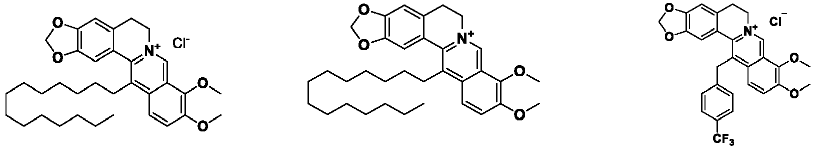

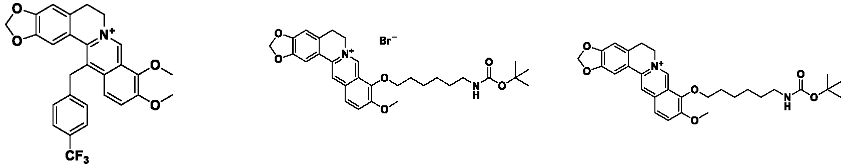

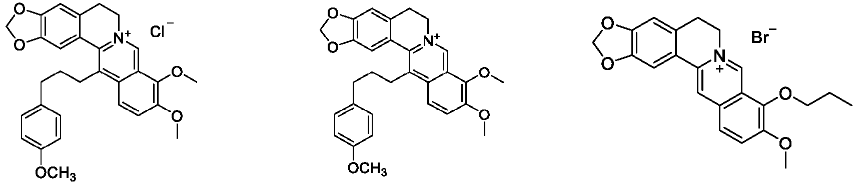

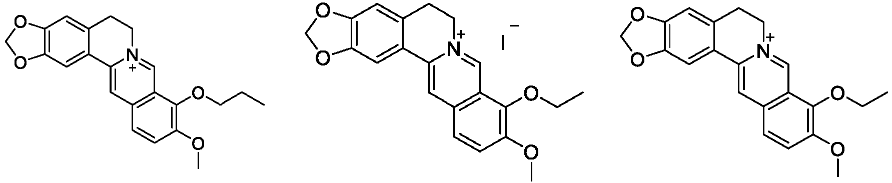

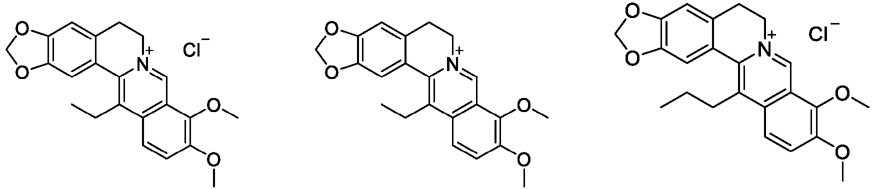

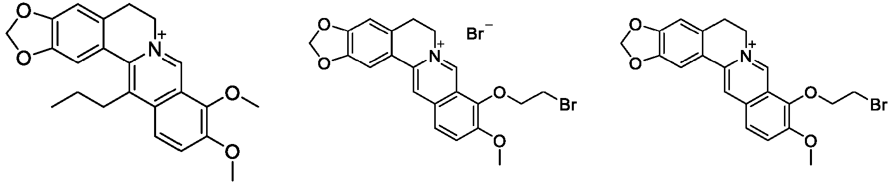

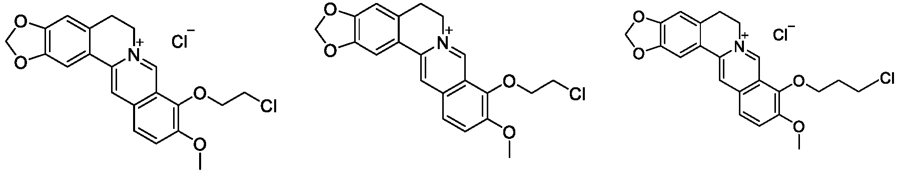

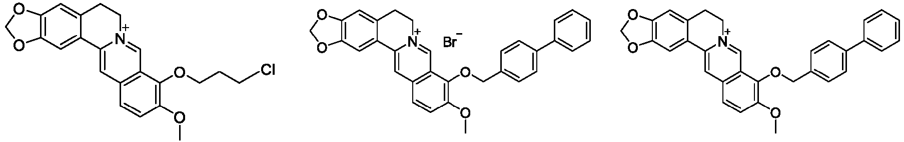

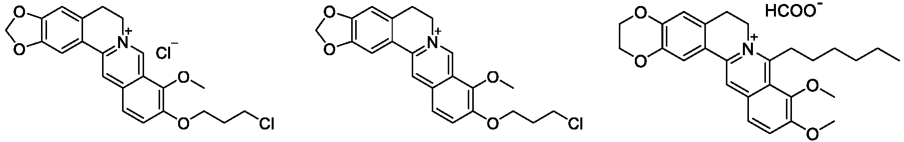

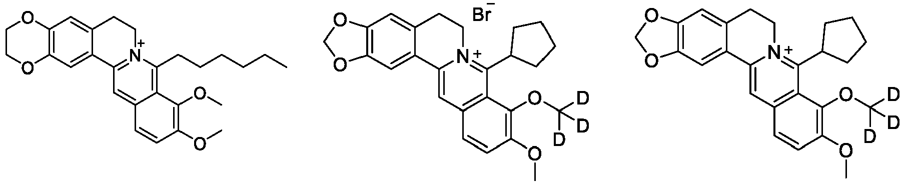

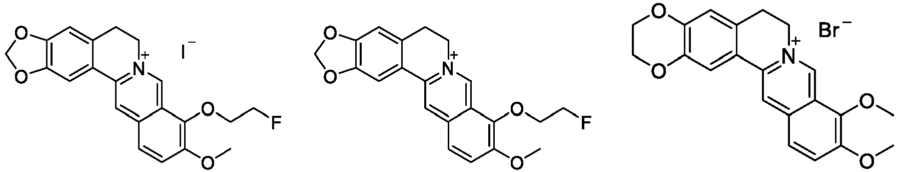

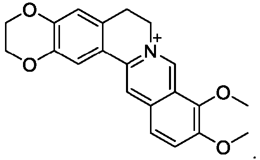

3. The use of claim 1, wherein the tumor comprises tumor with low or no expression of NNMT gene;
tumor with high expression of DNA methylase;
tumor with high expression of UHRFl;
tumor with high methylation level of nucleotide site of NNMT gene; and/or
tumor with high methylation level of DNA CpG site of NNMT gene.

4. The use of claim 3, wherein the DNA methylase is selected from the group consisting of DNMT1, DNMT3a, DNMT3b, and combinations thereof; and/or
the methylation level of DNA CpG site of NNMT gene comprises the methylation level of DNA CpG site in promoter region of NNMT gene.

5. The use of claim 1, wherein the tumor is selected from the group consisting of lung cancer, renal carcinoma, breast cancer, colon cancer, lymphoma, leukemia, pancreatic cancer, brain tumor, liver cancer, prostate cancer, and combinations thereof.

6. A marker for determining whether the compound of formula I, or an optical isomer thereof, or a racemate thereof, or a solvate thereof, or a pharmaceutically acceptable salt, or a deuterated compound thereof of claim 1 is suitable for use in the prevention and/or treatment of patient tumor, the marker comprises the expression level of NNMT gene, the expression level of DNA methylase, the expression level of UHRF1, the methylation level of nucleotide site of NNMT gene, and/or the methylation level of DNA CpG site of NNMT gene.

7. A use of a detection kit in the preparation of concomitant diagnose kit for determining whether the compound of formula I, or an optical isomer thereof, or a racemate thereof, or a solvate thereof, or a pharmaceutically acceptable salt, or a deuterated compound thereof of claim 1 is suitable for use in the prevention and/or treatment of patient tumor;
the detection kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene.

8. A medicine kit, wherein the medicine kit comprises:
(i) a detection reagent for detecting the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt, or a deuterated compound thereof of claim 1.

9. A method for preventing and/or treating tumor, wherein the method comprises administering the compound of formula I, or an optical isomer thereof, or a racemate thereof, or a solvate thereof, or a pharmaceutically acceptable salt, or a deuterated compound thereof of claim 1 to a subject in need.

10. A device or system, wherein the device or system comprises:
(i) a detection module, the detection module is used to detect the expression level of NNMT gene, the expression level of DNA methylase, the expression level of UHRF1, the methylation level of nucleotide site of NNMT gene, and/or the methylation level of DNA CpG site of NNMT gene;
(ii) an output module, the output module comprises the information as follows:
the compound of formula I, or an optical isomer thereof, or a racemate thereof, or a solvate thereof, or a pharmaceutically acceptable salt, or a deuterated compound thereof of claim 1 is suitable for use in the prevention and/or treatment of patient tumor with low or no expression of NNMT gene, high expression of DNA methylase, high expression of UHRFl, high methylation level of nucleotide site of NNMT gene, and/or high methylation level of DNA CpG site of NNMT gene; and/or
the compound of formula I, or an optical isomer thereof, or a racemate thereof, or a solvate thereof, or a pharmaceutically acceptable salt, or a deuterated compound thereof of claim 1 is not suitable for use in the prevention and/or treatment of patient tumor with high expression of NNMT gene, low expression of DNA methylase, low expression of UHRFl, low methylation level of nucleotide site of NNMT gene, and/or low methylation level of DNA CpG site of NNMT gene.

11. A compound of formula I, or an optical isomer thereof, or a racemate thereof, or a solvate thereof, or a pharmaceutically acceptable salt thereof, or a deuterated compound thereof; 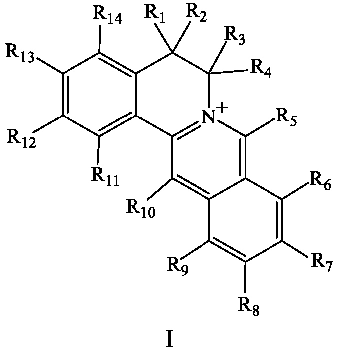 wherein,
R₁, R₂, R₃ and R₄ are each independently hydrogen, substituted or unsubstituted C1-C6 alkyl;
R₅ is hydrogen, substituted or unsubstituted C1-C10 alkyl, substituted or unsubstituted C3-C10 cycloalkyl;
R₆ is -O-R₁₅ or -N (R₁₆R₁₇);
R₇ is hydrogen, substituted or unsubstituted C1-C6 alkoxyl, substituted or unsubstituted C1-C8 haloalkoxyl;
R₈ is hydrogen, substituted or unsubstituted C1-C6 alkyl, halogen;
R₉ is hydrogen, substituted or unsubstituted C1-C6 alkyl, halogen;
R₁₀ is hydrogen, substituted or unsubstituted C1-C16 alkyl, substituted or unsubstituted C6-C12 aryl-substituted or unsubstituted C1-C6 alkyl-, substituted or unsubstituted C1-C6 alkyl-substituted or unsubstituted C6-C12 aryl-C1-C6 alkyl-, substituted or unsubstituted C1-C6 haloalkoxyl-substituted or unsubstituted C6-C12 aryl-substituted or unsubstituted C1-C6 alkyl-, substituted or unsubstituted C1-C6 haloalkyl-substituted or unsubstituted C6-C12 aryl-substituted or unsubstituted C1-C6 alkyl-, substituted or unsubstituted C1-C6 alkoxyl-substituted or unsubstituted C6-C12 aryl-substituted or unsubstituted C1-C6 alkyl-;
R₁₁ and R₁₄ are each independently hydrogen, substituted or unsubstituted C1-C6 alkyl;
R₁₂ and R₁₃ are connected to form a substituted or unsubstituted 3-10 membered heterocycloalkane ring;
R₁₅ is substituted or unsubstituted C1-C18 alkyl, deuterated C1-C18 alkyl, substituted or unsubstituted C6-C12 aryl, substituted or unsubstituted C6-C12 aryl-substituted or unsubstituted C6-C12 aryl-substituted or unsubstituted C1-C8 alkyl-, substituted or unsubstituted C6-C12 aryl-O-substituted or unsubstituted C1-C6 alkyl-, substituted or unsubstituted C1-C10 haloalkyl, substituted or unsubstituted C2-C16 acyl, substituted or unsubstituted C2-C8 alkenyl-substituted or unsubstituted C1-C4 alkyl-substituted or unsubstituted C2-C8 alkenyl-substituted or unsubstituted C1-C4 alkyl-, substituted or unsubstituted C3-C14 cycloalkyl-substituted or unsubstituted C2-C6 acyl-, substituted or unsubstituted C6-C12 aryl-substituted or unsubstituted C2-C8 alkenyl-C(O)-substituted or unsubstituted C1-C4 alkyl-, or 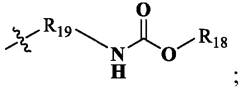
R₁₆ and R₁₇ are each independently hydrogen, substituted or unsubstituted C1-C12 alkyl, substituted or unsubstituted C6-C12 aryl-substituted or unsubstituted C1-C4 alkyl-;
R₁₈ is substituted or unsubstituted C1-C8 alkyl;
R₁₉ is substituted or unsubstituted C1-C10 alkylidene;
the heterocyclic ring of the heterocycloalkane ring and heteroaryl has 1-4 (preferably 1, 2 or 3) heteroatoms independently selected from the group consisting of N, O and S;
the "substituted" means that one or more (preferably 1, 2, 3, or 4) hydrogen atoms on the ring or group are each independently substituted by the substituent selected from the group consisting of C1-C6 alkyl, C2-C6 alkenyl, halogen, C3-C6 cycloalkyl, C1-C6 haloalkyl, C1-C6 alkoxyl, C1-C6 haloalkoxyl, C3-C6 cycloalkoxyl, C2-C6 acyl, C6-C12 aryl, 5-12 membered heteroaryl, C6-C12 aryl-O-,

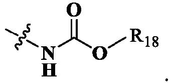

12. The use of claim 11, wherein the compound of formula I, or an optical isomer thereof, or a racemate thereof, or a solvate thereof, or a pharmaceutically acceptable salt thereof, or a deuterated compound thereof is selected from the following group:
